# EUROPEAN PATENT APPLICATION

(11) **EP 4 759 268 A2**
(43) Date of publication of application: **17.06.2026**
(21) Application number: 26174977.4
(22) Date of filing: 11.02.2022
(51) Int. Cl.: A61F 2/24

(54) **DELIVERY SYSTEMS FOR REPLACEMENT HEART VALVES**

(30) Priority: 11.02.2021 US 202163148501 P; 29.10.2021 US 202163273402 P
(62) Divisional of application: 22707557.9
(71) Applicant: Edwards Lifesciences Corporation, Irvine, CA 92614 (US)
(72) Inventor: LUONG, Hieu Minh, CA 92683 (US); SANCHEZ, Julio Cesar, CA 92614 (US); COOPER, Alexander H., CA 92626 (US); EDWARDS, Jesse Robert, CA 92614 (US); OKABE, Hiroshi, CA 92614 (US); NESBITT, Karen Fromell, CA 92614 (US); LANDON, David Robert, CA 92614 (US); POULSEN, Nikolai Brent, CA 92614 (US); SCHEINBLUM, Taylor Jacob, CA 92660-3287 (US); DIXON, Eric Robert, CA 92614 (US); RICKERSON, Cooper Ryan, CA 92614 (US); VANEVERY, Zachary Charles, CA 92614 (US)
(74) Representative: Venner, Julia Ann

(57) **Abstract**

A delivery system includes a suture attachment mechanism for maintaining a connection to a heart valve after initial deployment, thereby allowing recapture of the heart valve if desired. A plurality of suture portions is provided along a distal end portion of the delivery system, each suture portion having a first end fixed to the delivery system and a second end releasably coupled to a retention member on the delivery system. A knob on a handle of the delivery system is actuated for causing the second end of each suture portion to be released from its respective retention member, thereby allowing the suture portions to be decoupled from the heart valve. The suture portions are located only along a distal end portion of the delivery system for improving reliability and consistency.

## Description

### RELATED APPLICATIONS

This application claims the benefit of U.S. Provisional Application No. 63/148,501, filed February 11, 2021; and U.S. Provisional Application No. 63/273,402, filed October 29, 2021; the entirety of each of which is hereby incorporated by reference.

### FIELD

Certain embodiments disclosed herein relate generally to prostheses for implantation within a lumen or body cavity and delivery systems for a prosthesis. In particular, the prostheses and delivery systems relate in some embodiments to replacement heart valves, such as replacement mitral heart valves or replacement tricuspid heart valves.

### BACKGROUND

Human heart valves, which include the aortic, pulmonary, mitral and tricuspid valves, function essentially as one-way valves operating in synchronization with the pumping heart. The valves allow blood to flow downstream, but block blood from flowing upstream. Diseased heart valves exhibit impairments, such as narrowing of the valve or regurgitation, which inhibit the valves' ability to control blood flow. Such impairments reduce the heart's blood-pumping efficiency and can be a debilitating and life-threatening condition. For example, valve insufficiency can lead to conditions such as heart hypertrophy and dilation of the ventricle. Thus, extensive efforts have been made to develop methods and apparatuses to repair or replace impaired heart valves.

Prostheses exist to correct problems associated with impaired heart valves. For example, mechanical and tissue-based heart valve prostheses can be used to replace impaired native heart valves. More recently, substantial effort has been dedicated to developing replacement heart valves, particularly tissue-based replacement heart valves that can be delivered with less trauma to the patient than through open heart surgery. Replacement valves are being designed to be delivered through minimally invasive procedures and even percutaneous procedures. Such replacement valves often include a tissue-based valve body that is connected to an expandable frame that is then delivered to the native valve's annulus.

Development of prostheses including but not limited to replacement heart valves that can be compacted for delivery and then controllably expanded for controlled placement has proven to be particularly challenging. An additional challenge relates to the ability of such prostheses to be secured relative to intralumenal tissue, e.g., tissue within any body lumen or cavity, in an atraumatic manner.

Delivering a prosthesis to a desired location in the human body, for example delivering a replacement heart valve to the mitral valve, can also be challenging. Obtaining access to perform procedures in the heart or in other anatomical locations may require delivery of devices percutaneously through tortuous vasculature or through open or semi-open surgical procedures. The ability to control the deployment of the prosthesis at the desired location can also be challenging.

### SUMMARY

Examples of the present disclosure are directed to a delivery system, such as but not limited to a delivery system for a replacement heart valve. Further examples are directed to methods of use to deliver and/or controllably deploy a prosthesis, such as but not limited to a replacement heart valve, to a desired location within the body. In some configurations, a replacement heart valve and methods for delivering a replacement heart valve to a native heart valve, such as a mitral valve, an aortic valve, or a tricuspid valve, are provided.

In some implementations, a delivery system and method are provided for delivering a replacement heart valve to a native mitral valve location. The delivery system and method may utilize a transseptal approach. In some implementations, components of the delivery system facilitate bending of a delivery device of the delivery system to steer a prosthesis from the septum to a location within the native mitral valve. In some implementations, a capsule is provided for containing the prosthesis for delivery to the native mitral valve location. The capsule may also be configured to recapture the prosthesis after initial deployment if another target implantation location is desired. In other implementations, the delivery system and method may be adapted for delivery of implants to locations other than the native mitral valve.

A suture-based release mechanism adapted for use with a delivery device for delivery of an implant (e.g., replacement heart valve or valve prosthesis) may include dual coaxial sliding shafts or subassemblies. The inner shaft may be a manifold to which sutures or tethers (e.g., ends of suture loops of a continuous suture or tether strand) are attached. The outer shaft may include one or more release windows that pushes the sutures or tethers (e.g., ends of suture loops) off the manifold for release.

The suture-based release mechanism may be incorporated into the delivery device. In other words, a delivery device may include a suture-based release mechanism involving dual coaxial sliding shafts or subassemblies that operate in conjunction to facilitate transition of the implant between a tethered configuration and an untethered (e.g., released) configuration upon actuation of an actuator (e.g., rotatable knob) of a proximal handle of the delivery device. The delivery device may include multiple suture or tether portions that are fixedly attached to a distal end portion of the delivery device at one end and inserted through an opening of an implant and then releasably coupled to retention members at the distal end portion of the delivery device. Thus, the suture or tether portions are only connected at a distal end portion of the delivery device and do not extend to the proximal handle of the delivery device. The actuator of the proximal handle may be configured to cause translation of one of the dual coaxial sliding shafts with respect to the other.

In some configurations, a delivery device for delivering an implant includes a shaft assembly comprising a proximal end portion and a distal end portion. The proximal end portion of the shaft assembly includes a handle including at least one actuator. The delivery device also includes at least one suture (e.g., a plurality of suture portions). A first end of the at least one suture (e.g., each of the plurality of suture portions) is permanently coupled to the distal end portion of the shaft assembly. A second end of the at least one suture (e.g., each of the plurality of suture portions) is removably coupled to at least one retention member (e.g., tab, finger, hook) of the distal end portion of the shaft assembly after being inserted through a coupling member (e.g., hole, eyelet) of an implant. In use, actuation of the at least one actuator causes the second end of the at least one suture (e.g., each of the plurality of suture portions) to be decoupled from the at least one retention member of the distal end portion of the shaft assembly.

The delivery device may include additional shafts, lumens or subassemblies to facilitate delivery of the implant to a desired implantation site (e.g., an outer sheath subassembly, a rail subassembly, a mid-shaft subassembly, and/or a nose cone subassembly). An outer sheath subassembly may be adapted to recapture the implant in-situ and then redeploy the implant at a new implantation site. A rail subassembly may facilitate bending of the delivery device to reach the desired implantation site. A mid-shaft subassembly may be adapted to retain a portion of the implant in a compressed configuration until the desired implantation site is reached and the implant is ready to deploy. The nose cone subassembly may facilitate access to the desired implantation site and guidance of the delivery device to the desired implantation site. The delivery device may include a handle with actuators (e.g., knobs) adapted to control movement (axial, bending, rotational movement) of the various subassemblies of the delivery device. The implant may be a prosthetic replacement heart valve and the desired implantation site may be within an annulus of a native heart valve (e.g., mitral valve, tricuspid valve, aortic valve).

In some implementations, the suture-based release mechanism includes an outer release shaft or subassembly having a proximal end and a distal end and an inner manifold shaft or subassembly having a proximal end and a distal end. The manifold shaft is coaxially positioned within the release shaft. The suture-based release mechanism may include a plurality of suture portions (which may be formed of a continuous piece of suture or tether wire) adapted to be removably tethered to an implant (e.g., inserted through an opening of or wrapped around a feature of a valve prosthesis, such as a replacement heart valve). The plurality of suture loops may be coupled to the manifold shaft. For example, a first end of each of the plurality of suture portions (e.g., loops) may be adapted to be removably coupled to at least one suture loop receiving member (e.g., tab, peg, finger) of the manifold shaft that is positioned proximal of the distal end (e.g., terminus) of the manifold shaft. A second end of each of the plurality of suture loops may be permanently (e.g., non-removably) coupled to the distal end of the manifold shaft. Relative sliding movement of the manifold shaft with respect to the release shaft from a locked configuration to an unlocked configuration causes release of the first end of each of the plurality of suture loops from the at least one suture loop receiving member, thereby allowing the first end of each of the plurality of suture loops to be untethered from the implant.

The relative sliding movement may include movement of the manifold shaft distally while the release shaft is stationary. The suture-based release mechanism (or delivery device comprising the release mechanism) may include a spring in the handle of the delivery device that is configured to keep the release mechanism in the locked configuration by default, wherein the spring exerts a distal spring force on the release shaft that must be overcome to transition the release mechanism to the unlocked configuration.

The at least one suture loop receiving member may comprise a plurality of tabs arranged circumferentially around the distal portion of the manifold shaft, wherein each of the plurality of tabs is adapted to receive a first end of at least one of the plurality of suture loops. A distal portion of the release shaft may include a plurality of windows, wherein each window of the plurality of windows is adapted to align with a respective one of the plurality of tabs of the manifold shaft. Sliding movement of the manifold shaft in a distal direction while keeping the release shaft fixed in position may cause a distal edge of each window of the release shaft to push the second end of each suture loop proximally along a respective one of the plurality of tabs of the manifold shaft until the second end of each suture loop is released from the respective one of the plurality of tabs, thereby allowing the implant (e.g., replacement heart valve) to be decoupled from the delivery device.

The at least one suture loop receiving member (e.g., tab, peg, finger) of the manifold shaft may reside within a respective opening or window proximal of the distal end of the manifold shaft. The second end of each of the plurality of suture loops may be permanently, or non-removably, coupled to a cog at the distal end of the manifold shaft including a plurality of tether cleats and then permanently glued or sealed between suture retention rings positioned on both sides of the tether cleats.

The plurality of suture loops may include three, four, five, six, seven, eight, nine, or more suture loops. The number of suture loops may correspond to the number of proximal eyelets (or other opening) located on a proximal end of the implant. During assembly, the first end of each of the plurality of suture loops may be inserted through a respective eyelet of the proximal end of the implant before being threaded through a release window of the release shaft and removably coupled to the at least one suture loop receiving member of the manifold shaft.

In one implementation with nine suture loops, the at least one suture loop receiving member (e.g., tab, peg, finger) of the manifold shaft or subassembly may comprise three tabs arranged circumferentially around the distal portion of the manifold shaft, wherein each of the three tabs is adapted to receive a first end of one or more of the plurality of suture loops. In this implementation, each tab receives three first ends of three suture loops. In this implementation, a distal portion of the release shaft may include three windows, wherein each window of the three windows is adapted to align with a respective one of the three tabs of the manifold shaft. In such an implementation, a second end of each of the nine suture loops may be non-removably coupled to a cog at the distal end of the manifold shaft. A portion of each of the nine suture loops may be looped through a respective eyelet positioned at a proximal end of the replacement heart valve. Sliding movement of the manifold shaft in a distal direction while keeping the release shaft fixed in position causes a distal edge of each window of the release shaft to push the second end of each of the nine suture loops proximally along a respective one of the three tabs of the manifold shaft until the second end of each of the nine suture loops is released from the respective one of the three tabs, thereby allowing the implant (e.g., replacement heart valve) to be decoupled from the delivery device.

The release shaft may include at least one radially inwardly-protruding retention member configured to be received within at least one slot of the manifold shaft so as to prevent rotation of the release shaft with respect to the manifold shaft to thereby maintain alignment of each window with a respective tab. Each of the plurality of tabs may have the substantially the same length or a different length.

In accordance with several implementations, a method of making or manufacturing a suture-based release mechanism to facilitate delivery of an implant includes permanently attaching a first end of a suture loop to a distal end of an inner tube, threading a free second end of the suture loop through a hole of the implant, inserting the free second end of the suture loop through a window positioned along a distal end portion of an outer tube coaxially surrounding the inner tube, placing the free second end of the suture loop onto a tab positioned along a distal end portion of the inner tube to removably couple the free second end of the suture loop to the tab, and causing the distal end of the outer tube to be advanced distally to align with the distal end of the inner tube such that the second end of the suture loop is prevented from coming off of the tab until the implant is in a desired position for implantation.

In accordance with several implementations, a method of making a suture-based release mechanism to facilitate delivery of an implant includes permanently attaching a first end of a suture loop to a distal end of an inner tube, threading a loop end of the suture loop through a hole of the implant, inserting the loop end of the suture loop through a slot positioned along a proximal tether retention component at a distal portion of the inner tube to removably couple the loop end of the suture loop to the proximal tether retention component, and inserting a free end of a release suture through the loop end of the suture loop to secure the suture loop to the inner tube.

The process described above may be repeated for multiple suture loops formed from a single continuous suture or tether strand. The distal end of the inner tube may comprise multiple tether cleats spaced apart circumferentially. These tether cleats may form a plurality of proximal members that the single continuous suture or tether strand is wrapped around to form a plurality of proximal suture loop ends. An assembly member may include a plurality of circumferentially- spaced apart pegs or cleats to form a plurality of distal members that the single continuous suture or tether strand is wrapped around to form a plurality of distal suture loop ends. The words "suture" and "tether" may be used interchangeably herein.

The proximal suture loop ends and the distal suture loop ends may be circumferentially offset from each other such that each strand portion connects a proximal suture loop end to a circumferentially offset distal suture loop end in an alternating serpentine fashion. For example, a strand is wrapped around a first proximal member to form a first proximal suture loop end and then brought back down to a first distal member that may be spaced apart (or offset circumferentially) from the first proximal member and wrapped around the first distal member to form a second suture loop end (a first distal suture loop end) and then brought back up to a second proximal member spaced apart (or offset circumferentially) from the first distal member to form a third suture loop end (a second proximal suture loop end) in a serpentine fashion. This process is repeated until the desired number of suture loop ends have been created. The two ends of the single continuous suture or tether strand may be knotted together (and optionally glued or otherwise adhered together) after forming the multiple suture loops and coupling them to eyelets or other retention members on a proximal end of the implant.

In accordance with several implementations, a method of facilitating delivery of an implant within a body of a patient using a suture-based release mechanism includes advancing a distal end portion of a delivery device to a desired implantation location. The delivery device includes dual, coaxial sliding shafts (e.g., an inner shaft and an outer shaft). At least one suture loop is pre-attached to the implant during manufacture of the delivery device and a first end of the suture loop is non-removably coupled to a distal end of an inner shaft of the dual, coaxial sliding shafts during manufacture of the delivery device. A second end of suture loop is removably coupled to a suture retention member of the manifold after having been inserted through a retention member (e.g., cyclet) of the implant. A distal end portion of an outer shaft of the two shafts includes a release window adapted to push the second end of the suture loop off of the suture retention member upon relative sliding movement of the inner shaft with respect to the outer shaft. The method also includes advancing the inner shaft distally with respect to the outer shaft so as to cause decoupling of the second end of the suture loop from the suture retention member and out of the release window and withdrawing the shafts to allow the second end of the suture loop to be decoupled from the retention member of the implant, thereby allowing the implant to remain in the desired implantation location when the delivery system is removed from the patient.

In some implementations, a loop end of the suture loop is inserted through a slot of a proximal tether retention component of the inner shaft after having been inserted through a retention member (e.g., proximal-most eyelet of an inflow strut of a frame) of the implant. A release suture may be inserted through the loop end of the suture loop after the loop end of the suture loop is inserted through the slot. The method may also include advancing the inner shaft distally with respect to the outer shaft, withdrawing the release suture from the loop end of the suture loop, and decoupling the loop end of the suture loop from the retention member of the implant, thereby allowing the implant to remain in the desired implantation location when the delivery device is removed from the patient.

During implant delivery, the outer release shaft or subassembly may be kept in a distal position by a spring in the handle at the proximal end of the delivery device, securing the suture loop(s) to the inner manifold shaft or subassembly. When the user advances a manifold/release knob of the handle, the outer release shaft moves forward with the inner manifold shaft via the biased compression spring force of the spring until a release shaft handle adapter hits a hard stop member in the handle. Continued advancement of the inner manifold shaft extends the inner manifold shaft distally while the outer release shaft stays in place due to contact with the hard stop member in the handle. The distal edge of a release shaft window abuts the suture loop end and pushes it proximally, releasing it from a suture receiving member (e.g., tab, finger, peg) on the underlying inner manifold shaft. Retraction of the release and manifold shafts (e.g., by rotating the manifold/release knob proximally) unthreads or uncouples the suture loops from the valve eyelets. The suture loops are removed from the body with the delivery system. The suture loops may be formed from a single continuous tether strand in which the two ends of the continous tether strand are knotted and glued together after forming the suture loops.

In accordance with several configurations, a valve prosthesis adapted for non-uniform compression during loading into a capsule includes a self-expanding frame configured to transition between a compressed configuration and an expanded configuration. The frame includes at least one row of cells forming a ring. The valve prosthesis also includes a plurality of prosthetic valve leaflets coupled to the frame. The frame includes a plurality of pre-curved axial connection portions, each axial connection portion extending between a top end and bottom end of each cell of the at least one row of cells. Each axial connecting portion is adapted to bend in a predetermined manner for accommodating changes in cell height during non-uniform compression of the valve prosthesis.

In accordance with several configurations, a valve prosthesis includes a self-expandable frame configured to transition between a compressed configuration and an expanded configuration. The frame includes a plurality of rows of cells formed by struts, wherein the cells form a chevron-shaped cell structure. At least one cell of a distal-most row of the plurality of rows of cells includes an axial strut connecting a distal apex of the cell with a distal apex of a bordering cell in a row immediately above the distal-most row. The axial strut includes a bow-spring structure adapted to prevent cell ovality during the transition between the compressed configuration and the expanded configuration, and vice-versa.

The bow-spring structure may include a dual bow-spring structure in which the axial strut comprises two axial strut segments connected at their proximal and distal ends but separated along their lengths. Each of the cells of the distal-most row may include an axial strut connecting a distal apex of the respective cell with a distal apex of a respective bordering cell in a row immediately above the distal-most row. Each of the axial struts of the cells of the distal-most row comprises a bow-spring structure adapted to prevent cell ovality during the transition between the compressed configuration and the expanded configuration, and vice-versa. The bow-spring structures may be asymmetric or symmetric.

In accordance with several configurations, a dual-frame valve prosthesis includes an inner frame including an inflow portion having an inflow end, an outflow portion having an outflow end, and an intermediate portion extending between the inflow portion and the outflow portion. The inflow end of the inner frame includes a plurality of inflow struts (e.g., axial proximal struts or beams) including a plurality of eyelets (e.g., two, three or more eyelets). The outflow end of the inner frame includes a plurality of anchors (e.g., distal anchors or ventricular anchors). The valve prosthesis also includes an outer frame including an inflow portion having an inflow end, an outflow portion including an outflow end, and an intermediate portion extending between the inflow portion and the outflow portion. The inflow end of the outer frame includes a plurality of inflow struts (e.g., axial proximal struts or beams) including a plurality of eyelets. At least one of the plurality of eyelets of each of the plurality of inflow struts of the outer frame is configured to engage with at least one of the plurality of eyelets of the plurality of inflow struts of the inner frame.

The valve prosthesis may also include a skirt assembly positioned between the inner frame and the outer frame. The skirt assembly includes an integral piece of cloth material with varying diameters, the integral piece of cloth material including a body portion, a plurality of proximal extensions extending from the body portion, and a plurality of distal extensions extending from the body portion. In some configurations, the plurality of proximal extensions is positioned between the inflow portion of the inner frame and the inflow portion of the outer frame. The body portion of the skirt assembly may be positioned external to the intermediate portion of the outer frame. The plurality of distal extensions may be positioned between the outflow portion of the inner frame and the outflow portion of the outer frame.

In some implementations, one or more of the plurality of proximal extensions include a tab configured to be positioned between one or more of the plurality of inflow struts of the inner frame and one or more of the plurality of inflow struts of the outer frame. In some implementations, one or more of the plurality of distal extensions include a hole configured to allow blood to flow into a volume between the inner frame and the outer frame.

In some implementations, the plurality of proximal extensions and/or the plurality of distal extensions comprise a trapezoidal shape. In some implementations, the plurality of proximal extensions is sewn together via one or more sutures when the valve prosthesis is assembled. In some implementations, the plurality of distal extensions is sewn together via one or more sutures when the valve prosthesis is assembled.

The one or more sutures may include at least one interlock stitch instead of a knot. At least one edge of the cloth material of the skirt assembly may be melted (e.g., using laser or soldering iron) to create a smooth edge surface. In some implementations, a valve assembly is positioned within the inner frame, the valve assembly including a plurality of prosthetic leaflets, wherein a cusp of each of the plurality of prosthetic leaflets is sutured to the skirt assembly using two different stitch lines (e.g., double stitch line).

In some configurations, the inflow struts of the outer frame each include a bendable tab that is unattached to the inflow strut of the outer frame along at least a portion of the bendable tab such that the bendable tab can bend along an independent plane from the respective inflow strut of the outer frame. The bendable tab may include at least one eyelet that is configured to engage with at least one of the plurality of eyelets of the plurality of axial inflow struts of the inner frame.

In some implementations, the inflow end of the outer frame and the inflow end of the inner frame are mechanically attached together via a dovetail joint configuration or a "puzzle piece" fit configuration.

In some implementations, the inflow struts of the inflow end of the outer frame and the inflow struts of the inflow end of the inner frame are attached together and proximal-most ends of at least two of the axial inflow struts are configured to be positioned at an offset distance from each other (e.g., staggered heights). Each adjacent inflow strut may be offset or they may be offset in pairs or other numbered groups.

In some implementations, at least some of the plurality of anchors include an attachable anchor dampener that does not comprise foam. The attachable anchor dampener may be configured to have a first portion configured to engage a native heart valve leaflet. The first portion may be more rigid than a second portion configured to contact a septal wall or annulus of a heart. The second portion may be configured to provide a cushioned contact surface.

In some implementations, at least some of the plurality of anchors include a metallic cushion anchor tip configured to distribute and dampen a load exerted on native tissue in contact with the anchor tip. The metallic cushion anchor tip may include a nitinol material. In one configuration, the metallic cushion anchor tip is a whisk configuration formed from a plurality of wire hoops.

In some implementations, at least some of the plurality of anchors include an anchor tip that is configured to provide a cushioning effect in a radially outward direction to reduce a likelihood of conduction disturbances caused by the anchor in contact with a septal wall of a heart and to provide rigidity in a radially inward direction to facilitate capture of native heart valve leaflets.

In accordance with several configurations s, a dual-frame valve prosthesis comprising co-organizing features to facilitate alignment and registration during compression and expansion of the dual frames of the dual-frame valve prosthesis includes an inner frame and an outer frame comprising one or more co-organizing features (e.g., a hammer-head proximal eyelet design and/or the distal apexes of the inner frame and the outer frame are circumferentially offset).

### BRIEF DESCRIPTION OF THE DRAWINGS

Figure 1 shows an embodiment of a delivery system for an implant, such as a dual-frame heart valve prosthesis.
Figure 2 shows a perspective view of a dual-frame valve prosthesis that may be delivered using the delivery system described herein.
**Figure 2A** shows a side view of an inner frame of the dual-frame valve prosthesis of **Figure 2****.**
**Figure 2B** shows a side view of an outer frame of the dual-frame valve prosthesis of **Figure 2****.**
**Figure 2C** shows a side perspective view of a fully-assembled dual-frame valve prosthesis including a skirt assembly and padding.
**Figures 2D-1 to 2D-3** illustrate how structural instability (e.g., strut buckling) can occur during compression of a standard chevron-cell frame structure.
**Figures 2E-1 to 2E-4** illustrate various views of an embodiment of an inner frame having asymmetric "bow spring" structural mechanisms in compressed, partially-compressed, and expanded configurations.

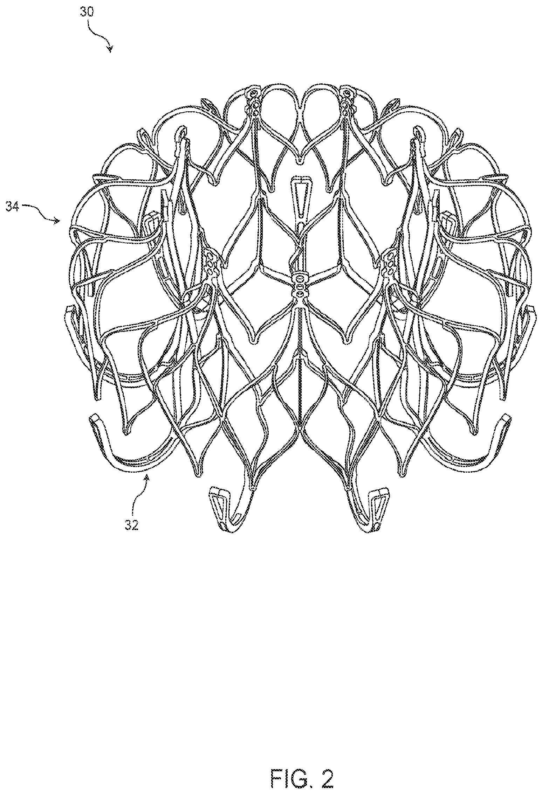

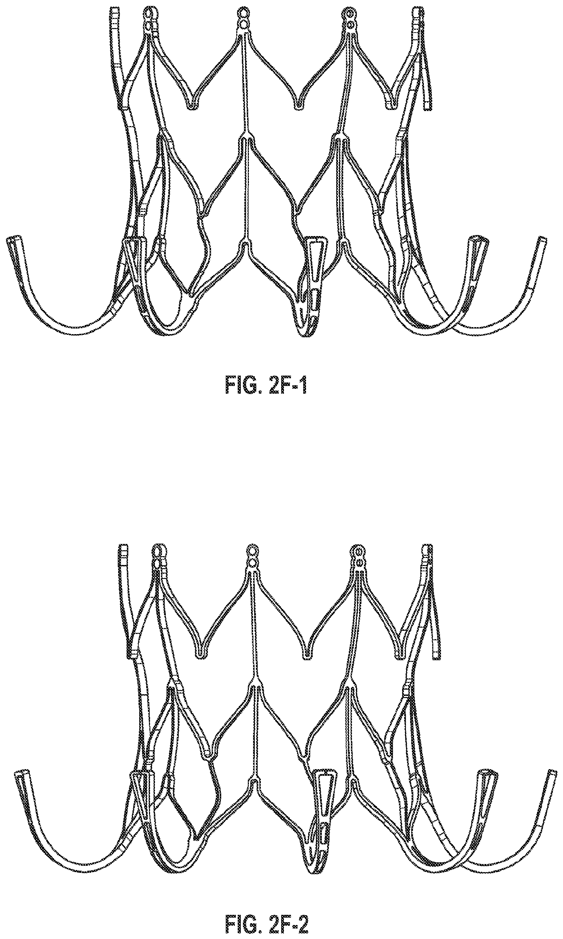

**Figures 2F-1 and 2F-2** illustrate embodiments of inner frames having highly asymmetric "bow spring" structural mechanisms and minimally asymmetric "bow spring" structural mechanisms, respectively.
**Figures 2G-1, 2G-2, and 2G-3** show various views of an embodiment of an inner frame having symmetric "bow spring" structural mechanisms.
**Figures 2G-4A, 2G-4B, 2G-5, 2G-6, 2G-7, 2G-8, 2G-9A, 2G-9B, 2G-10, 2G-11A, 2G-11B, 2G-11C, 2G-12, 2G-13, 2G-14, 2G-15, 2G-16, and 2G-17** illustrate various views of embodiments of anchor tips of a frame of a replacement heart valve, such as an inner frame of a dual-frame valve prosthesis.
**Figures 2G-18A, 2G-18B, 2G-19, 2G-20A, 2G-20B, 2G-21A, 2G-21B, 2G-22, 2G-23A, 2G-23B, 2G-24A, 2G-24B, 2G-25A, 2G-25B, 2G-26A, 2G-26B, 2G-26C, 2G-27A and 2G-27B** illustrate various views of embodiments of an anchor tip of a frame of a replacement heart valve, such as an inner frame of a dual-frame valve prosthesis.

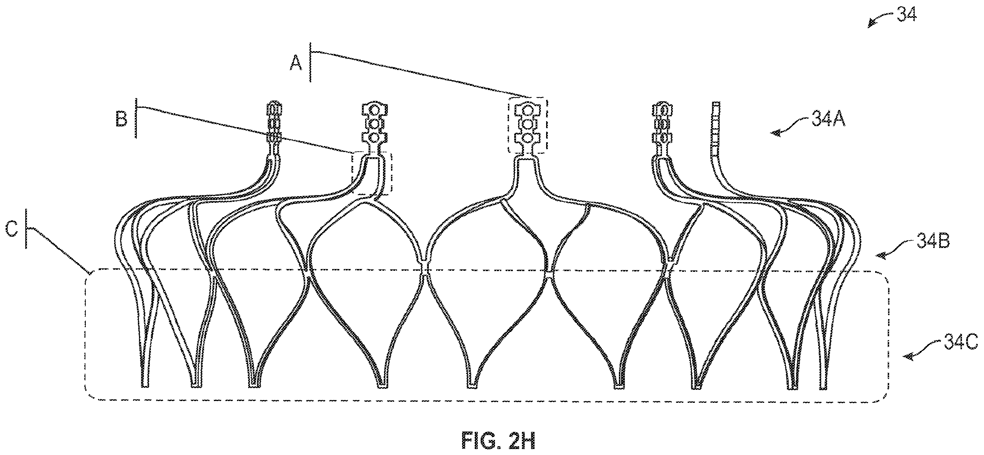

**Figure 2H** shows a side view of an embodiment of an outer frame including co-organizing frame features to facilitate improved operation with the inner frames described herein throughout transitory loading and deployment configurations.

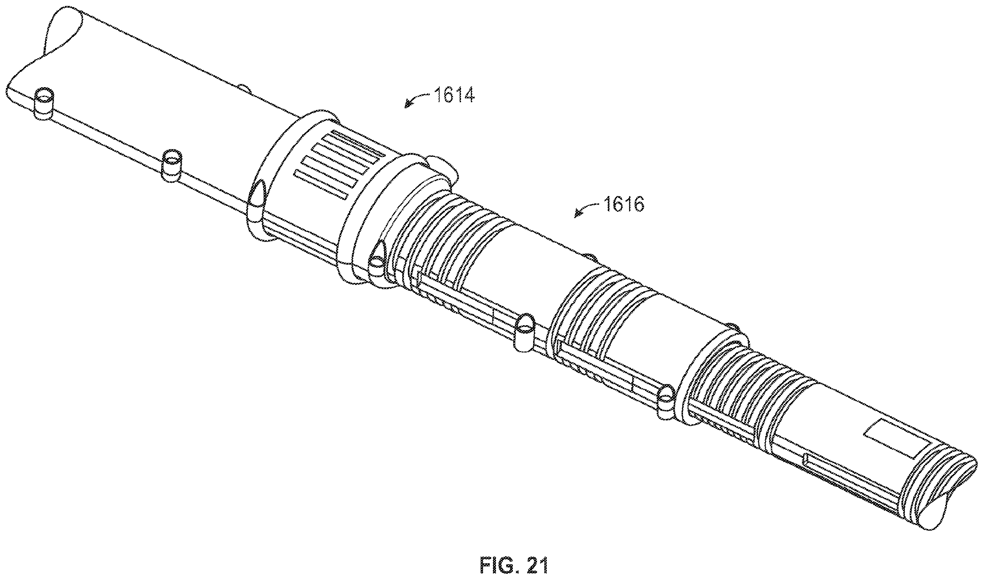

**Figures 21-1 to 2I-3** illustrate various eyelet designs configured to reduce rotational and/or translational movement between an outer frame and inner frame of a dual-frame valve prosthesis.

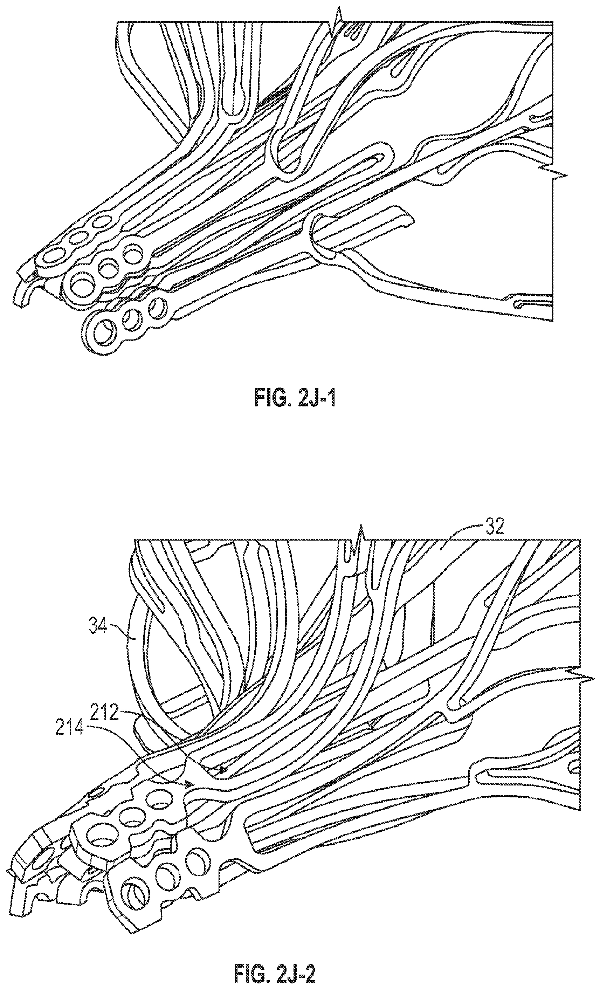

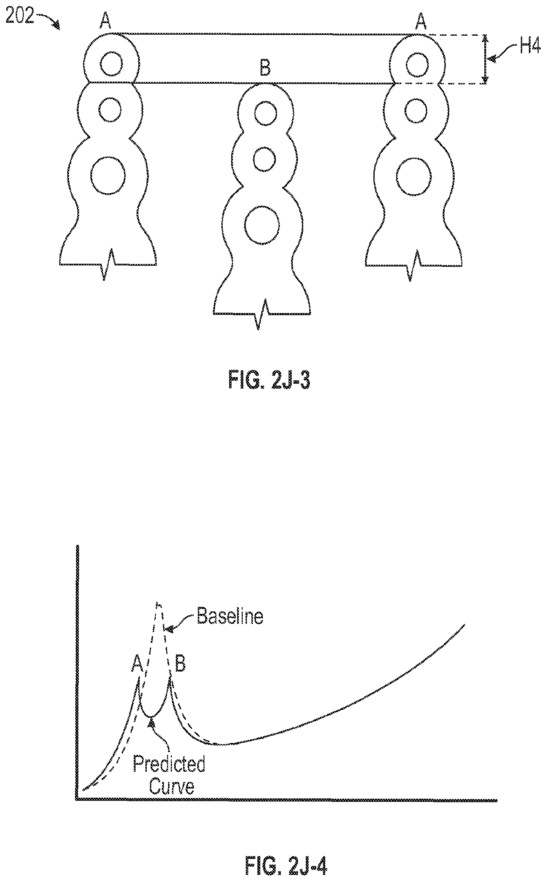

**Figure 2J-1** illustrates an outer frame without certain co-organizing frame features. **Figure 2J-2** illustrates an outer frame having a co-organizing feature designed to straddle an inner frame axial strut to facilitate alignment.
**Figures 2J-3 and 2J-4** illustrate another embodiment of a frame of a heart valve prosthesis where heights of proximal-most struts (e.g., tether attachment struts) of the frame are alternately varying or are offset.

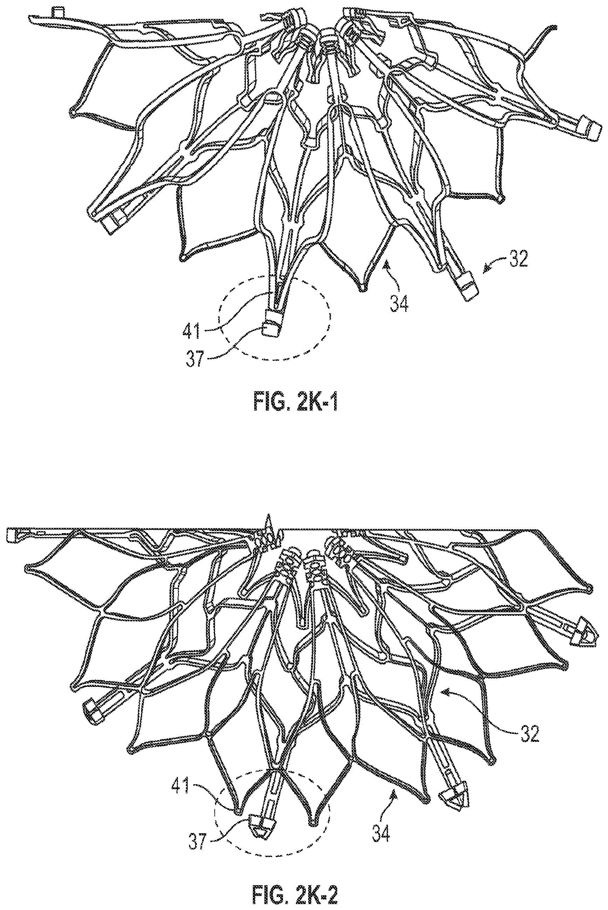

**Figure 2K-1** illustrates how an outer frame can adversely interact with an anchor on an inner frame of a dual-frame valve prosthesis during crimping. **Figure 2K-2** shows how an implementation of an outer frame can be designed such that distal outflow portions of the outer frame avoid interaction with inner frame anchors during crimping.
**Figures 2L-1 to 2L-3** illustrates various implementations of an outer frame design of a dual-frame valve prosthesis showing various options of a connection or attachment structure between the proximal eyelets and the connecting struts of the outer frame.

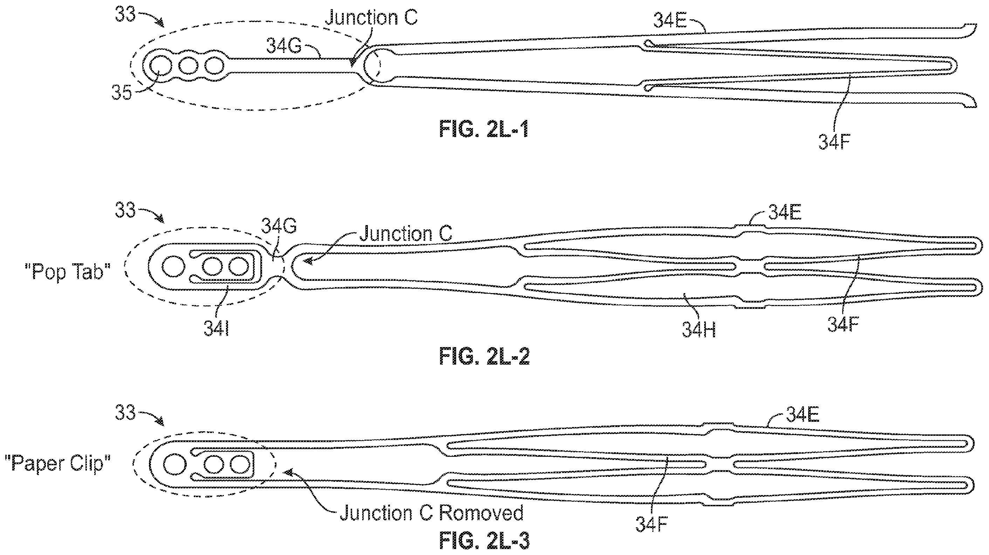

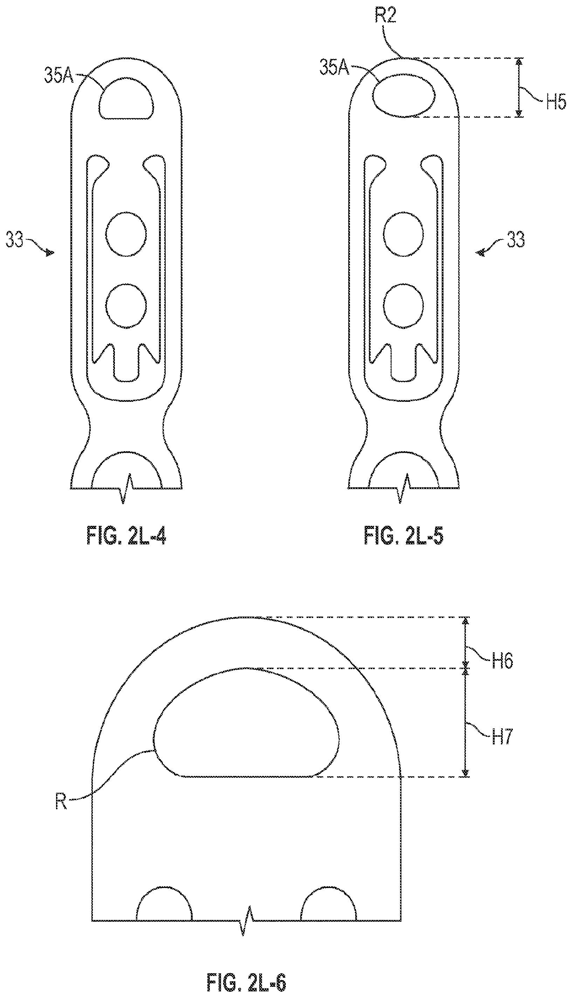

**Figures 2L-4 to 2L-6** illustrate various embodiments of tabs and/or eyelets of a frame, such as an outer frame of a dual-frame valve prosthesis.

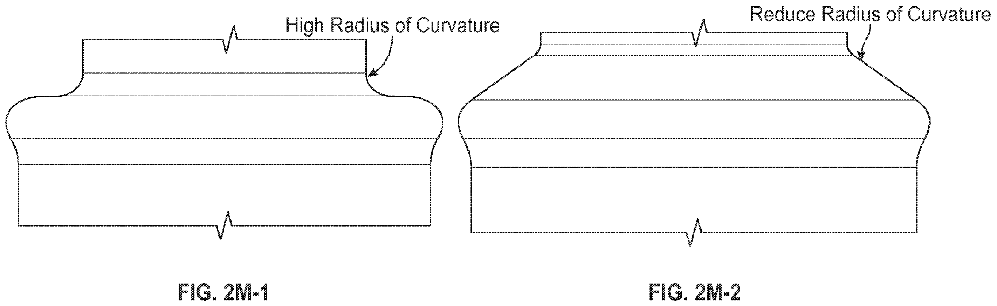

**Figures 2M-1 and 2M-2** illustrate various implementations of a dual-frame valve prosthesis having various radii of curvature profiles when an inner frame and an outer frame are engaged.

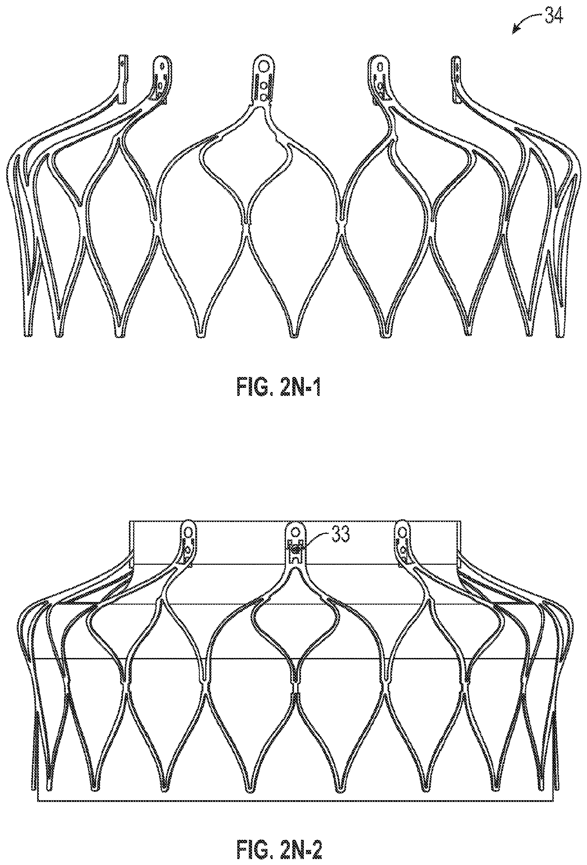

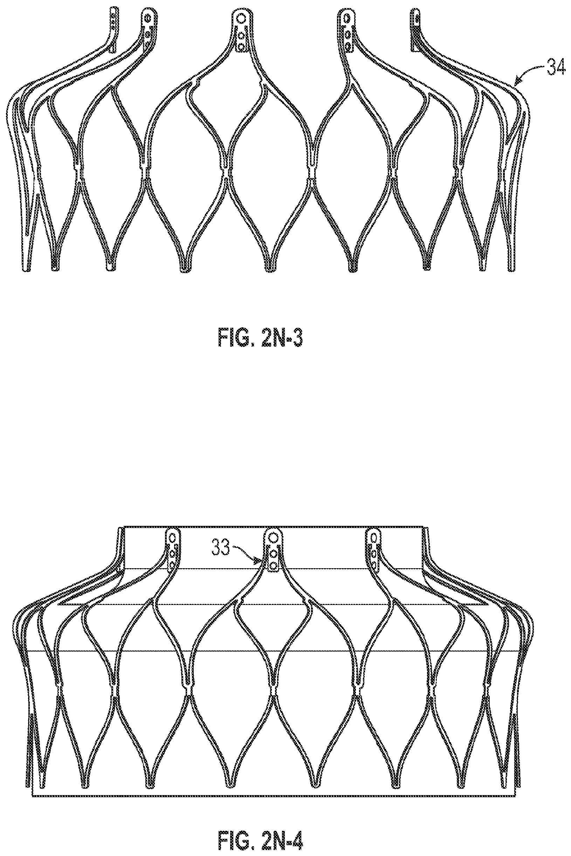

**Figures 2N-1 and 2N-2** illustrate one example of an outer frame. **Figures 2N-3 and 2N-4** illustrate another example of an outer frame.

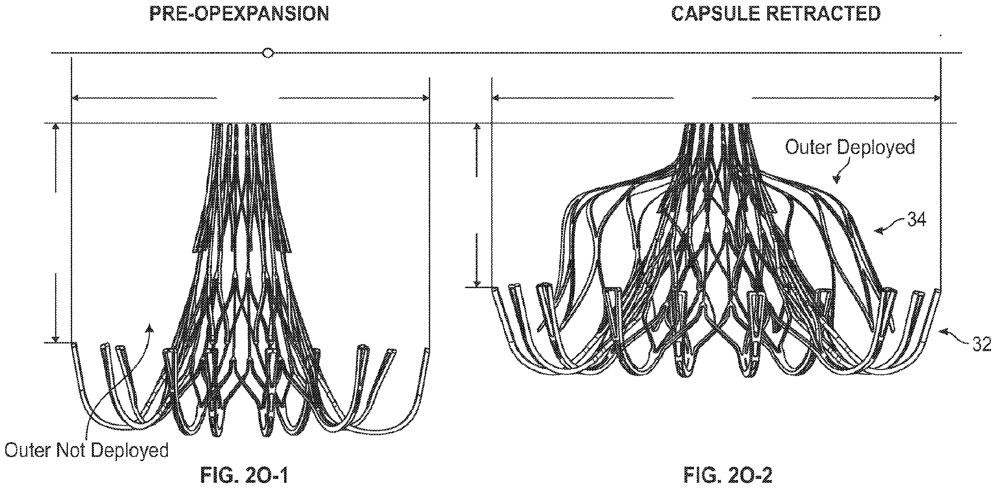

**Figure 20-1** illustrates a dual-frame valve prosthesis in which an inner frame and an outer frame are engaged in a pre-expansion state where the outer frame is not deployed. **Figure 2O-2** illustrates a dual-frame valve prosthesis in which an inner frame and an outer frame are engaged in a capsule retracted state where the outer frame is deployed.
**Figures 2P-1 to 2P-7** illustrate various embodiments of engaging an inner frame and an outer frame for forming the dual-frame heart valve prosthesis.

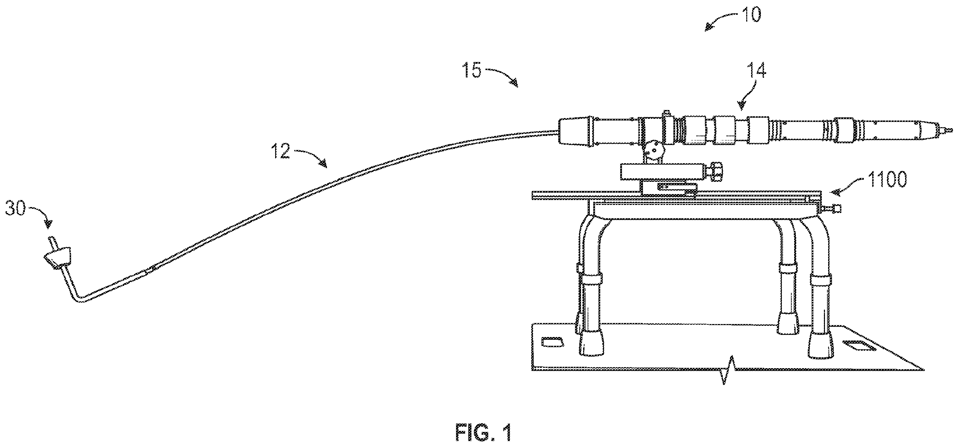

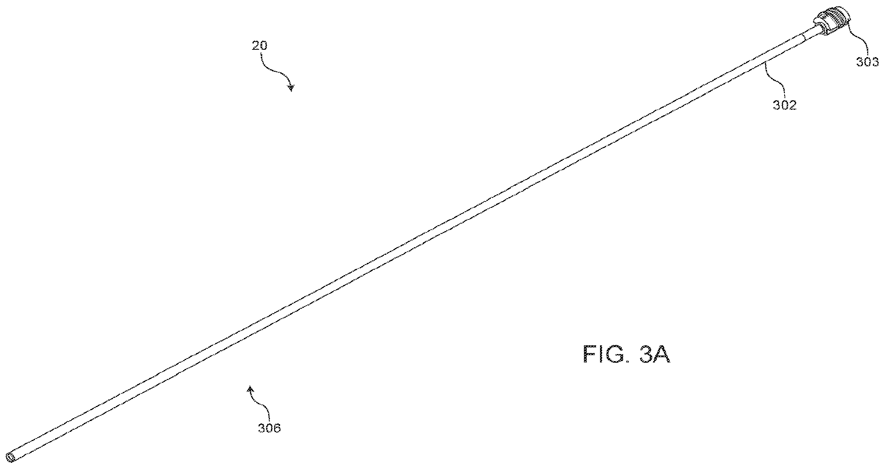

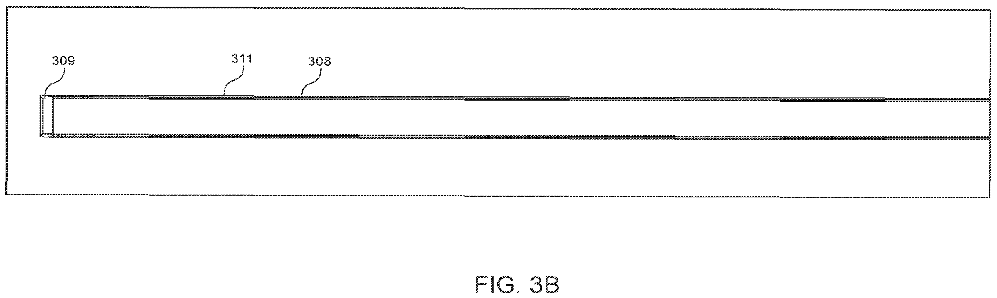

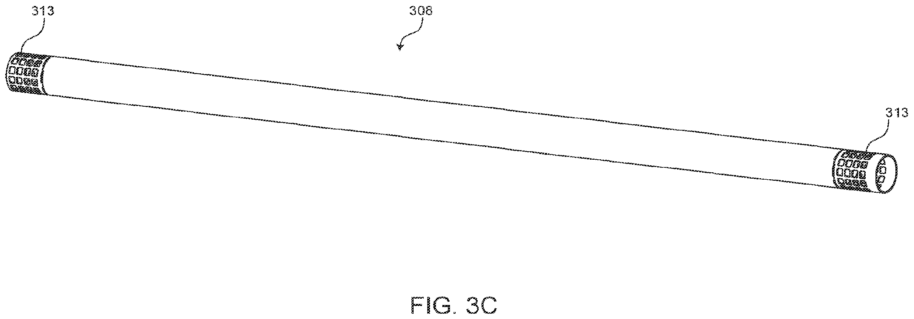

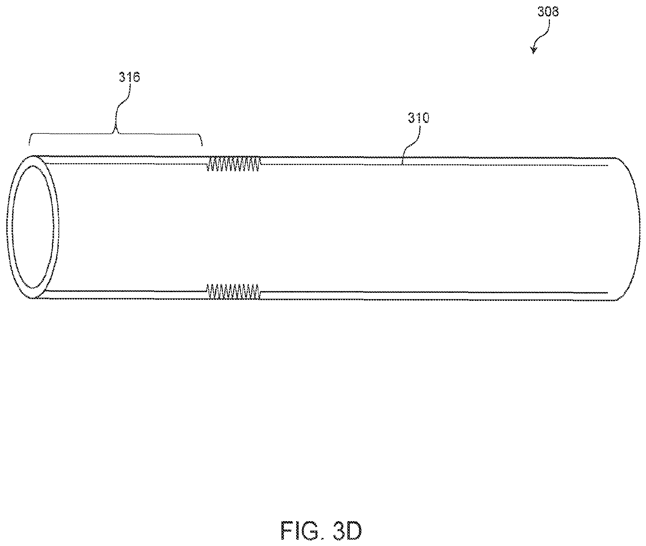

**Figure 3A** shows a perspective view of an embodiment of an outer subassembly of a delivery device of the delivery system of **Figure 1****.** **Figure 3B** illustrates a side-cross-section view of a capsule subassembly of the outer sheath subassembly of **Figure 3A****.** **Figure 3C** shows a perspective view of a capsule stent, or distal hypotube, of the outer sheath subassembly of Figure 3A. **Figure 3D** shows how a portion of a liner extending along a length of the outer sheath subassembly can have built-in slack to facilitate flexible bending of the outer subassembly.

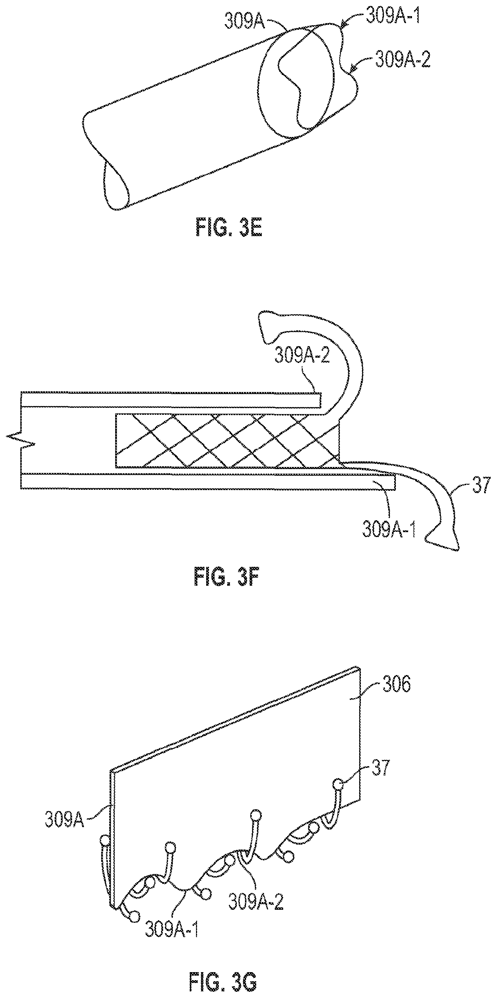

**Figures 3E to 3G** illustrate another embodiment of a distal capsule tip of a capsule subassembly.

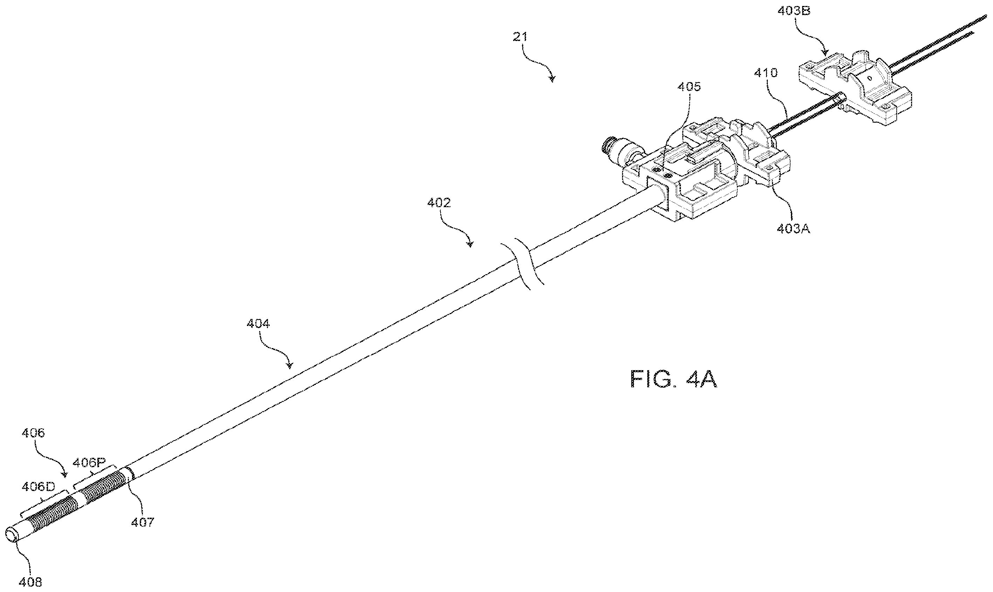

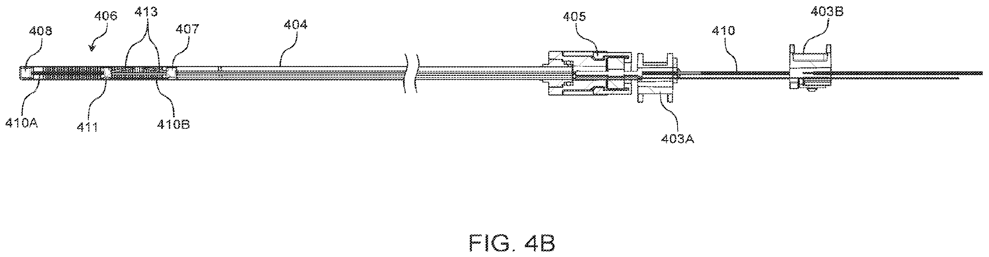

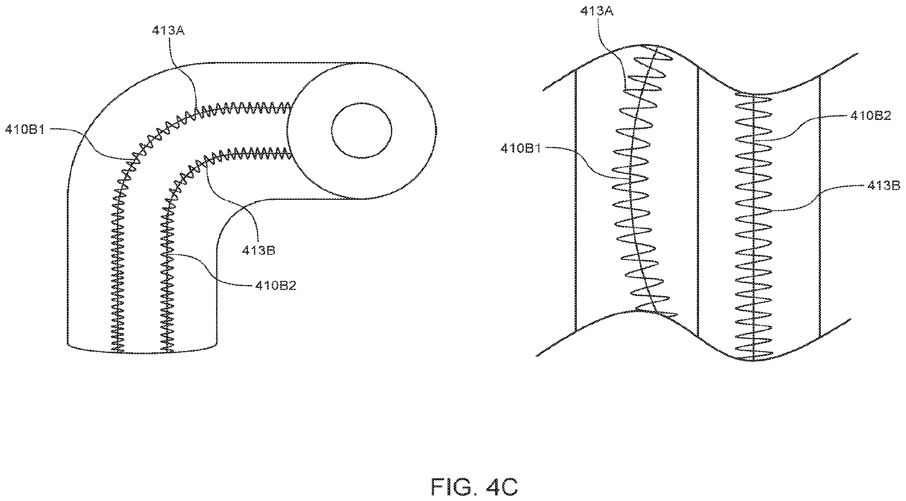

**Figure 4A** shows a perspective view of a rail subassembly of the delivery device of the delivery system of **Figure 1****.** **Figure 4B** shows a side cross-section view of the rail subassembly of **Figure 4A****.** **Figure 4C** schematically illustrates how an outer compression coil and pull wire can have a longer length than an inner compression coil and pull wire of the rail subassembly. **Figures 4D-1 and 4D-2** schematically illustrates thru-wall welding techniques performed during manufacture of the rail subassembly (as compared to prior direct welding techniques).

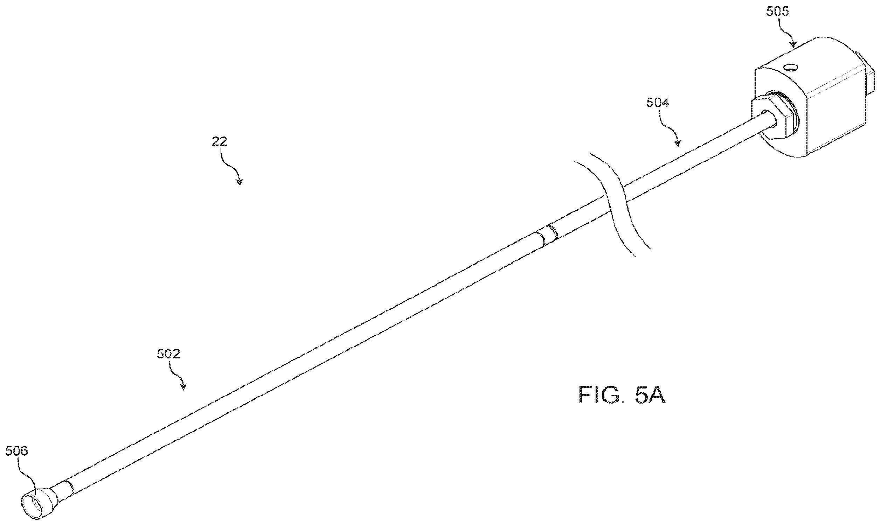

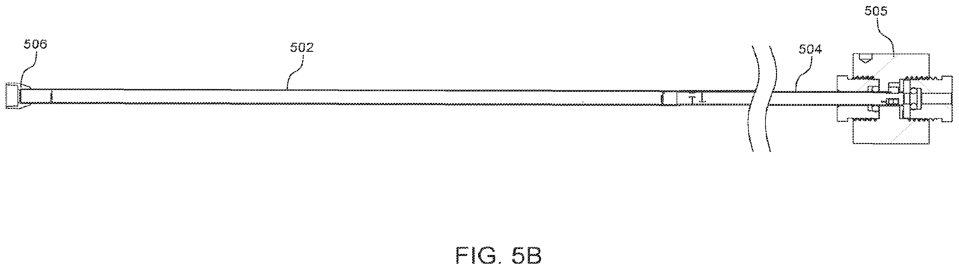

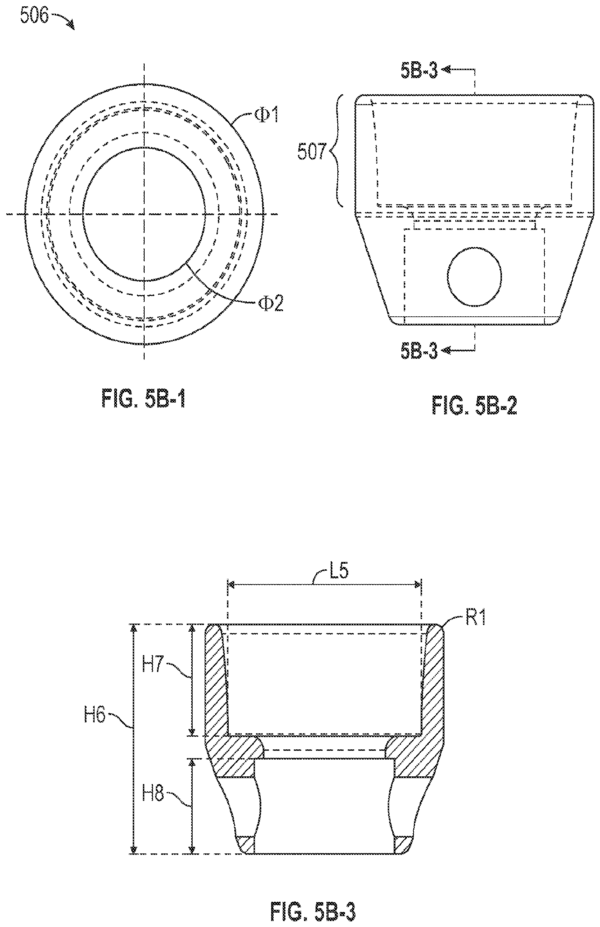

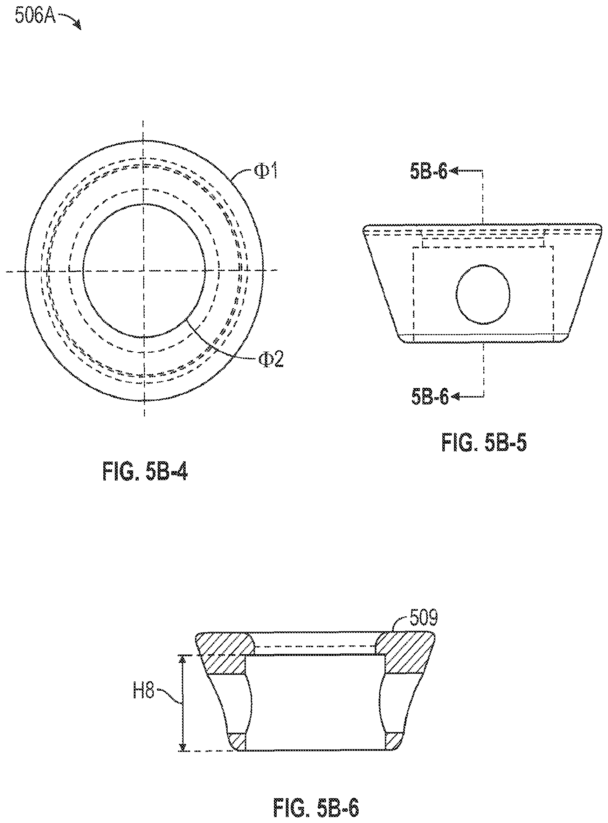

**Figure 5A** shows a perspective view of a mid-shaft subassembly of the delivery device of the delivery system of **Figure 1****.** **Figure 5B** illustrates a side cross-section view of the mid-shaft subassembly of **Figure 5A****.**

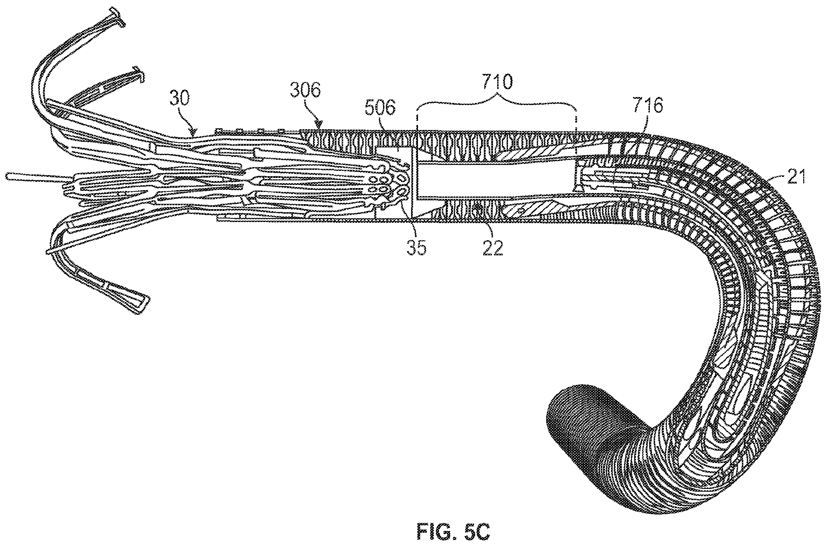

**Figures 5B****-1 to 5B-3** illustrate an embodiment of a distal end of the mid-shaft subassembly. **Figures 5B****-4 to 5B-6** illustrate another embodiment of a distal end of the mid-shaft subassembly. **Figure 5C** illustrates a side cross-section view of a distal end portion of the shaft assembly, including the mid-shaft subassembly.

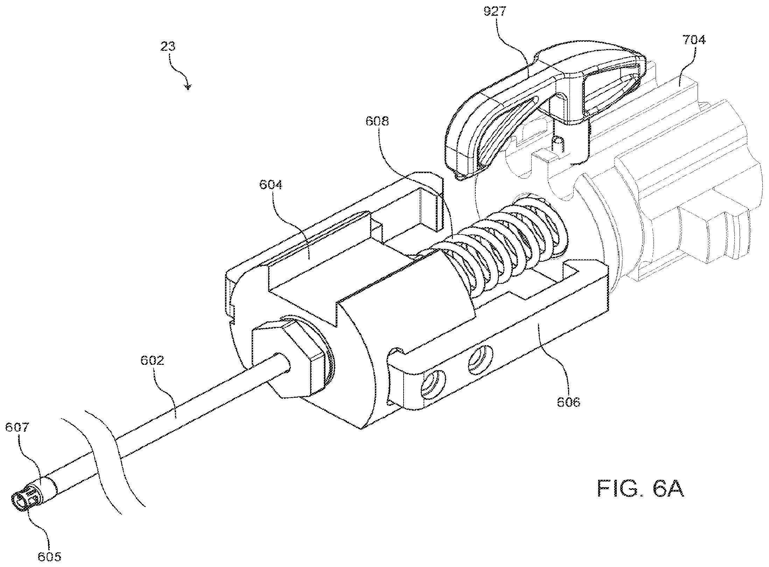

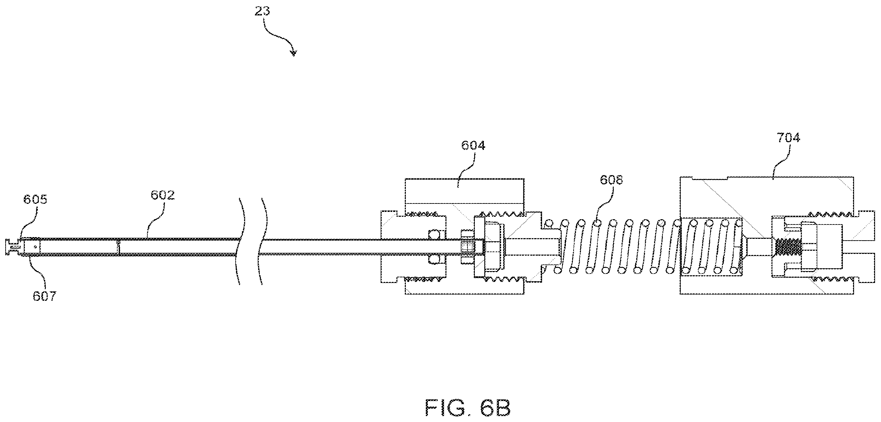

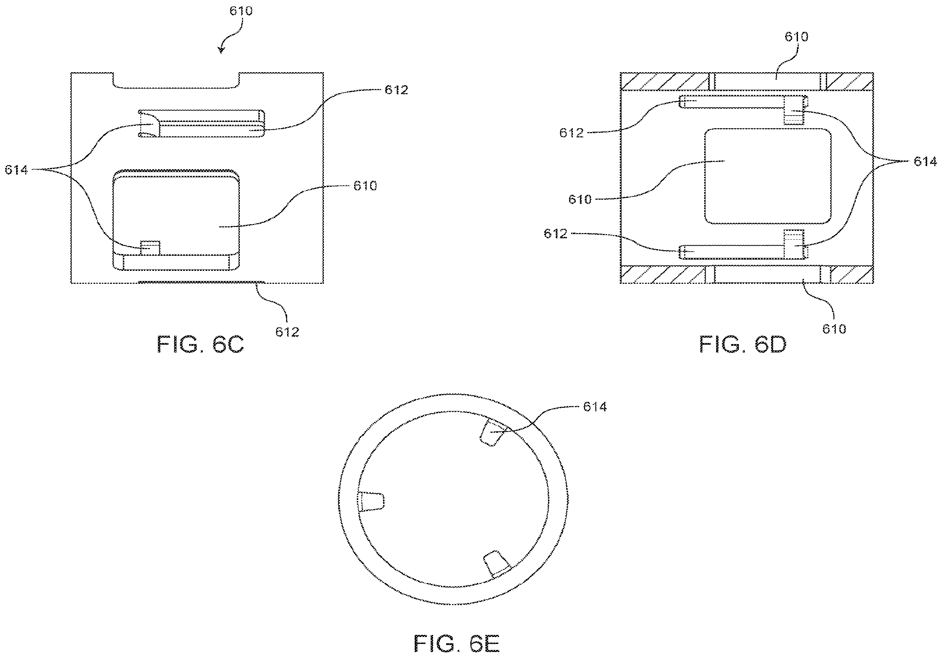

**Figure 6A** shows a perspective view of a release subassembly of the delivery device of the delivery system of **Figure 1****.** **Figure 6B** shows a side cross-section view of the release subassembly of **Figure 6A****.** **Figure 6C** shows a close-up side view of a distal end portion of the release subassembly. **Figure 6D** shows a side cross-section view of the distal end portion of the release subassembly. **Figure 6E** shows a bottom view of the distal end of the release subassembly.

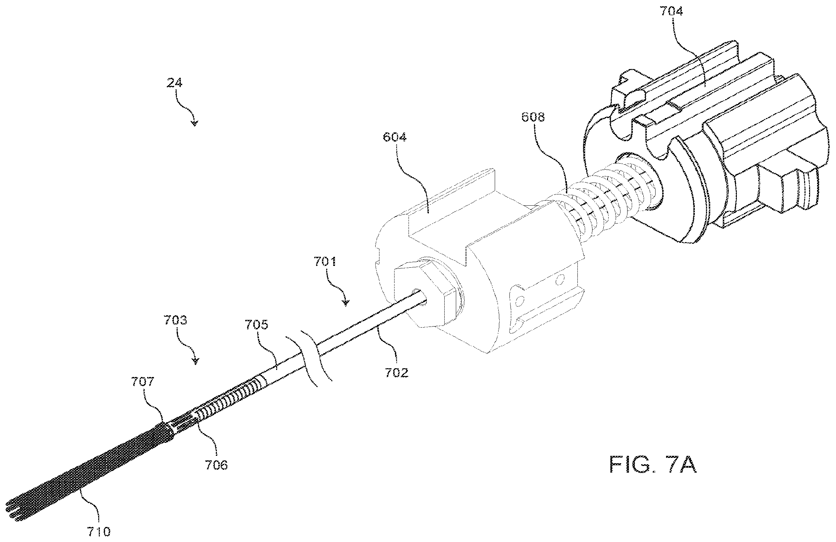

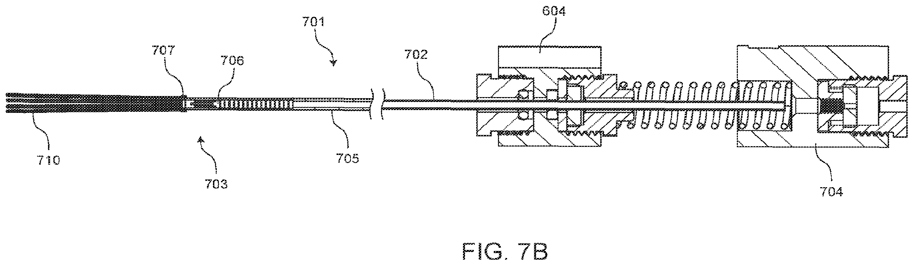

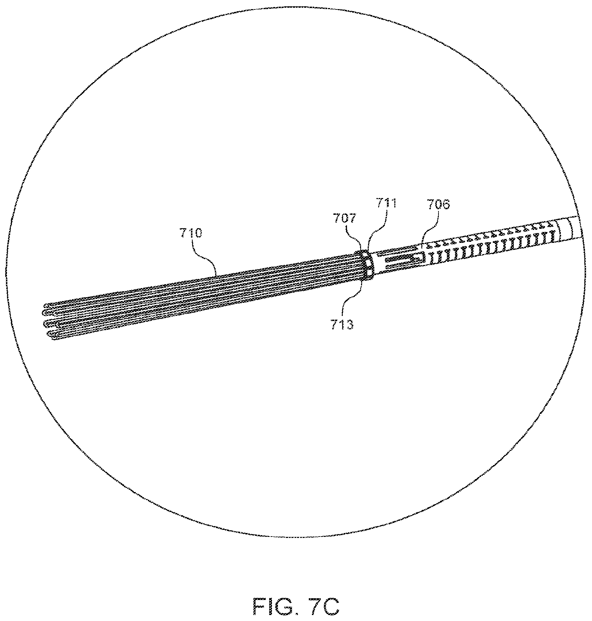

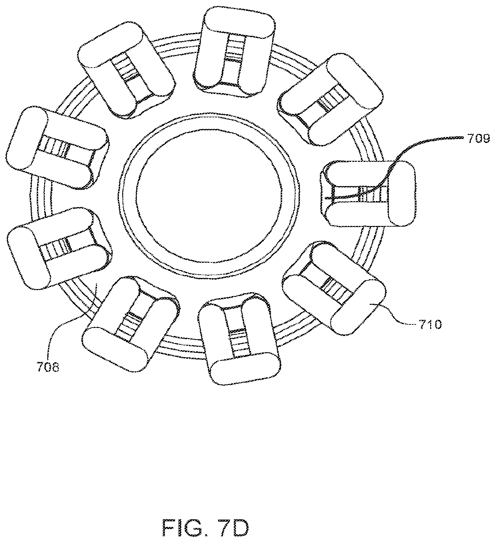

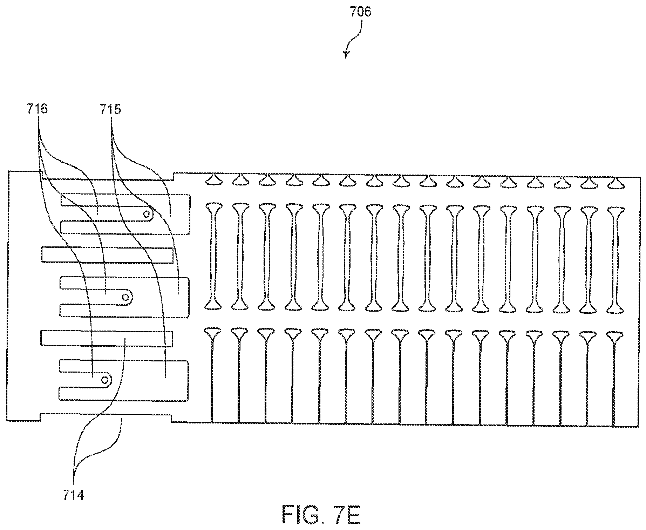

**Figure 7A** shows a perspective view of a manifold subassembly of the delivery device of the delivery system of **Figure 1****.** **Figure 7B** shows a side cross-section view of the manifold subassembly of **Figure 7A****.** **Figure 7C** shows a close-up view of a distal end portion of the manifold subassembly. **Figure 7D** shows a bottom view of the distal end portion of the manifold subassembly. **Figure 7E** shows a flat cut pattern of a distal end portion of the manifold subassembly.

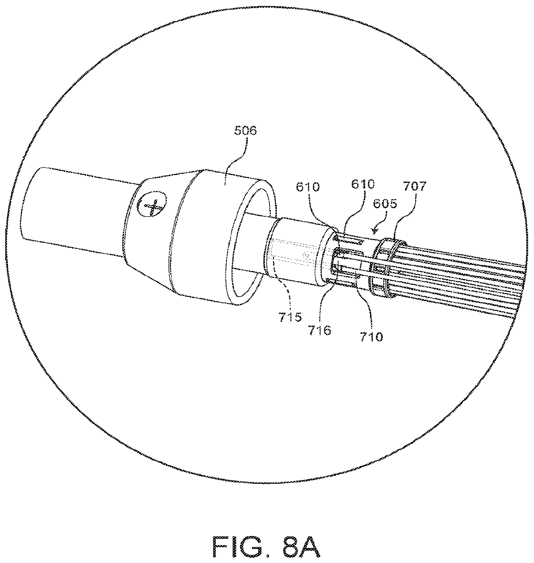

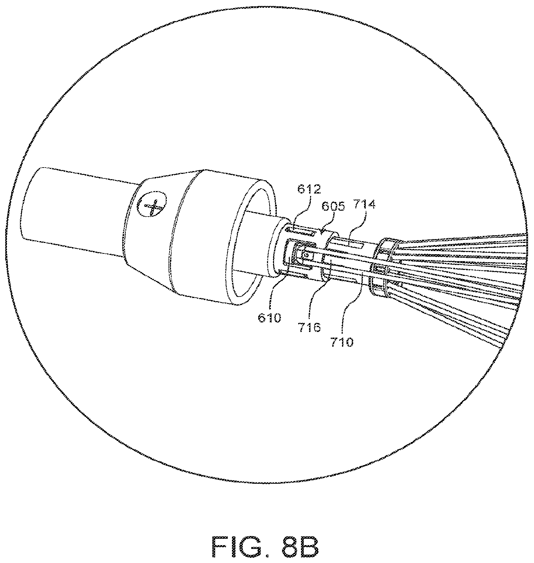

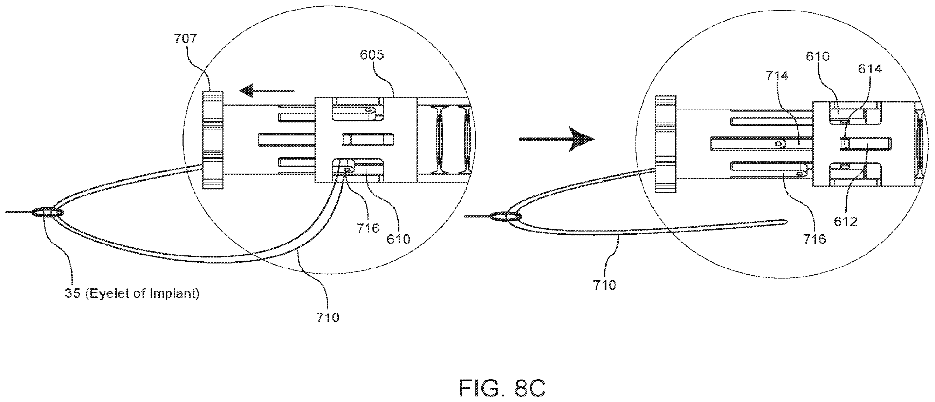

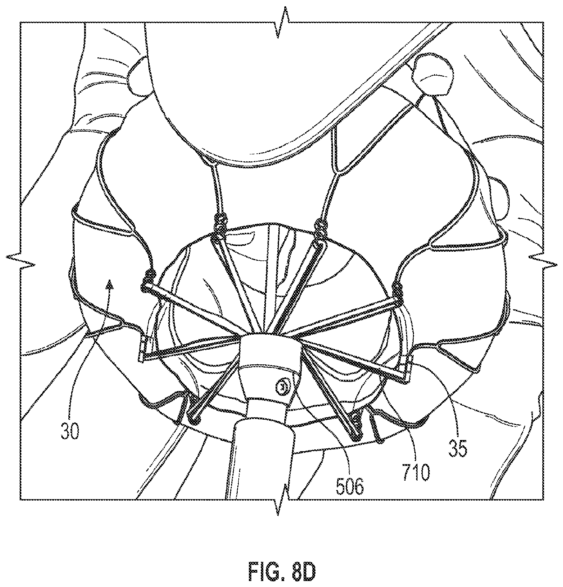

**Figures 8A** **and** **8B** show distal end portions of the release and manifold assemblies in a locked configuration and unlocked configuration, respectively. **Figure 8C** illustrates tethering and untethering of a suture using the release and manifold assemblies. **Figure 8D** shows suture loops tethered to the eyelets of the valve prosthesis while also tethered to the manifold subassembly of the delivery device.

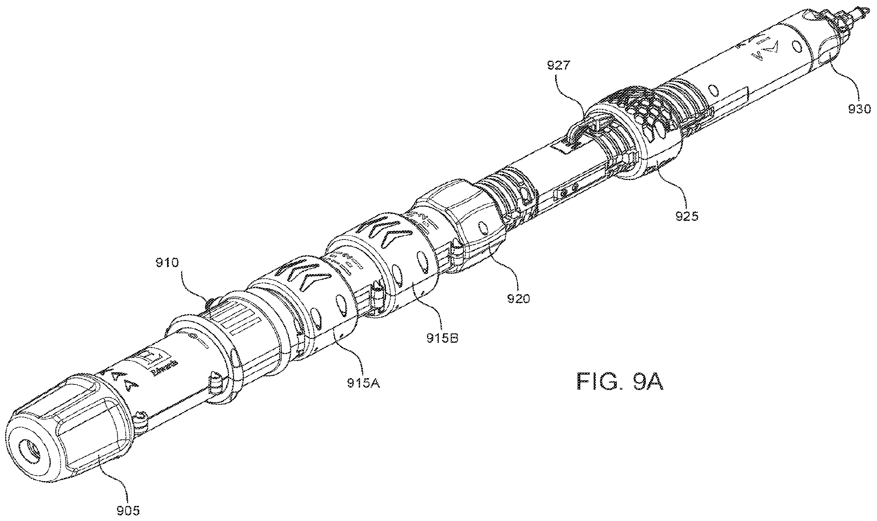

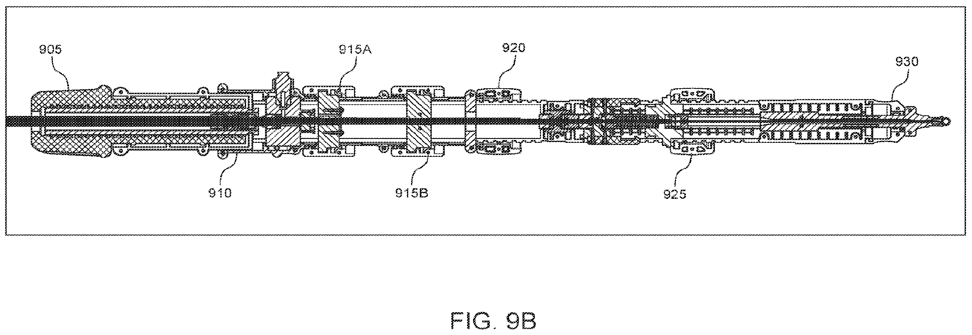

**Figure 9A** shows a perspective view of a handle of the delivery device of **Figure 1****.** **Figure 9B** shows a side cross-section view of the handle of the delivery device.

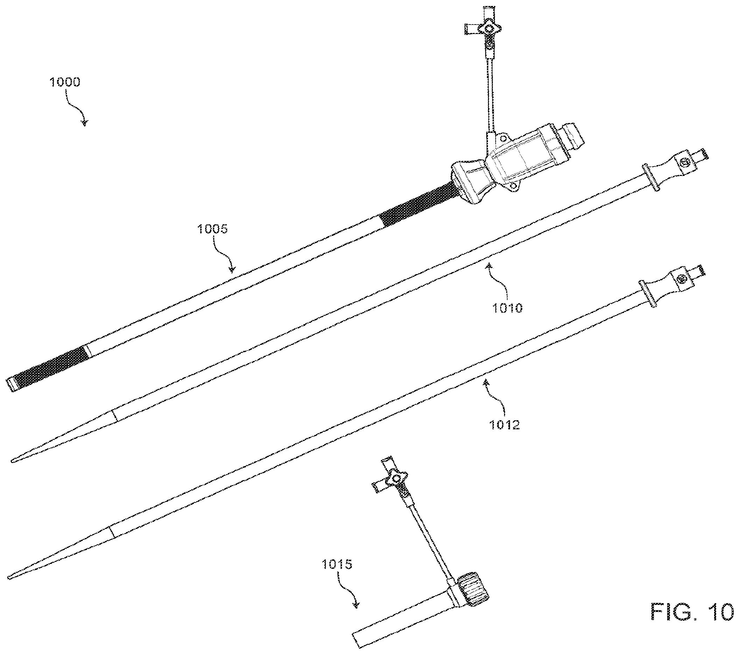

**Figure 10** shows components of an introducer assembly of the delivery system of **Figure 1****.**

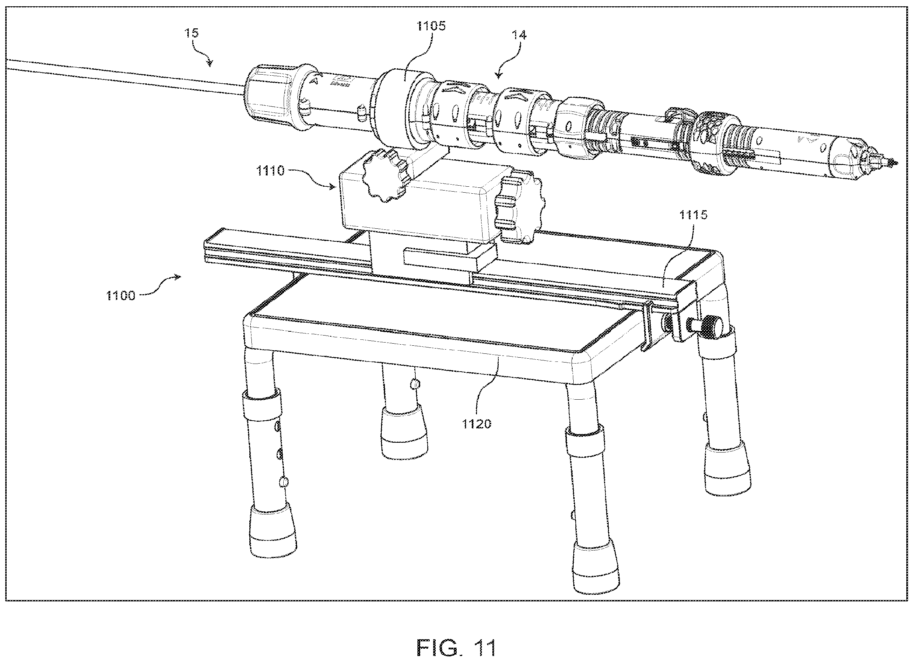

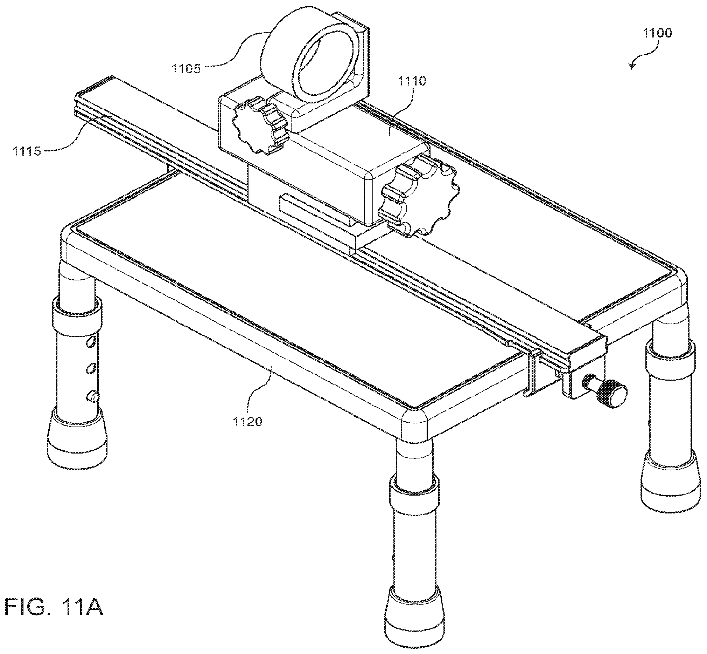

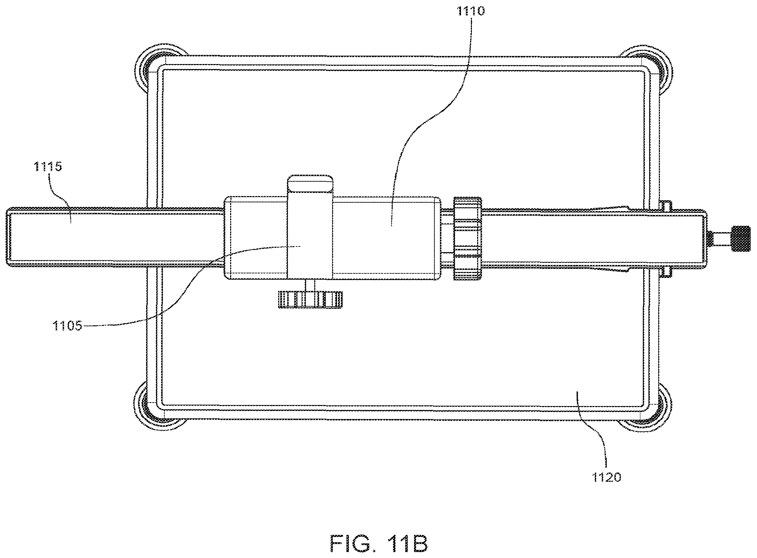

**Figure 11** illustrates how the handle of the delivery device interfaces with an embodiment of a stabilizer assembly of the delivery system of **Figure 1****.** **Figure 11A** shows a perspective view of the stabilizer assembly without the delivery device attached. **Figure 11B** shows a top view of the stabilizer assembly of **Figure 11A****.**

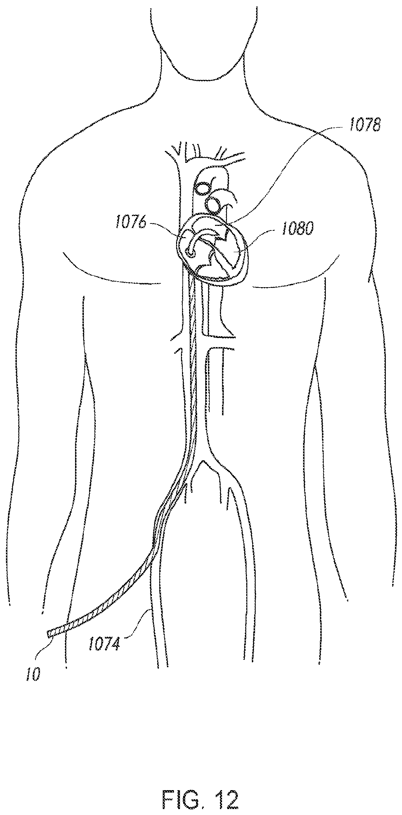

**Figure 12** illustrates a schematic representation of a transfemoral and transseptal delivery approach.

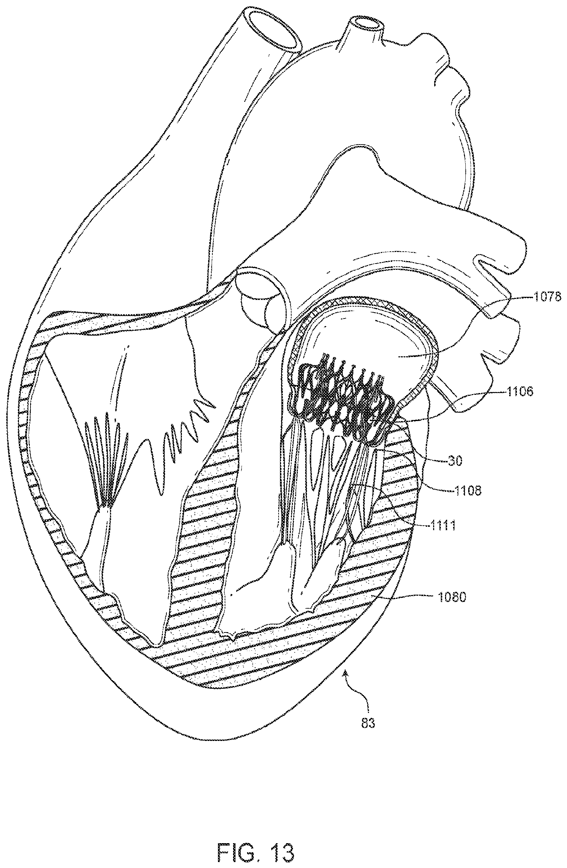

**Figure 13** illustrates a schematic representation of a valve prosthesis positioned within a native mitral valve (shown without a skirt assembly to facilitate visualization of interface with native heart valve structures).

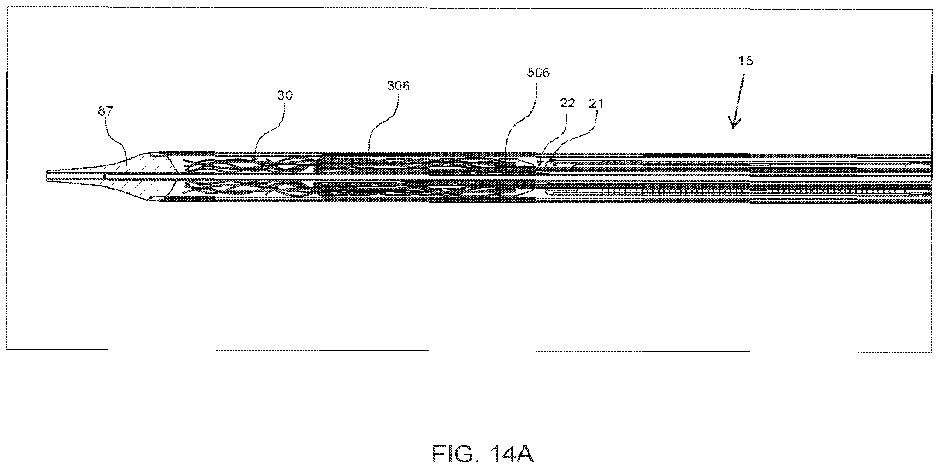

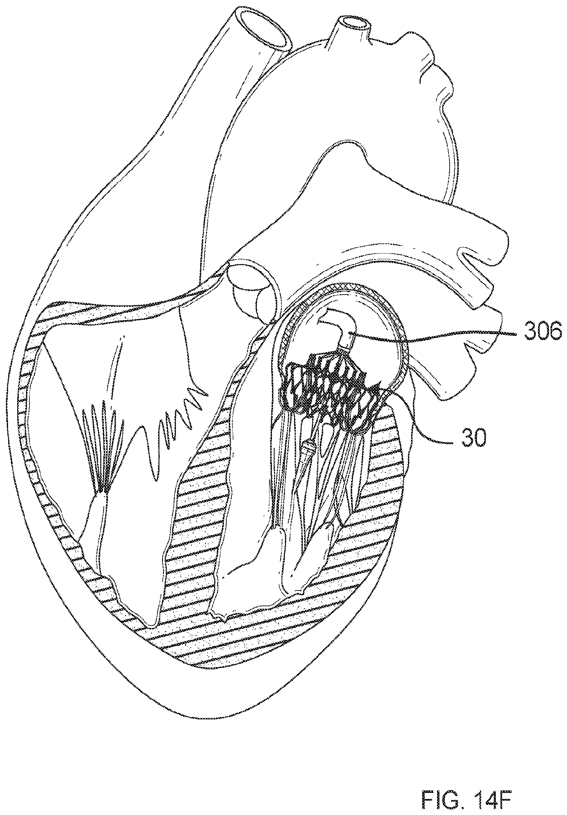

**Figures 14A****-14E** illustrate various steps of deployment of the valve prosthesis using the delivery device described herein, with a focus on the positioning of the various subassemblies of the delivery device with respect to each other and with respect to the valve prosthesis at the different steps. **Figures 14F to 14K** illustrate various steps of deployment and recapture of the valve prosthesis using the delivery device described herein shown with reference to an example implantation location within the heart.

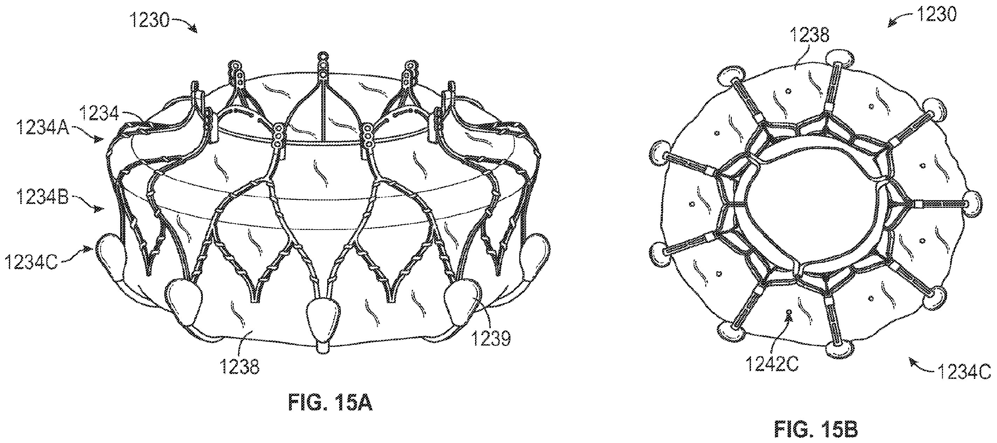

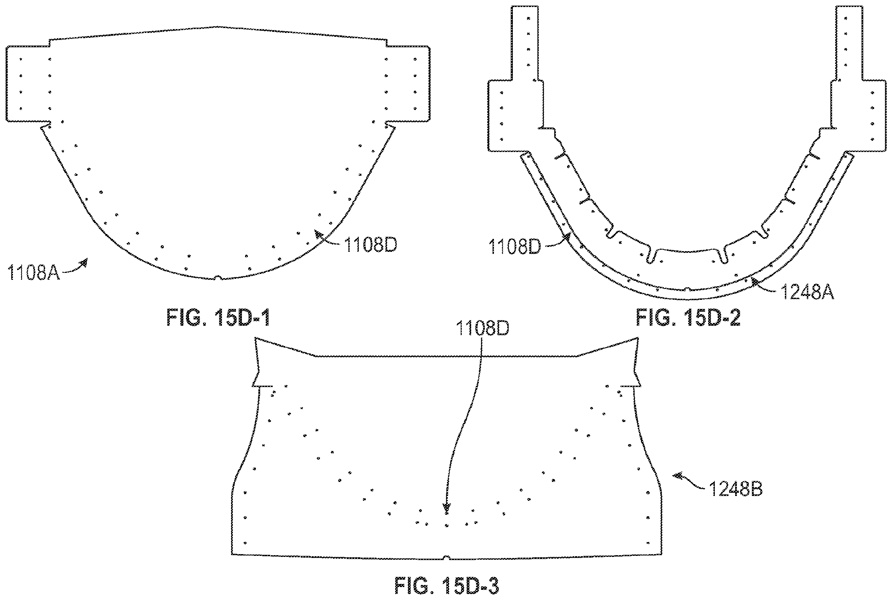

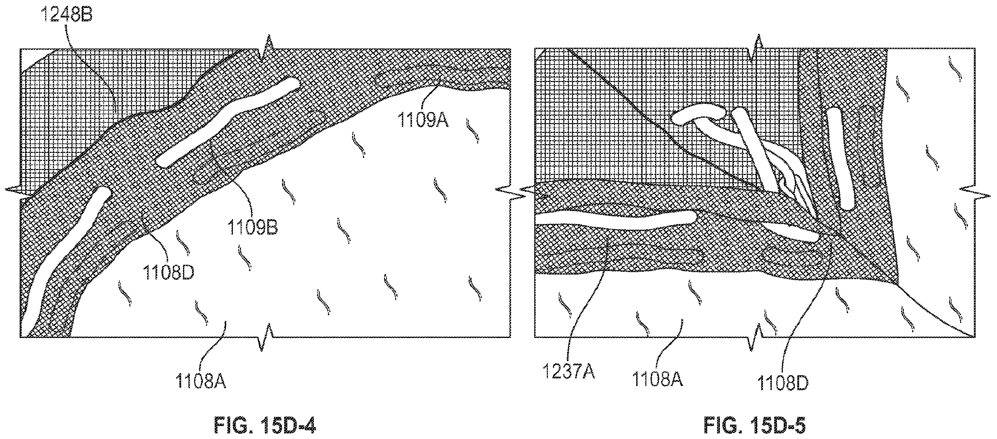

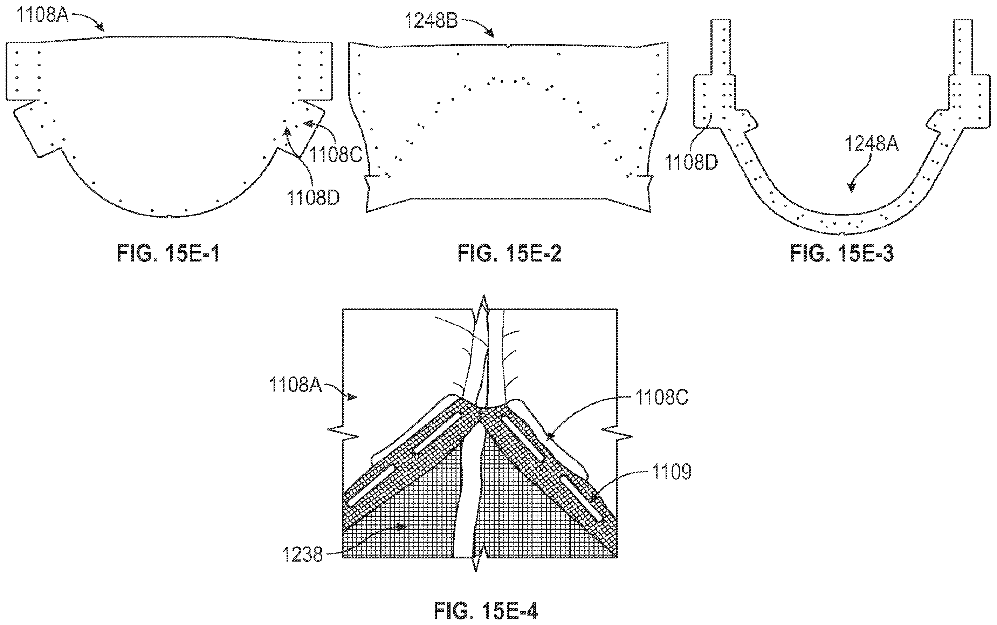

**Figure 15A** shows a side perspective view of a configuration of a fully-assembled dual-frame valve prosthesis including a skirt assembly and padding. **Figure 15B** shows a side view of the fully-assembled dual-frame valve prosthesis of **Figure 15A****.**

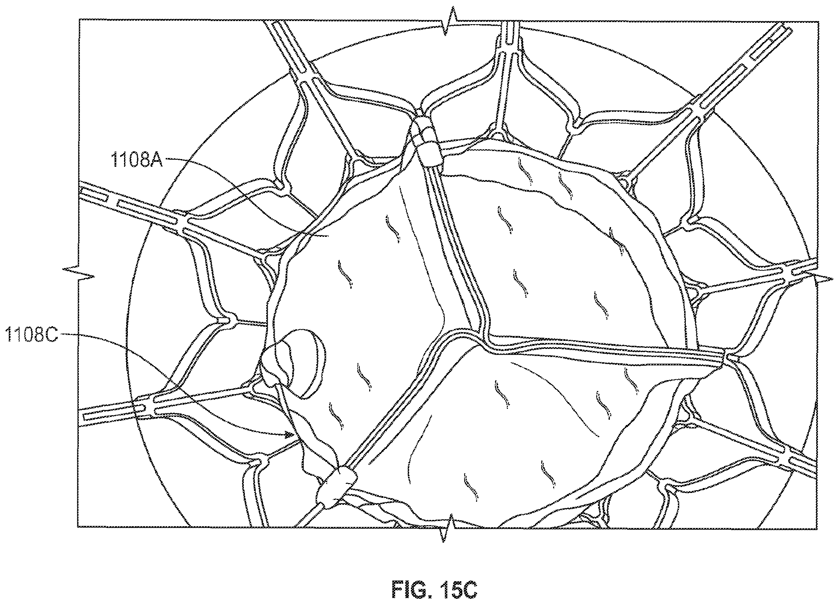

**Figure 15C** shows a prosthetic leaflet stitched to an inner frame of the dual-frame valve prosthesis.
**Figure 15D-1 to 15D-5 and 15E-1 to 15E-4** show double stitching applied to a prosthetic leaflet to securely attach the prosthetic leaflet to an inner frame of the dual-frame valve prosthesis.

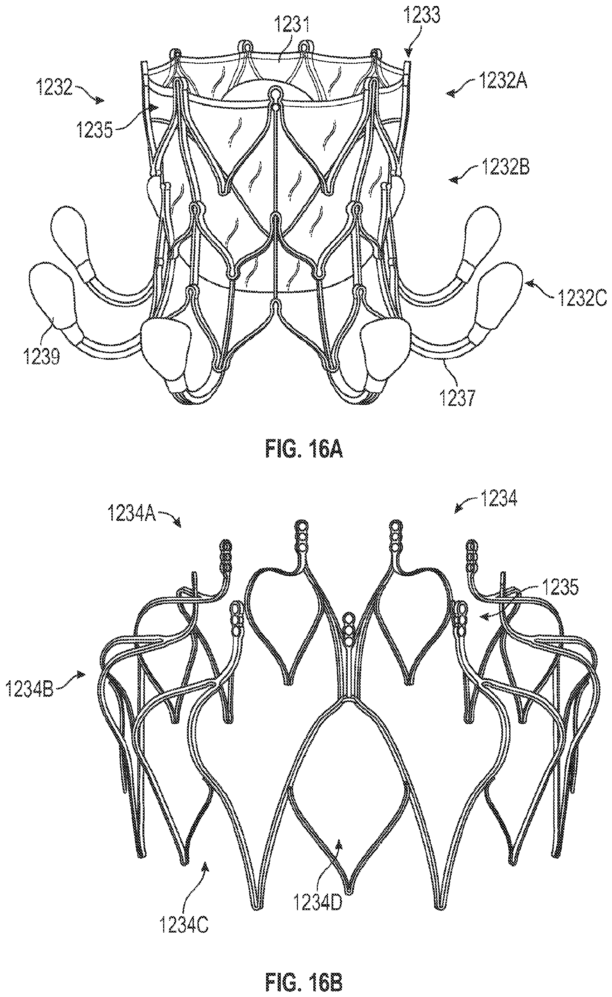

**Figure 16A** shows a side perspective view of an inner frame of the dual-frame valve prosthesis of **Figures 15A and 15B****.** **Figure 16B** shows a side perspective view of an outer frame of the dual-frame valve prosthesis of Figures **15A and 15B****.**

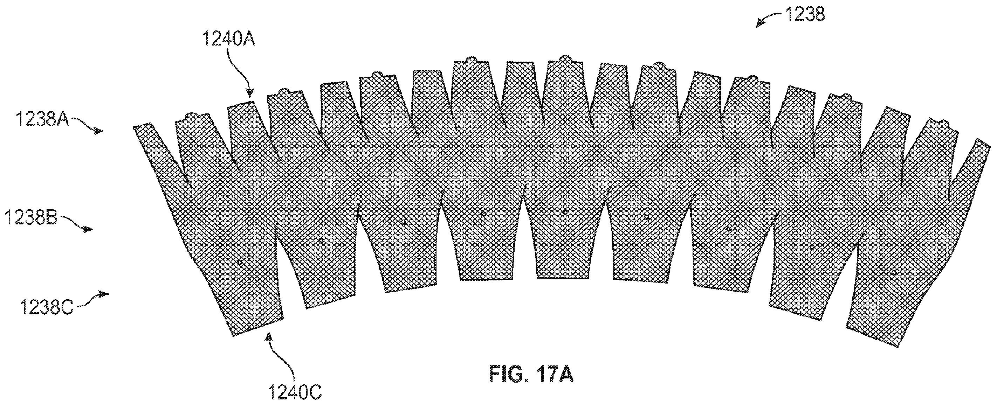

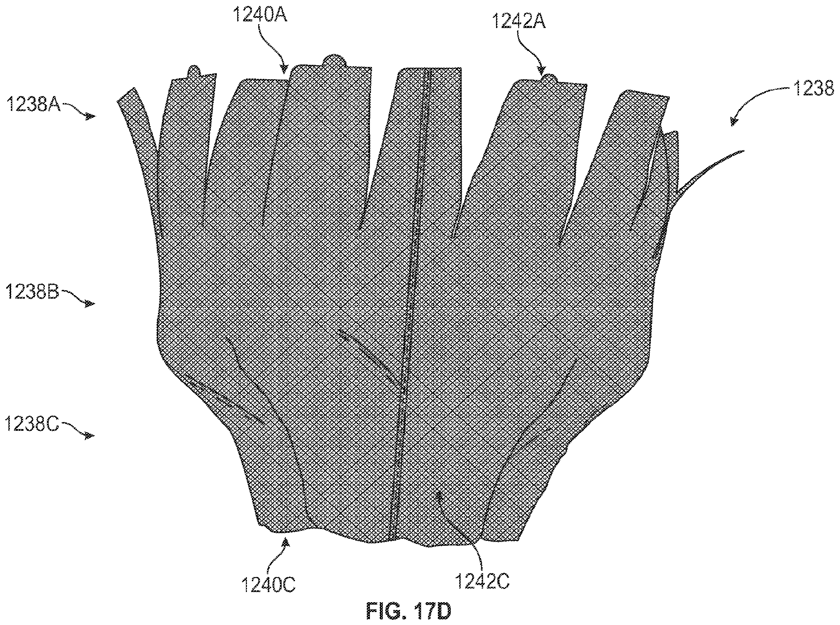

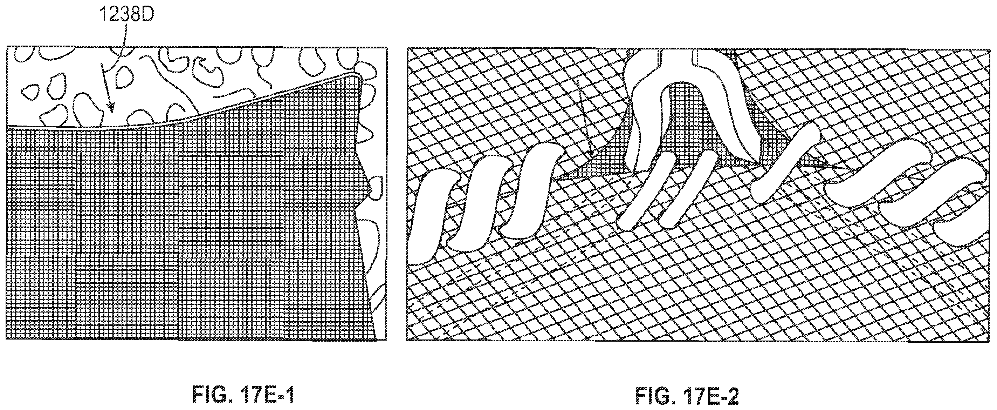

**Figures 17A to 17C** show the skirt assembly of the dual-frame valve prosthesis of **Figures 15A and 15B** in a flat configuration. **Figure 17D** shows a side view of the skirt assembly of the dual-frame valve prosthesis of **Figures 15A and 15B** in a partially folded configuration.
**Figures 17E-1 and 17E-2** show softened edges of cloth material used for the skirt assembly of **Figures 17A to 17D****.**

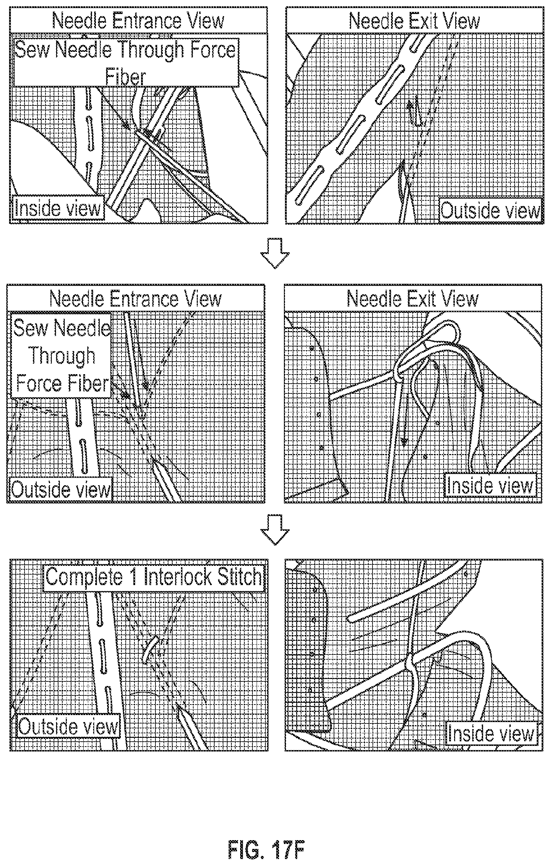

**Figure 17F** shows a process of applying an interlocking stitch of the cloth material used for the skirt assembly of **Figures 17A to 17D** to eliminate knots.

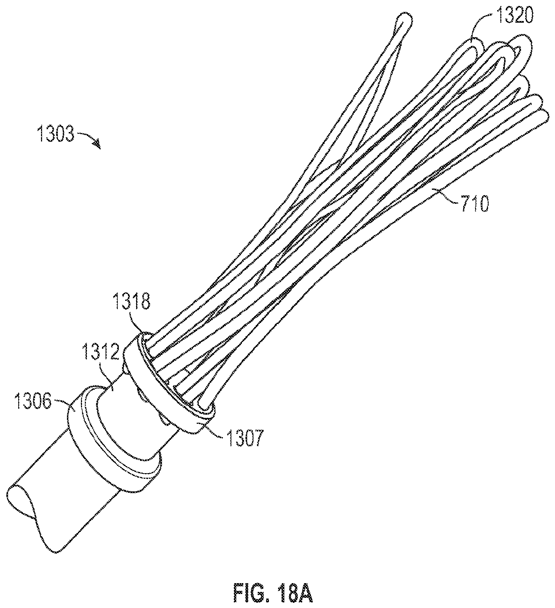

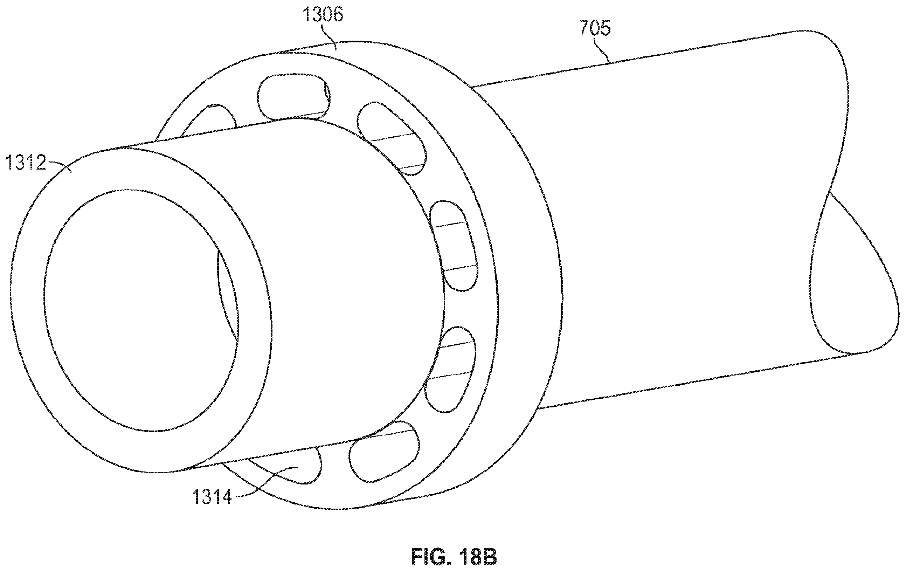

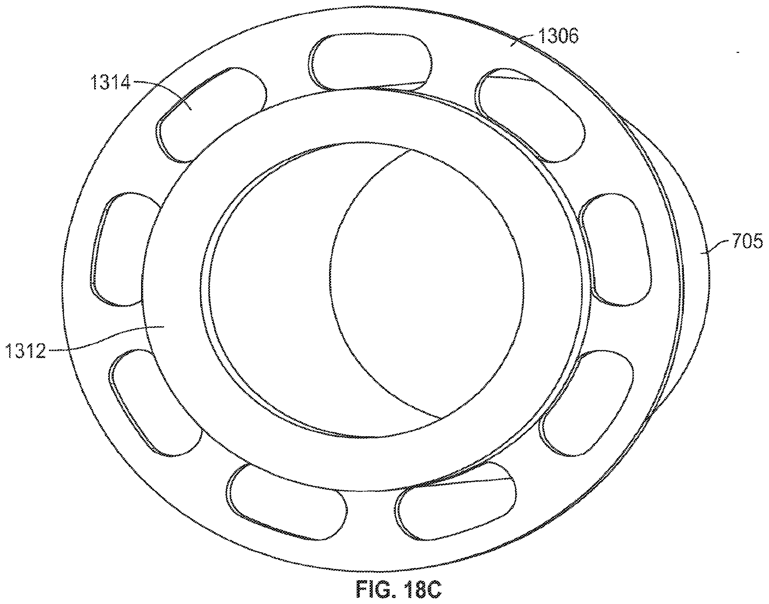

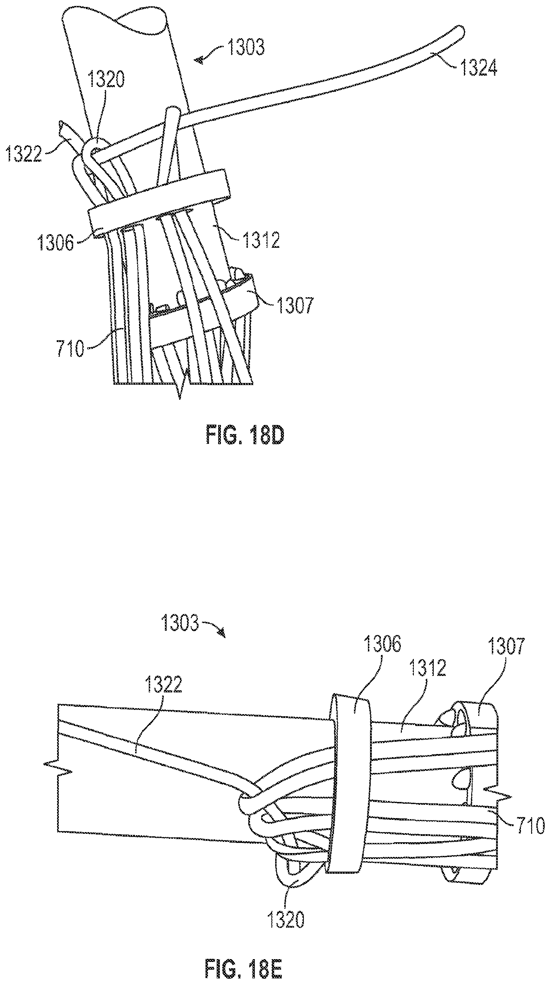

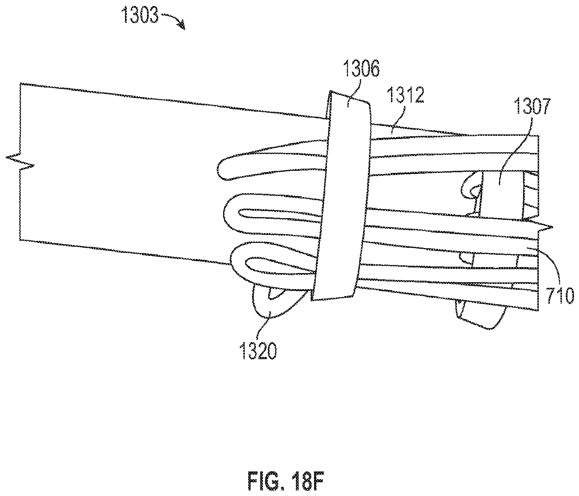

**Figure 18A** shows a close-up view of a distal end portion of a configuration of a manifold subassembly with suture or tether loops assembled thereto. **Figure 18B** shows a perspective side view of the distal end portion of the configuration of the manifold subassembly of **Figure 18A****.** **Figure 18C** shows a perspective bottom view of the distal end portion of the configuration of the manifold subassembly of **Figure 18A****.** **Figure 18D** shows a perspective view of a tether or a suture arrangement being secured to the distal end portion of the configuration of the manifold subassembly of **Figure 18A****.** **Figures 18E** **and** **18F** show a perspective view of the manifold subassembly illustrating how a retention portion of the tether or suture arrangement can be removed from the distal end portion of the configuration of the manifold subassembly of **Figure 18A****.**

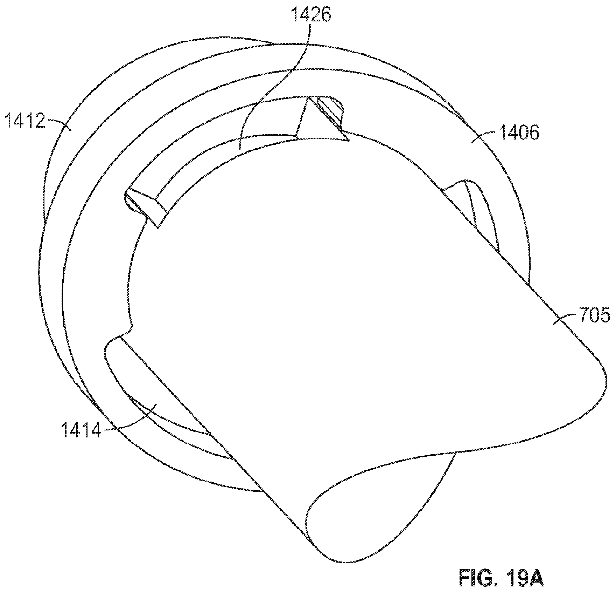

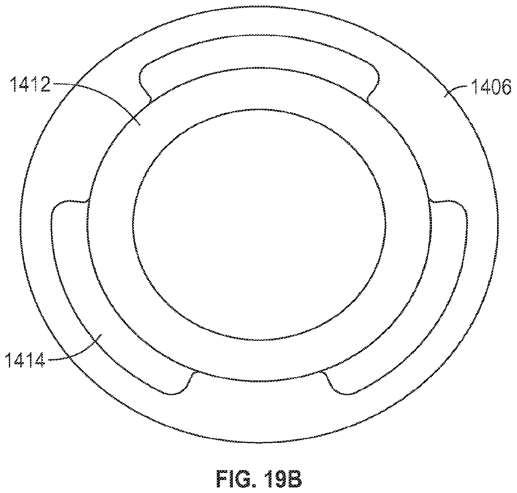

**Figure 19A** shows a perspective side view of a distal end portion of another configuration of a manifold subassembly. **Figure 19B** shows a plan view of the distal end portion of the configuration of the manifold subassembly of **Figure 19A****.**

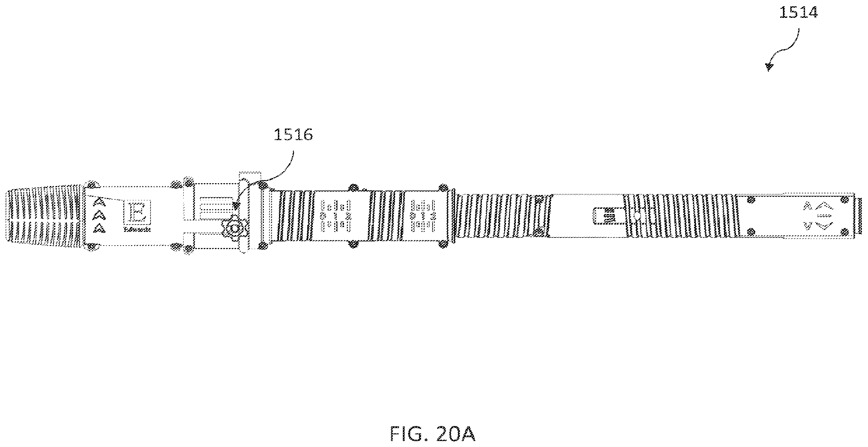

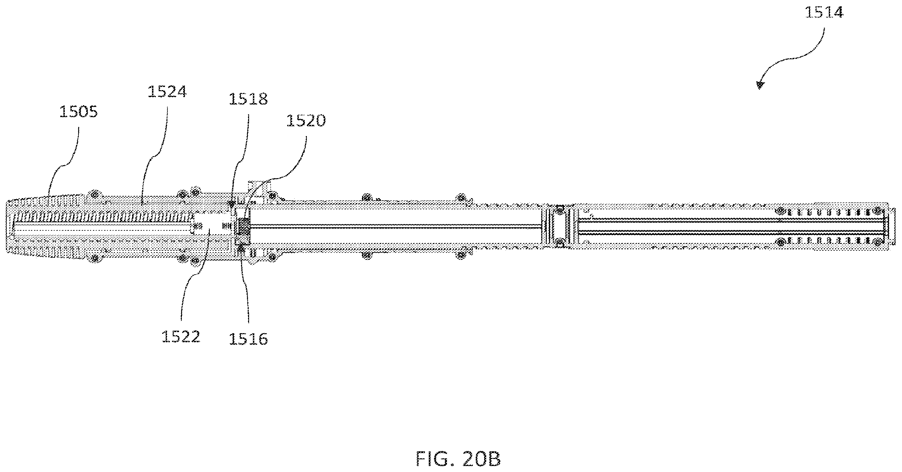

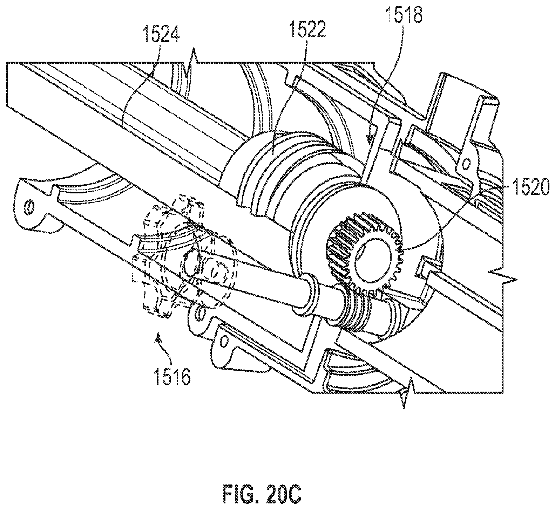

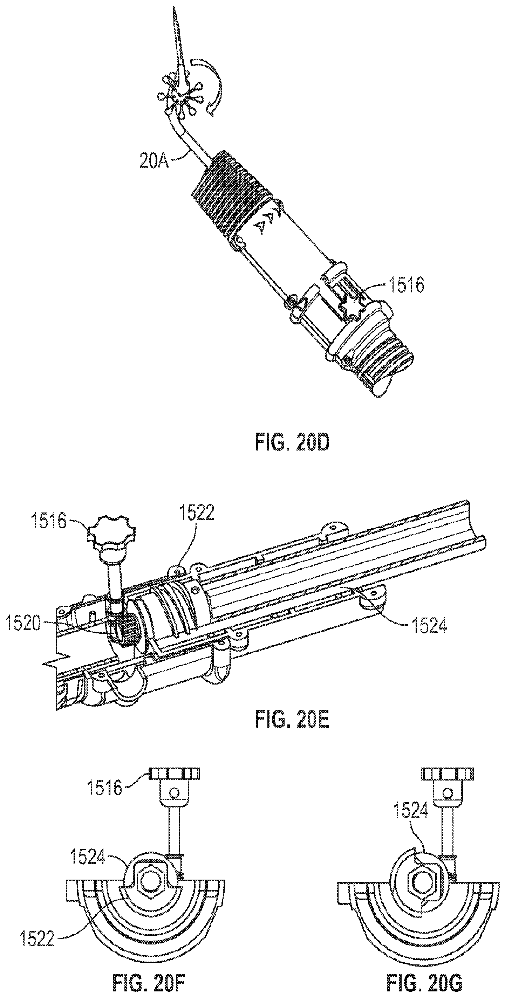

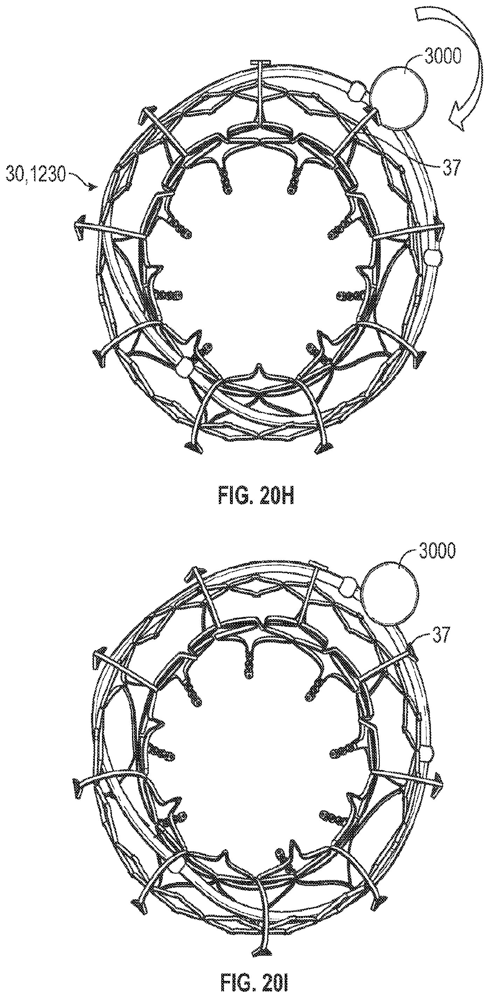

**Figure 20A** shows a side view of a configuration of a handle of a delivery device. **Figure 20B** shows a side cross-section view of the handle of **Figure 20A****.** **Figure 20C** shows a close-up cross-section view of the handle of **Figure 20A****.** **Figures 20D, 20E, 20F and 20G** illustrate an orientation mechanism of **Figure 20C** connected to an outer lumen within which a dual-frame valve prosthesis rotates to facilitate clocking of the prosthesis at a desired implantation location. **Figures 20H and 20I** schematically illustrate a clocking mechanism utilizing direct fluoroscopic visualization.
**Figure 21** shows a perspective view of a configuration of a handle of a delivery device.

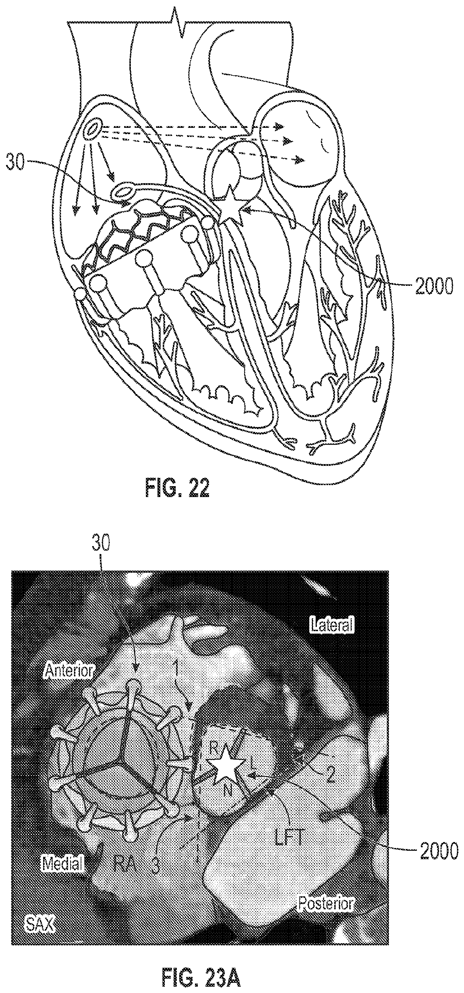

**Figure 22** shows a configuration of an implant within a heart of a patient.

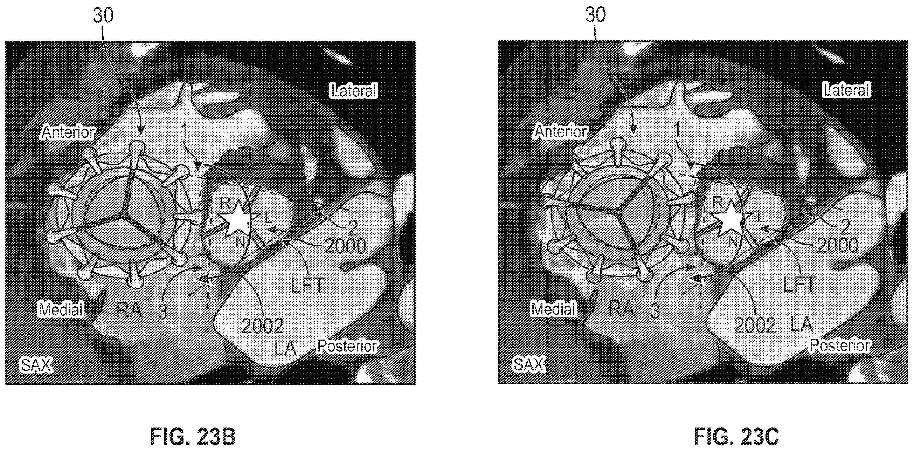

**Figures 23A to 23C** show the implant shown in **Figure 22** being rotated within the heart of the patient.

### DETAILED DESCRIPTION

The present specification and drawings provide aspects and features of the disclosure in the context of several embodiments of replacement heart valves, delivery systems and methods that are configured for use in the vasculature of a patient, such as for replacement of natural heart valves in a patient. These embodiments may be discussed in connection with replacing specific valves such as the patient's aortic, tricuspid, or mitral valve. However, it is to be understood that the features and concepts discussed herein can be applied to products other than heart valve implants. For example, the controlled positioning, deployment, and securing features described herein can be applied to medical implants, for example other types of expandable prostheses, for use elsewhere in the body, such as within an artery, a vein, or other body cavities or locations. In addition, particular features of a valve, delivery system, etc. should not be taken as limiting, and features of any one embodiment discussed herein can be combined with features of other embodiments as desired and when appropriate. While certain of the embodiments described herein are described in connection with a transfemoral delivery approach, it should be understood that these embodiments can be used for other delivery approaches such as, for example, transapical or transjugular approaches. Moreover, it should be understood that certain of the features described in connection with some embodiments can be incorporated with other embodiments, including those which are described in connection with different delivery approaches.

### Delivery System

**Figure 1** illustrates an embodiment of a delivery system **10.** The delivery system **10** can be used to deploy a prosthesis, such as a replacement heart valve, to a location within a body of a subject (e.g., human or veterinary subject). Replacement heart valves can be delivered to a subject's heart mitral or tricuspid valve annulus or other heart valve location in various manners, such as by open surgery, minimally-invasive surgery, and percutaneous or transcatheter delivery through the subject's vasculature. Example transfemoral approaches are described further in U.S. Pat. Publ. No. 2015/0238315, published August 27, 2015, the entirety of which is hereby incorporated by reference in its entirety. While the delivery system **10** is described in connection with a percutaneous delivery approach, and more specifically a transfemoral delivery approach, it should be understood that features of delivery system **10** can be applied to other delivery approaches, including delivery systems for a transapical delivery approach.

The delivery system **10** can be used to deploy a prosthesis, such as a replacement heart valve as described elsewhere in this specification, to a location within the body of a subject. The delivery system **10** can include multiple components, devices, or subassemblies. As shown in **Figure 1****,** the delivery system **10** can include a delivery device **15,** a stabilizer assembly **1100,** and an introducer assembly **1000** (not shown in Figure 1 but shown in Figure 10). The delivery device 15 includes a shaft assembly 12 and a handle 14. An implant (e.g., valve prosthesis or replacement heart valve) **30** can advantageously be pre-attached to the delivery device **15** during manufacture or assembly such that the clinician does not have to attach the implant **30** prior to use. The delivery device **15** may be configured to facilitate delivery and implantation of the implant (e.g., valve prosthesis) **30** to and at a desired target location (e.g., a mitral or tricuspid heart valve annulus). The implant (e.g., replacement heart valve) **30** may be pre-attached to or within a distal end portion of the shaft assembly **12** and removably tethered to one or more retention components of the shaft assembly **12** during manufacturing or assembly. The delivery device **15** with the pre-attached implant **30** may then be packaged, sterilized, and shipped for use by one or more clinicians. In accordance with several embodiments, the implant **30** is not supplied pre-crimped in the shaft assembly **12** delivery device **15.** In other embodiments, the implant **30** is pre-loaded or supplied pre-crimped in the shaft assembly **12.**

### Implants for Use with Delivery System

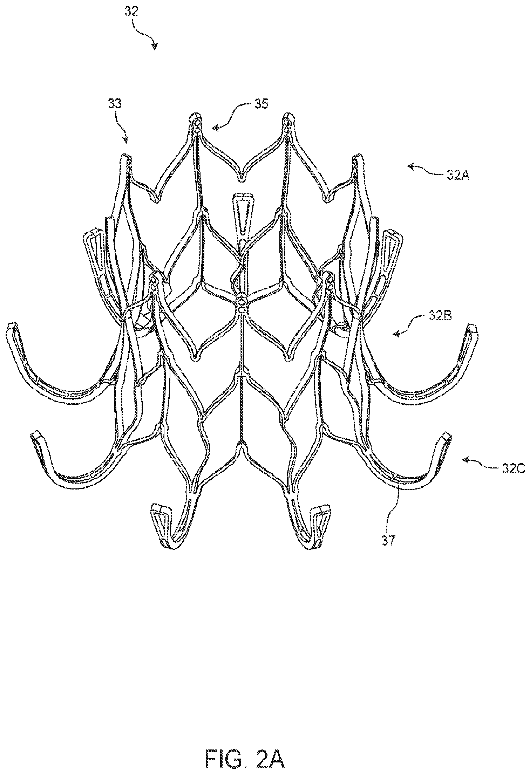

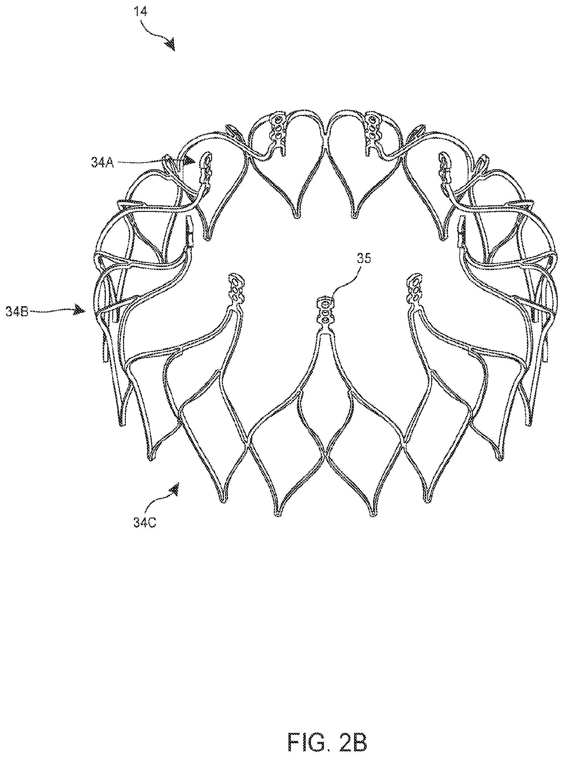

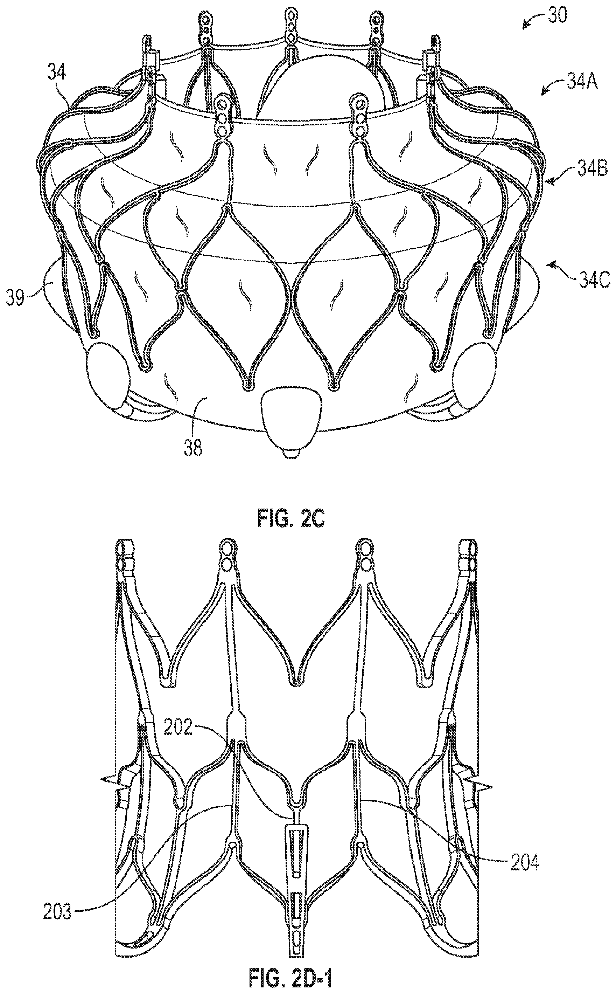

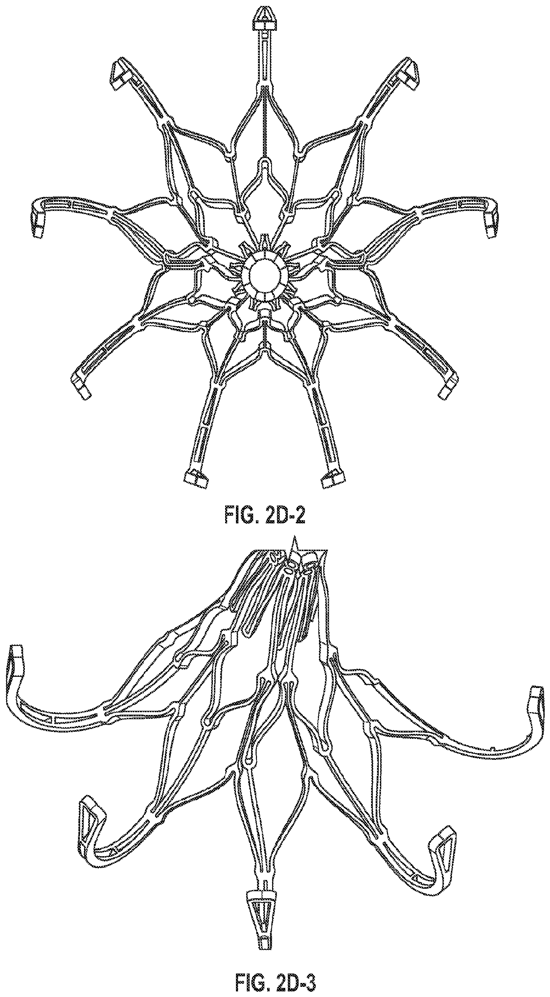

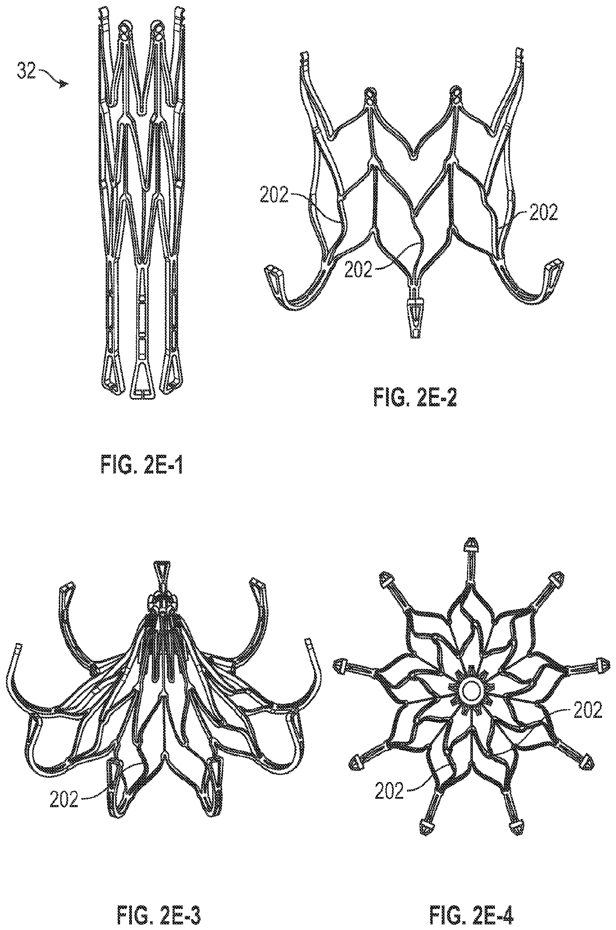

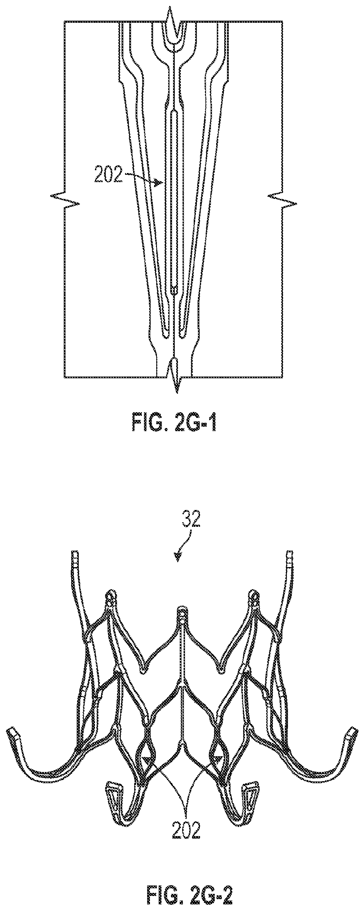

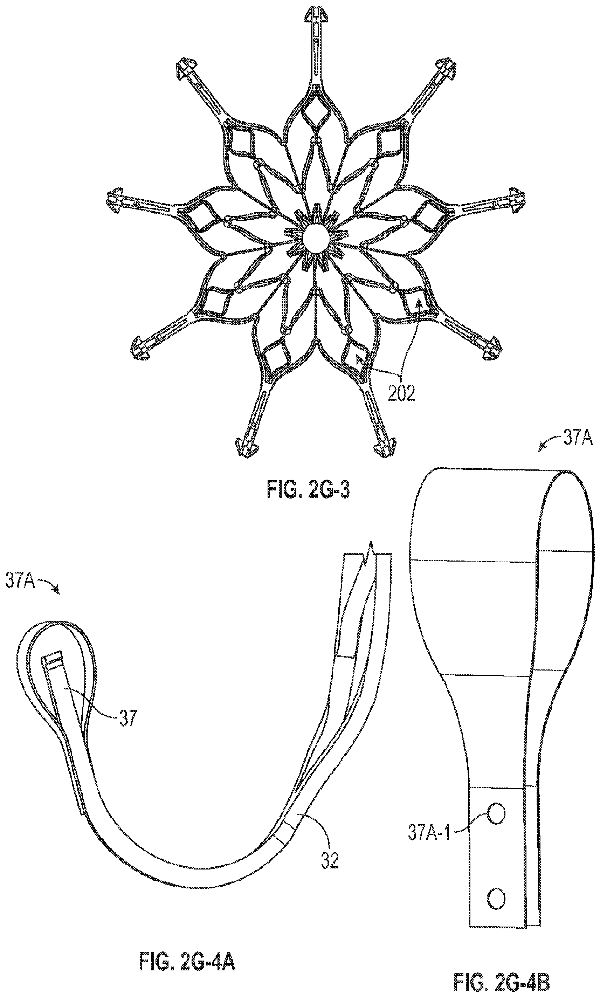

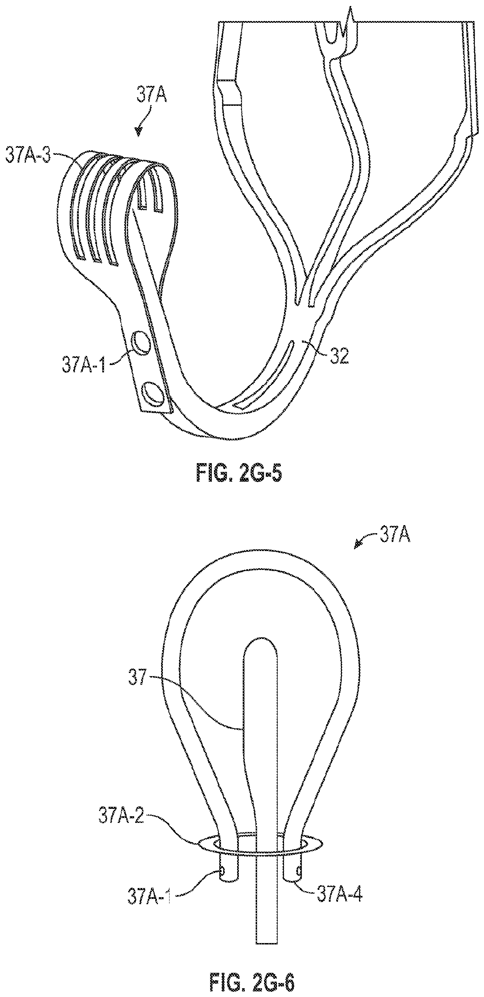

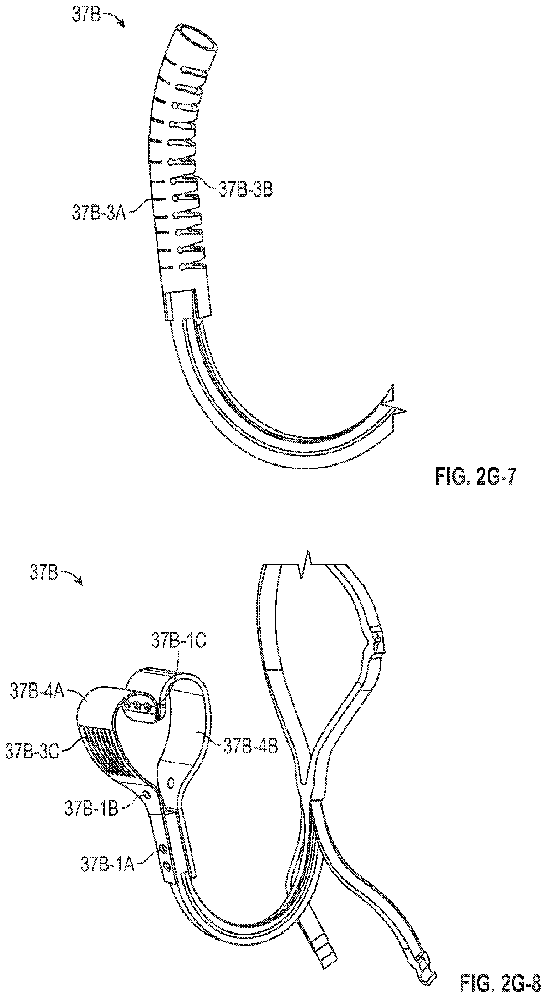

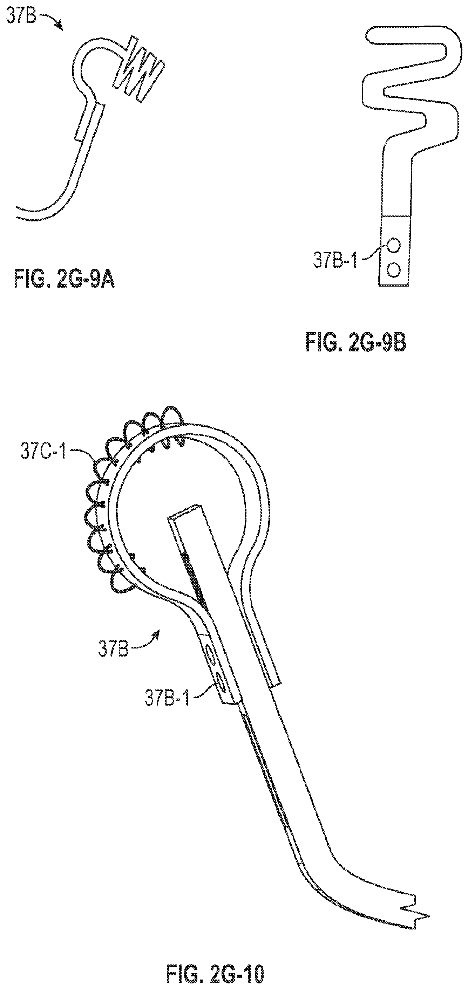

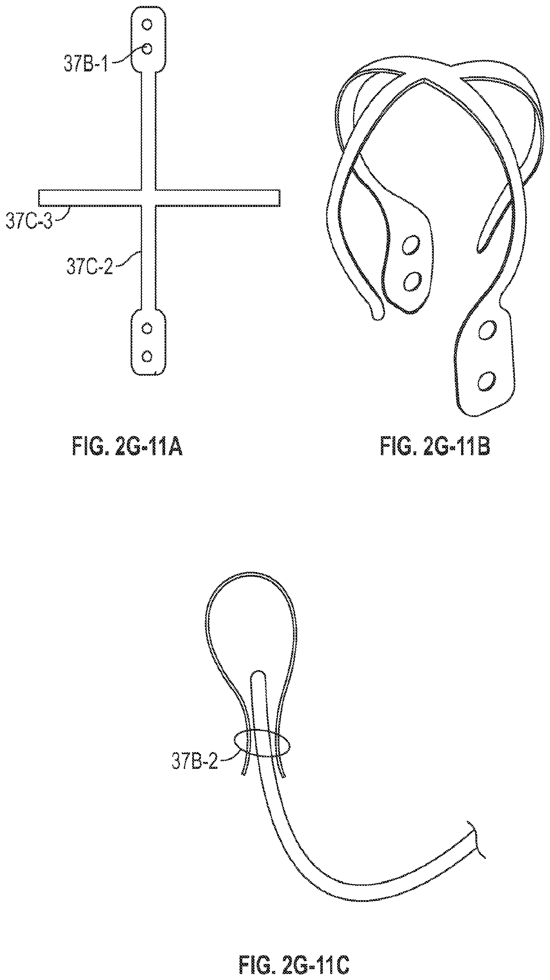

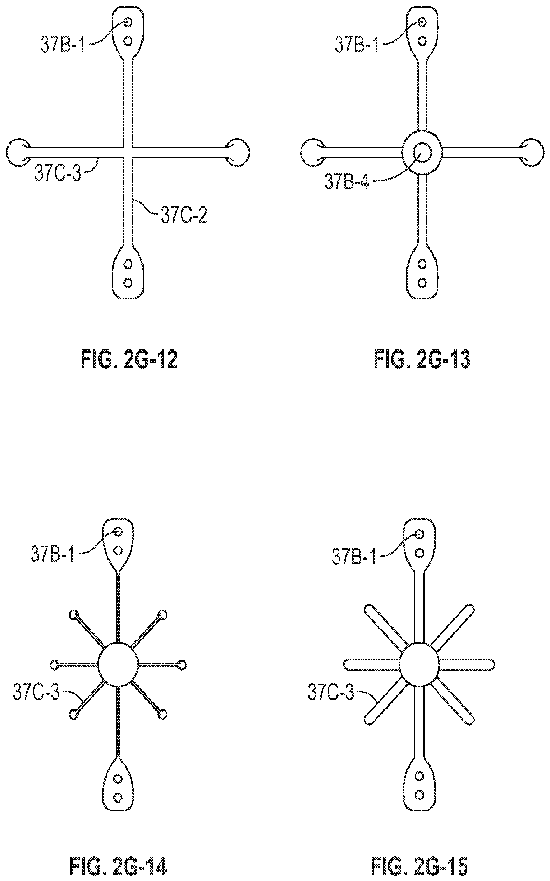

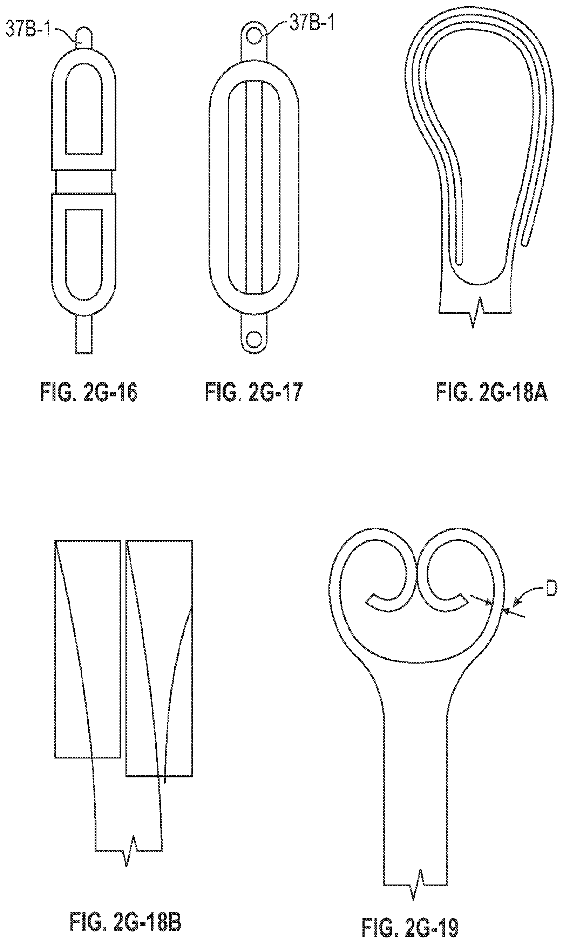

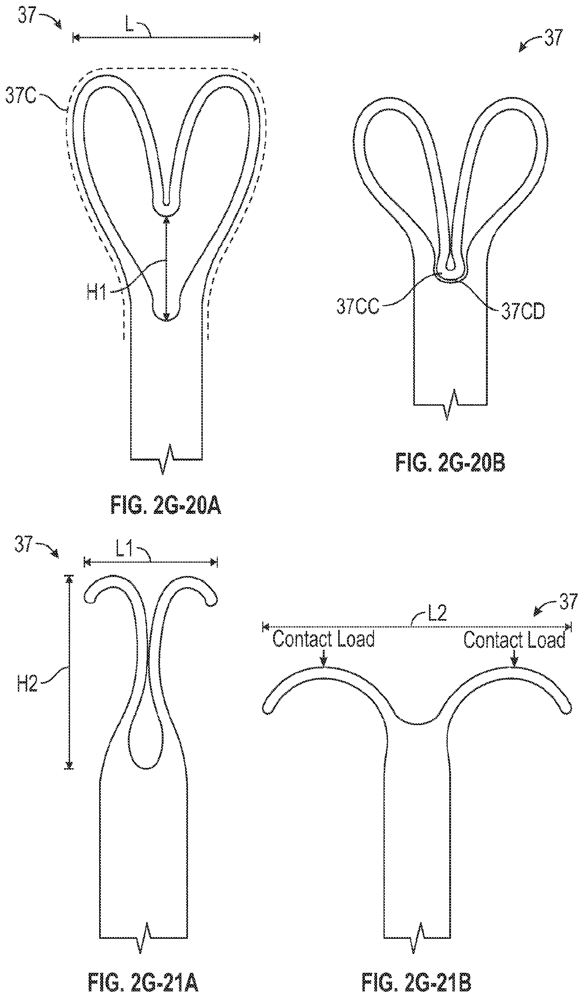

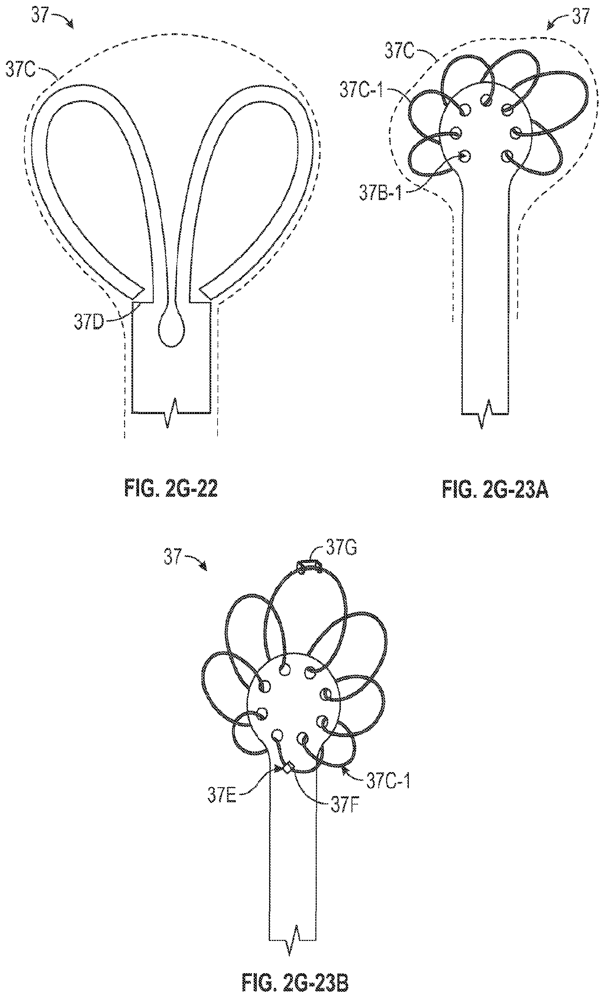

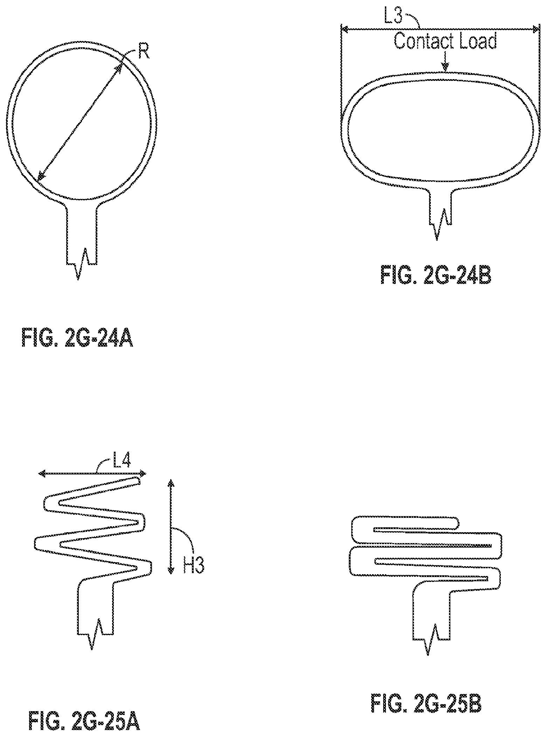

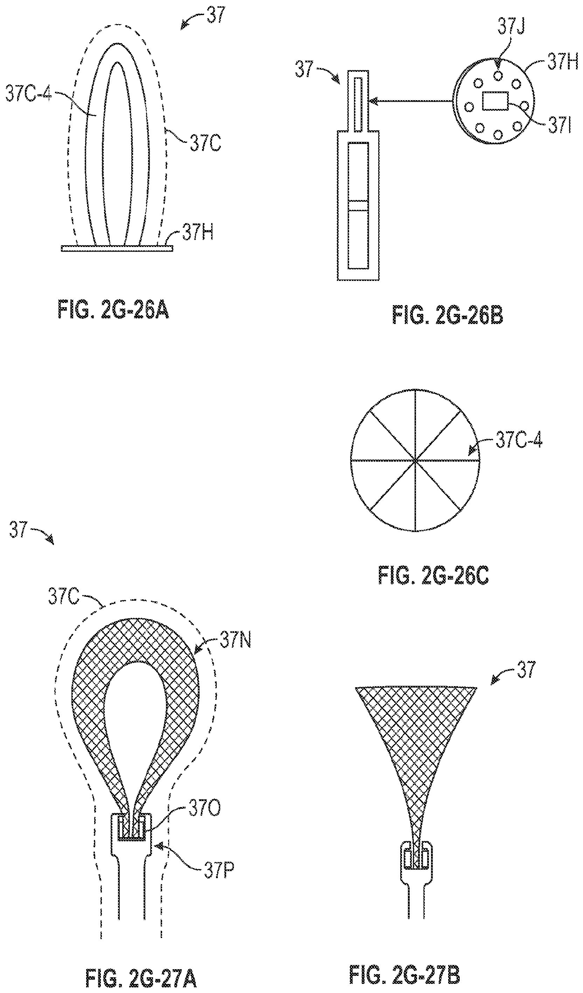

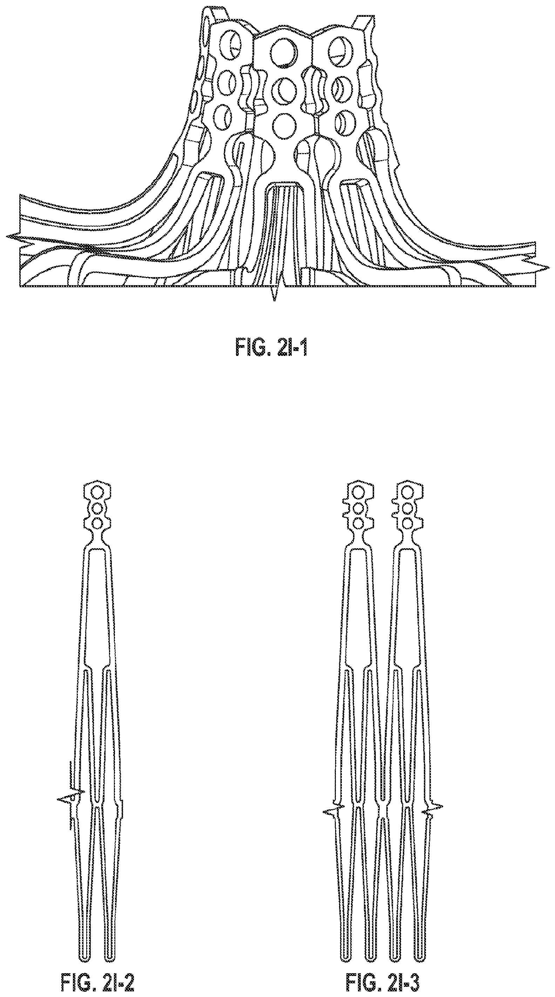

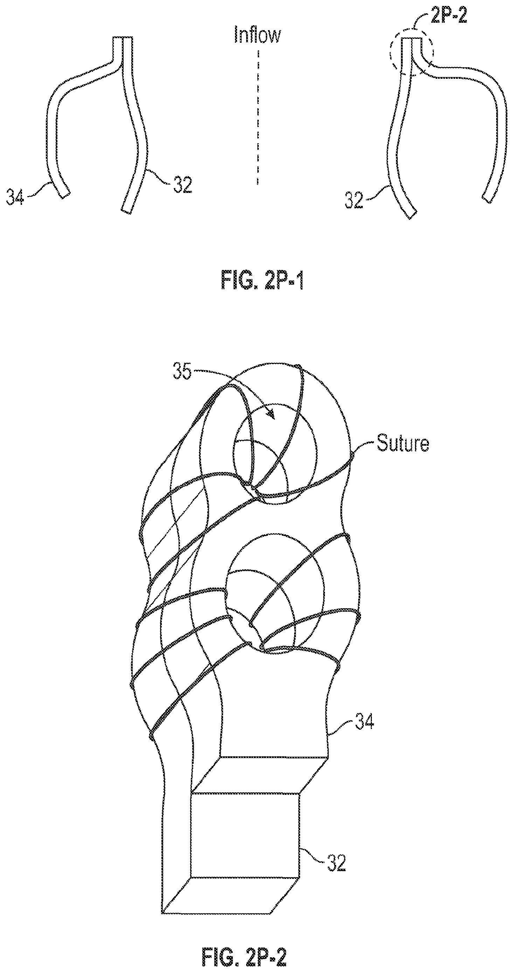

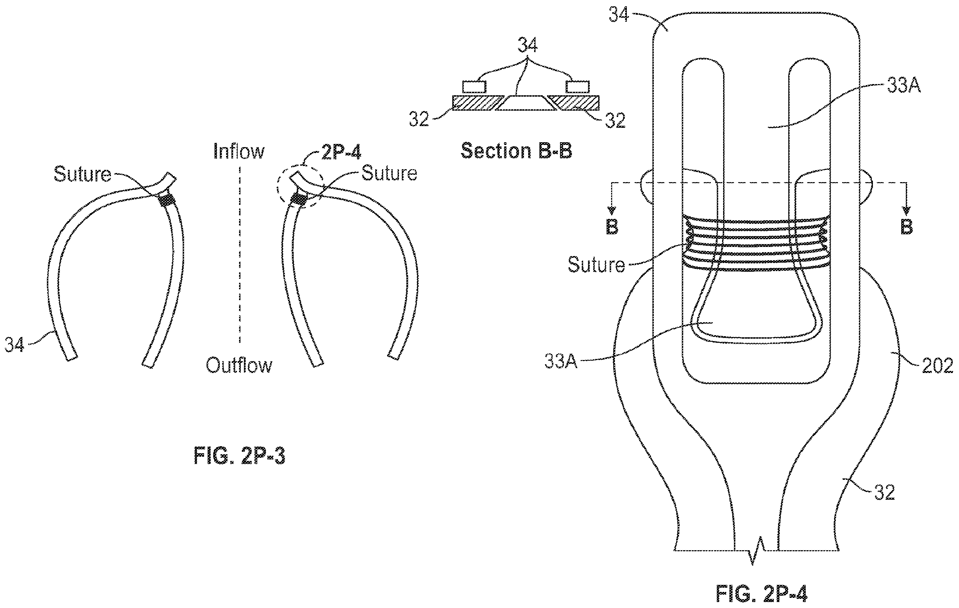

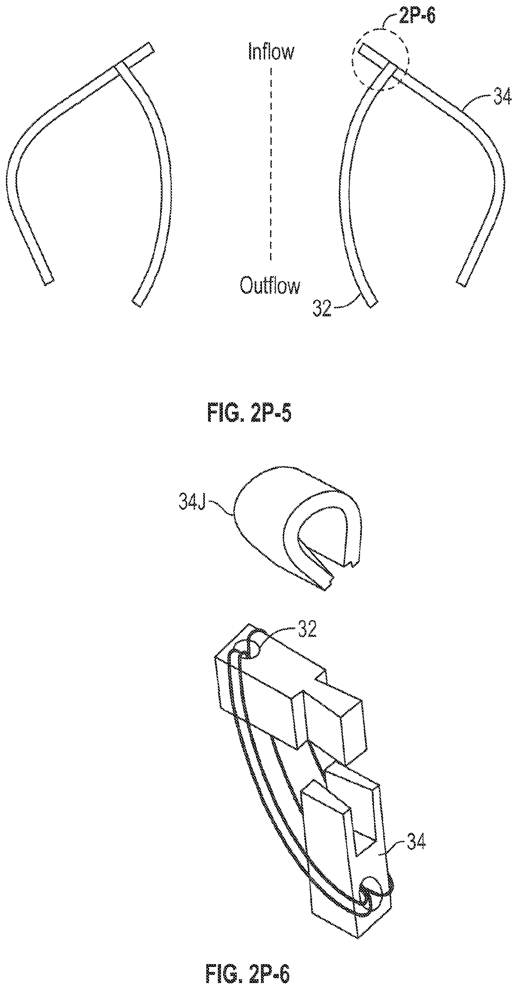

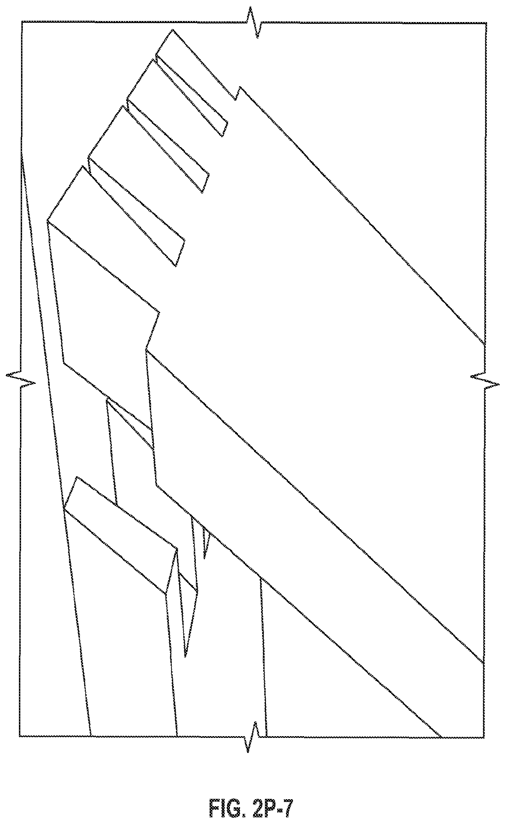

**Figure 2** shows an example frame structure for an implant (e.g., valve prosthesis) **30** that can be pre-loaded into and delivered by the delivery device **15.** The implant **30** includes a dual frame assembly including an inner frame **32** and an outer frame **34** that are aligned and coupled together during manufacture. **Figure 2A** illustrates an embodiment of the inner frame **32.** The inner frame **32** can include a proximal, or inflow, portion **32A,** a middle, or intermediate, portion **32B,** and a distal, or outflow, portion **32C.** The inner frame **32** can be shaped to exhibit a generally hourglass shape in an expanded configuration, in which the middle portion **32B** has a smaller cross-sectional width than the cross-sectional width of the proximal portion **32A** and the distal portion **32C.** The proximal portion **32A** may include tabs **33** and/or eyelets **35** to facilitate engagement with other structures or materials (e.g., the outer frame **34,** a skirt or fabric assembly, a prosthetic valve assembly, and/or tethers or retention sutures of the delivery device **15**). The distal portion **32C** may include outwardly and upwardly-extending anchors **37** to facilitate anchoring at a desired target location (e.g., a native heart annulus). The inner frame **32** may have a chevron cell structure as shown in Figure 2A. However, other cell structures may be used. The inner frame **32** may include a prosthetic valve assembly coupled thereto comprising a plurality of prosthetic valve leaflets (not shown). **Figure 2B** illustrates an embodiment of the outer frame **34.** The outer frame **34** may also include a proximal, or inflow, portion **34A,** a middle, or intermediate, portion **34B,** and a distal, or outlet, portion **34C.** Similar to the proximal portion **32A** of the inner frame **32,** the proximal portion **34A** of the outer frame **34** may also include one or more eyelets **35** to facilitate coupling to one or more structures or materials (e.g., the inner frame **32,** a skirt or fabric assembly, and/or to tethers or retention sutures of the delivery device **15**). For ease of understanding, in **Figures 2****,** **2A****,** **2B****,** the prosthesis **30** is shown with only the bare metal frame structures illustrated. **Figure 2C** illustrates an embodiment of a fully-assembled implant (e.g., valve prosthesis) **30** including a skirt assembly **38** positioned between the frames **32,34** and padding **39** surrounding the anchors **37.** The implant (e.g., prosthesis) **30** can take any number of different forms or designs.

Additional details and example designs for an implant (e.g., prosthesis or replacement heart valve) are described in U.S. Patent Nos. 8,403,983, 8,414,644, 8,652,203 and U.S. Patent Publication Nos. 2011/0313515, 2012/0215303, 2014/0277390, 2014/0277422, 2014/0277427, 2018/0021129, 2018/0055629 and 2019/0262129 (e.g., hourglass shape of inner frame). The entirety of these patents and publications are hereby incorporated by reference and made a part of this specification. Further details and embodiments of a replacement heart valve or prosthesis and its method of implantation are described in U.S. Publication Nos. 2015/0328000, 2016/0317301, 2019/0008640, and 2019/0262129, the entirety of each of which is hereby incorporated by reference and made a part of this specification.

### Frame Structural Features

Figures **2D-1 to 2D-3** illustrate how structural instability (e.g., strut buckling) can occur during compression (e.g., crimping, mid-loading) of a standard chevron-cell frame structure. When a chevron-cell frame is progressively reduced in diameter (e.g., funneled), such as when a frame is loaded into a shaft assembly of a delivery device having a smaller diameter than the frame in the expanded configuration, structural instability (e.g., ovality) of the cells and struts of the chevron-cell frame can occur. This structural instability can hamper an implantation procedure and, in extreme cases, can reduce structural integrity of the frame. The structural instability can produce unpredicted stress or strain on the frame, which could compromise durability, leading to device failure. With reference to Figure 2D-1, the chevron-cell structure, as it is crimped or funneled, drives internal forces through its constituent struts. When a chevron-cell frame is partially funneled or crimped, the internal forces are at a maximum, with some cells partially open and others partially closed. A conventional chevron-cell structure can become an inherently unstable system, wherein the portion or section of the frame that is undergoing reduction in diameter begins to forelengthen. Forelengthen may be the converse of foreshorten. In some implementations, forelengthen may mean the same as lengthen. The portion or section of the frame that is still fully expanded resists the forelengthening, and strut buckling can occur as a result. When partially funneled, axial beam or strut **202,** for example, of the fully expanded portion of the frame can buckle in an unpredictable direction, which can lead to ovality cascade, as shown in Figure 2D-2 (bottom view of a partially-funneled, or partially-crimped, inner frame with a conventional chevron-cell structure) and Figure 2D-3 (side perspective view of a partially-funneled, or partially-crimped, inner frame with a conventional chevron-cell structure). When partially funneled, axial beam or strut **202** may be under compression and axial beams or struts **203, 204** may be under tension.

**Figures 2E-1 to 2E-4, 2F-1 and 2F-2, and 2G-1 to 2G-3** illustrate various views of embodiments of inner frames having a chevron-cell structure that include structural mechanisms or features configured to dynamically absorb, or compensate for, the forelengthening of the partially-crimped section of the inner frame. The structural mechanisms are designed to be able to compress or expand in a controlled manner, thereby changing the frame from an unstable system during loading or deployment into a stable system. In several embodiments, the structural mechanisms are design to compensate for internal compression forces on slotted strut members and provide dynamic frame stability, thereby ensuring improved frame integrity and patient safety. In several embodiments, the structural mechanisms provide frame stability by increasing lateral and/or circumferential bending stiffness similar to that of a diamond cell structure but without increasing crimp length as a diamond cell structure would. In several embodiments, the structural mechanisms advantageously prevent, or reduce the likelihood of, oval loading and deployment (e.g., by creating radially non-uniform, out-of-plane expansion of slotted strut members (e.g., axial beams or struts).

In accordance with several embodiments, an expandable and compressible frame can include a plurality of structural mechanisms (e.g., axial (longitudinal) connecting portions, such as strut components, within one or more chevron or diamond-shaped cells of a distal or outflow end portion of the expandable frame) that are capable or reducing in length (e.g., foreshortening) in a predictable manner. The structural mechanisms are configured to cause at least a portion of the frame (e.g., certain cells or struts) to buckle, deform, or bend in a predictable manner or in a desired direction (such as when the frame is being compressed in a non-uniform manner (e.g., a portion of the frame is being compressed while another portion remains expanded) through a funnel-shaped loader or when the frame is being compressed in a non-uniform manner as it is being recaptured within a delivery device). The structural mechanisms may comprise bendable axial struts that can shorten and accommodate the temporary non-uniform shape. Although the structural mechanisms may only be included in some of the cells of the frame, the predictable bending may cause adjacent cells or portions to also bend or crimp in a similar manner, thereby providing controlled bending, and compression, of the frame. In some configurations, the structural mechanisms may be biased in a particular configuration or shape so as to bend, deform, or crimp in a desired direction.

In some configurations, an implant (e.g., replacement heart valve) includes a self-expandable frame configured to transition between a compressed configuration and an expanded configuration. The frame includes a plurality of rows of cells (e.g., chevron-shaped cells) formed by cell struts. At least one cell of a distal-most row of the plurality of rows of cells includes a structural component that is adapted to prevent cell ovality during transition between the compressed configuration and the expanded configuration, and vice-versa. The structural component may include, for example, an axial strut connecting a distal apex of the at least one cell with a distal apex of a bordering cell in a row immediately above the distal-most row. Rows other than the distal-most row may include the structural component in addition to or as an alternative to the distal-most row.

**Figures 2E-1 to 2E-4** illustrate an embodiment of an inner frame **32** having axially asymmetric "bow spring" structural mechanisms. Figure 2E-1 shows the inner frame **32** in a crimped configuration and Figure 2E-2 shows the inner frame **32** in an expanded configuration. The bow spring structural mechanisms are built into one or more of the axial struts **202** extending between the chevron cells. Figure 2E-3 shows a side perspective view of the inner frame **32** in a partially-crimped, or partially-compressed configuration in which a proximal, or inflow portion, **32A** of the inner frame **32** is crimped or compressed but the distal, or outflow portion, **32C,** of the inner frame **32** is still fully expanded. With reference to Figure 2E-3, the V-shaped struts forming the top boundaries of at least the distal-most row or ring of cells fold up or compress prior to the V-shaped struts forming the lower boundaries of the distal-most row or ring of cells. Therefore, the distance between the endpoints of the bowspring axial struts **202** shortens during crimping. The bowspring axial struts could be removed but this could result in the frame being more flimsy. Figure 2E-4 shows a top view of Figure 2E-3 with the inner frame **32** in the same configuration. As shown in Figures 2E-3 and 2E-4, the bowspring axial struts **202** are designed to dynamically compensate for compression during device loading so as to avoid ovality. The bowspring axial struts **202** deform in a stable and predictable manner. The bowspring axial struts **202** may advantageously not elongate when crimped such that the frame crimp length does not increase during loading or deployment. The laser cut pattern of the bowspring axial struts **202** may comprise a narrow slot to facilitate non-lengthening (e.g., no forelengthening) of the frame during loading, deployment, and/or recapture. The bowspring axial struts **202** can be created at an angle less than perpendicular or perpendicular to a long axis of the frame **32,** as desired and/or required. The performance of the bowspring feature (e.g., bowspring axial struts **202**) is governed by the geometry of the intended bending region. Within this bending region, the length, wall thickness, strut width, lasercut arc, and/or taper region directly affect the degree of bending and strain experienced by the material. The embodiment shown in Figures 2E-1 to 2E-4 depicts a bowspring axial strut **202** wherein the intended bending region has a tapered strut width, which reduces to a minimum at the midpoint of the bowspring are, and an arched shape, generated by the lasercut pattern, which predisposes the intended bending region to bend in the desired direction. The ratio of the bowspring features' wall thickness to strut width ensures the bending is predictable and mostly unidirectional. In some implementations, the length of the bowspring axial strut **202** is tailored to ensure the required compressive travel is within material limits.

The bowspring embodiment in Figures 2E-1 to 2E-4 demonstrates a mechanism to compensate for frame forelengthening under compression, wherein the bowspring axial struts **202** dynamically reduce in length. The bowspring axial struts **202** in Figures 2E-1 to 2E-4comprise single curved struts that bow to one side in a predictable manner. As can be seen in the transition between Figure 2E-2 and 2E-4, the bend in the bowspring axial struts **202** becomes more pronounced and the bends all bow in a uniform, single direction. The principles of the mechanism work the same in reverse, wherein a pre-shaped bowspring mechanism under tension could dynamically elongate to compensate for the progressive forelengthening of the chevron-style frame design as it is loaded/deployed from its delivery device or system.

The bowspring mechanism (e.g., bowspring axial struts **202**) may be suitable for frames constructed of nitinol or any other super-elastic shape-memory alloy. This mechanism may also be employed for frames comprised of steel, cobalt-chromium, or other alloys, ensuring a conically crimped implant remains circular as it is diametrically reduced along its length. Use of this design in a frame made of these materials would remain deformed and be beneficial for use in applications where forcing local regions of a frame radially inward or outward is desired, such as to generate an hourglass shape (inward) or anchoring protrusions (outward).

The ability of the axial strut (e.g., bowspring axial strut **202**), the part of the unstable chevron cell structure under compression, to dynamically reduce in length during device (e.g., implant) loading, can be achieved by via number of different mechanisms, of which the bowspring concept is one. Another mechanism to achieve dynamic length change is to seed the axial beam with a multitude of latitudinal lasercut windows that could close or open to balance the compressive forces exerted on the strut during loading. Another mechanism to achieve dynamic length change of the axial beam is to build in a slot and pin mechanism, wherein the proximal section of the axial beam or strut terminates in a pin which engages a slot in the distal section of the axial beam or strut. As the frame is loaded, the pin can translate along the slot, thereby balancing the forelengthening of the chevron design, and when fully expanded and experiencing anatomical forces, the pin can lock to ensure a dependable frame structure.

The degree of axial asymmetry may vary. **Figure 2F-1** illustrates an embodiment of an inner frame **32** having highly asymmetric "bow spring" structural mechanisms and **Figure 2F-2** illustrates an embodiment of an inner frame **32** having minimally asymmetric "bow spring" structural mechanisms. The bowspring structural mechanisms may also be axially symmetric. **Figures 2G-1, 2G-2, and 2G-3** show various views of an embodiment of an inner frame **32** having symmetric dual "bow spring" structural mechanisms. The dual bow spring structural mechanisms comprises a pair of struts that bow to opposite sides similar to how a coin purse functions. Figure 2G-1 shows a close-up view of one symmetric dual "bow spring" structural mechanism while the inner frame is in a crimped or compressed configuration. Figure 2G-2 shows the inner frame **32** in an expanded configuration. Figure 2G-3 shows the inner frame **32** in a partially-crimped, or partially-compressed configuration in which a proximal, or inflow portion, **32A** of the inner frame **32** is crimped or compressed but the distal, or outflow portion, **32C,** of the inner frame **32** is still fully expanded. If a frame has a curved profile in the region of interest, as is the case with the hourglass profile of the inner frames **32** described herein, the out-of-plane frame expansion may convert the slot within the chevron cell into a dual how spring mechanism. The dual bow spring mechanism converts compressive loads that, if left unchecked or uncompensated for would lead to uncontrolled buckling, into a controlled bending of the bow spring struts.

### Anchor Features

In accordance with several embodiments, the anchors **37** of an expandable frame (e.g., the inner frame **32** of a dual-frame replacement heart valve) may be formed without the use of foam cushions on the anchor tips that contact native heart tissue. The anchors may include non-foam and/or non-fabric dampeners made from flexible material (e.g., metal or metal alloy material) that is attached to an anchor tip that can be bent, deformed, or contoured to provide a cushioning effect. In some embodiments, the dampeners or anchor tips are designed to be "softer", or more cushioned, in one direction to reduce conduction disturbances (e.g., conduction disturbances caused by pressure applied to a septal wall by a rigid anchor tip portion) and more rigid in the other opposite direction to preserve capture of native valve leaflets. The anchor tips may also have reduced anchor profiles to facilitate easier procedural navigation and placement of the replacement heart valve. The anchor tips may be further designed so as not to puncture heart anatomy (e.g., no sharp edges and provide a cushioning effect). The anchor tips may additionally be designed to reduce loading forces in the catheter or to make the loading forces more predictable.

**Figures 2G-4A, 2G-4B, 2G-5, 2G-6, 2G-7, 2G-8, 2G-9A, 2G-9B, 2G-10, 2G-11A, 2G-11B, 2G-11C, 2G-12, 2G-13, 2G-14, 2G-15, 2G-16, and 2G-17** illustrate various views of embodiments of atraumatic anchor tips of an expandable frame of a replacement heart valve. In particular, **Figures 2G-4A, 2G-4B, 2G-5 and 2G-6,** illustrate embodiments of an attachable tip, or attachable anchor dampener, **37A,** and **Figures 2G-7, 2G-8, 2G-9A, 2G-9B, 2G-10, 2G-11A, 2G-11B, 2G-11C, 2G-12, 2G-13, 2G-14, 2G-15, 2G-16, and 2G-17** illustrate other embodiments of an attachable anchor tip, or padded tip, **37B.** The embodiments of **Figures 2G-4A, 2G-4B, 2G-5, 2G-6, 2G-7, 2G-8, 2G-9A, 2G-9B, 2G-10, 2G-11A, 2G-11B, 2G-11C, 2G-12, 2G-13, 2G-14, 2G-15, 2G-16, and 2G-17** may not incorporate the use of foam padding and may or may not incorporate the use of a cloth covering. Thus, a cloth covering may be optional in accordance with these embodiments. The anchor tips may be incorporated into all, some, or one of the anchors.

In more detail, **Figures 2G-4A and 2G-4B** illustrate one embodiment of an attachable anchor tip or dampener **37A** which can be attached to an anchor **37** of inner frame **32** of a dual-frame valve prosthesis. The dampener **37A** may be a single, thin polymeric (e.g., plastic or elastomeric) or metal strip (e.g., or other material flexible enough to be easily bent). For instance, the dampener **37A** of Figure **2G-4B** has a thin strip shape that is bent over the distal tip (e.g., upwardly-extending tip when in an expanded configuration) of the anchor **37** to form a saddle-like design. In some configurations, the dampener **37A** is formed of a flat raw material (e.g., a thin metal material). Alternatively, the dampener **37A** may be formed from tubing, may be 3D printed, and/or may be formed of wire material. The material may include but is not limited to nitinol, cobalt chrome, stainless steel, or polymer material. As the dampener **37A** contacts anatomical tissue, a radius of the bent loop portion increases due to the flexibility of the material, thereby resulting in a "cushioning" effect. The dampener **37A** may be adhered to the anchor **37** via adhesive, welding, suture, or other attachment mechanism. For example, the dampener **37A** can be tied to the anchor **37** using threads or wires inserted through one or more suture holes **37A-1** formed on the end portions of the dampener **37A.** Different shapes or designs can be implemented. For example, **Figure 2G-5** illustrates another embodiment of a dampener **37A** which has a plurality of slits **37A-3** so as to reduce vibration when there is an external impact on the dampener **37A.** The plurality of slits **37A-3** may also cause a fanning out of the contact surface to increase surface area. Such a dampener **37A** can also provide a cushioning effect while protecting the tip of the anchor **37.** The dampener **37A** can be tied to the anchor **37** of inner frames **32** as shown in **Figure 2G-6** by suturing around end portions **37A-4** of the dampener **37A** using threads or wires **37A-2** wrapped around the end portions **37A-4** and/or inserted through one or more suture holes **37A-1** formed on the end portions **37A-4** of the dampener **37A.**

**Figures 2G-7, 2G-8, 2G-9A, 2G-9B, 2G-10, 2G-11A, 2G-11B, 2G-1C, 2G-12, 2G-13, 2G-14, 2G-15, 2G-16, and 2G-17** also illustrate embodiments of attachable anchor tips similar to those illustrated in **Figures 2G-4A, 2G-4B, 2G-5 and 2G-6** except that the attachable tips in **Figures 2G-7, 2G-8, 2G-9A, 2G-9B, 2G-10, 2G-11A, 2G-11B, 2G-1C, 2G-12, 2G-13, 2G-14, 2G-15, 2G-16, and 2G-17** may be made of a flat/thin raw material or a thicker rigid material. For instance, **Figure 2G-7** shows a tube-shaped attachable tip **37B** that may have horizontally-formed slits **37B-3A** at one side (e.g., front side) which allow inward flexion while preventing outward flexion. The slit cuts **37B-3A** may help to maintain rigidity for leaflet capture. The tube-shaped attachable tip **37B** further includes open cuts **37B-3B** on the opposite side (e.g., radially inward side facing the inner frame **32**) which allow inward flexion. The slits **37B-3A** and the open cuts **37B-3B** can be formed, for example, by laser cutting a flexible hypotube. The tube-shaped attachable tip **37B** can distribute and dampen loads and reduce force applied inside the patient's body, and further the slits **37B-3A** can maintain for rigidity for leaflet capture. An optional padded anchor tip can be attached to a top of the tube to distribute and dampen load.

**Figure 2G-8** shows a double half loop attachable tip **37B** design that includes an outer half loop **37B-4A** (the loop that is farther from the inner frame **32**) and an inner half loop **37B-4B** (the loop closer to the inner frame **32**) that provide asymmetric stiffness. The half loop shapes may advantageously facilitate distributing of load. The inner half loop **37B-4B** may be thicker than the outer half loop **37B-4A** and thus more rigid for maintaining reliable leaflet capture. The outer half loop **37B-4A** may optionally incorporate a plurality of relief cuts **37B-3C.** The outer half loop **37B-4A** is designed to provide a cushion effect to aid in reducing conduction disturbances and in decreasing the amount of force applied to the anatomy (e.g., septum, annulus). Similar to other attachable tip, the double half loop attachable tip **37B** may have one or more suture holes **37B-1A** for attaching the half loops to the inner frame **32** or anchor tip **37** by suturing or other attachment method. In addition, the double half loop attachable tip **37B** may have upper suture holes **37B-1C** and lower suture holes **37B-1B** for suturing the outer half loop **37B-4A** and the inner half loop **37B-4B** together. The half loops may be laser cut from a flat sheet or tube (may be the same tube or different tubes of different thickness so that the inner tube is thicker) and shape set to the same shape using the same tooling. One or both of the half loops may optionally be covered with a sleeve (e.g., cloth sleeve).

**Figure 2G-9A and 2G-9B** show a side view and a front view, respectively, of another embodiment of an attachable anchor tip **37B** that includes a half loop that terminates in a flexible spring-shaped end. The attachable anchor tip **37B** of Figures 2G-9A and 2G-9B may be rigidly and fixedly attached to the anchor **37** by suturing one end having suture holes **37B-1** to the anchor **37** while the opposite end (e.g., spring-shaped end) thereof may remain free and unattached. The spring-shaped end of the half loop attachable tip **37B** may allow the whole anchor to deflect off of sensitive anatomy (e.g., septal wall), thereby providing a cushioning effect, reducing force applied to the anatomy along the conduction pathway, and reducing conduction disturbances. The entire anchor tip design may be laser cut from a flat sheet and then the half loop portion can be shape set into a half loop shape without requiring the spring-shaped end to be shape set. **Figure 2G-10** shows an anchor tip loop attachable tip **37B** similar to the embodiment of **Figures 2G-4A and 2G-4B,** but further includes a wire **37C-1** wrapped over at least a portion of the loop to provide further springy and cushiony effects. The wire may only extend along an outer side and top of the loop (e.g., side configured to contact a septal wall or annulus) and not along the entire loop. The wire **37C-1** allows the anchor tip to deflect off of sensitive anatomy as opposed to pressing rigidly into it. On the inward side (e.g., leaflet side) of the loop, there may be no wire wrap in order to preserve leaflet capture ability. The attachable tip **37B** of **Figure 2G-10** can be also made by laser cutting a flat sheet to have a loop shape and the wire **37C-1** can be wrapped through holes cut through a thickness of the loop. The ends of the loop may be sutured to the anchor **37** via suture holes **37B-1** or other attachment mechanisms as described previously.

**Figures 2G-11A, 2G-11B, 2G-11C, 2G-12, 2G-13, 2G-14, and 2G-15** illustrate other embodiments of an attachable tip **37B** of one or more anchors of an inner frame. The attachable tips shown in these embodiments may have more than two arms. For example, referring to **Figures 2G-11A and 2G-11C,** the attachable tip **37B** may include first opposite arms **37C-2** having suture holes **37B-1** for attachment to the anchor at each end thereof, and second opposite arms **37C-3** having arms of a generally continuous width and having free, unattached ends. The attachable tip **37B** can be formed of a wire, a thin metal, or any flexible polymeric or metallic material to bend over the anchor distal tip as shown in **Figure 2G-11C,** and the first opposite arms **37C-2** can be attached to the anchor **37** by suturing sutures or threads **37B-2** through the suture holes **37B-1** while the second opposite arms **37C-3** may be free at their ends as shown in **Figure 2G-11B. Figures 2G-12 to 2G-15** illustrate various embodiments of attachable tip designs similar to **Figure 2G-11A.** That is, an attachable tip **37B** of **Figure 2G-12** may have circular ends at the second opposite arms **37C-3,** and an attachable tip **37B** of **Figure 2G-13** may be similar to that of **Figure 2G-12** but may have a circular shape at the center with a center hole **37B-4** forming a larger surface contact with the anatomy. **Figures 2G-14 and 2G-15** are variants of **Figures 2G-12 and 2G-13,** respectively, with more than two second opposite arms **37C-3.** The number of free, unattached arms may vary.

**Figures 2G-16 and 2G-17** illustrate an attachable tip for attaching to the end of the inner frame **32** or the anchor **37** similar to the above-described embodiments. The attachable tip of **Figures 2G-16 and 2G-17** has a symmetric configuration so that they can be folded so that the upper and lower tips can be in contact and attached to the inner frame **32** by suturing through suture holes **37B-1.**

**Figures 2G-18A, 2G-18B, 2G-19, 2G-20A, 2G-20B, 2G-21A, 2G-21B, 2G-22, 2G-23A, 2G-23B, 2G-24A, 2G-24B, 2G-25A, 2G-25B, 2G-26A, 2G-26B, 2G-26C, 2G-27A and 2G-27B** illustrate various embodiments of anchor tips for anchors designed to capture native heart valve leaflets (e.g., native leaflets of the mitral or tricuspid valve). The anchor tip configurations may advantageously provide a cushioning function without use of, or a reduction in an amount of, foam or cloth components. In accordance with several embodiments, the anchor tips represent modifications to existing frame material (e.g., modifications to on, some or all, of the anchors of the frame themselves) instead of attachments to the anchors, such as embodiments described previously. The anchor tip designs may be incorporated into one, some, or all of the anchors of a frame. In some implementations, the anchor tips comprise non-fabric and/or non-foam anchor tips made from a flexible material (e.g., metal or metal alloy material such as nitinol) that can be bent, contoured, or depressed to provide a cushioning effect on at least a portion of the anchor tip.

**Figures 2G-18A and 2G-18B** illustrate a dual-layer hoop anchor forming two independent hoops stacked on top of one another. The hoops may be cut from the anchor tube stock and then shape set to separate the independent hoops out of plane to double the contact surface area (as shown best in Figure 2G-18B). **Figure 2G-19** illustrates a dual inward spiral anchor formed by two independent spirals positioned side by side that may be formed by cutting an anchor tube stock. The spirals can deflect to provide a cushioning effect. This anchor design may not require any shape setting or welding. A thickness **D** of the spirals of the dual inward spiral anchor may be, for example, from 100 µm to 200 µm.

**Figures 2G-20A and 20B** each illustrate a heart-shaped hoop anchor **37** formed by a single hoop with two lobes such that the center of the heart shape can deflect to cushion the anchor load. In particular, **Figure 2G-20A** may have a length **L** which narrows to slip past chordae tendineae and a height **H1** which deflects to reduce impact loading and wear on a leaflet or annulus as a shock absorber. In addition, the heart shaped hoop anchor **37** of **Figure 2G-20A** may have a sleeve or cloth sock **37C** around the anchor **37.** The heart shaped hoop anchor **37** of **Figure 2G-20B** may optionally have a snap configuration where a top member **37CC** of the hoop snaps into a base **37CD** of the hoop for shape setting and/or for reducing a crimped length (e.g., by several millimeters). Upon uncrimping, such a hoop snap becomes free. The heart shaped hoop anchor design of either **Figures 2G-20A or 20B** may not require any shape setting or welding.

**Figures 2G-21A and 2G-21B** each illustrates a bunny ear cushion anchor configuration formed by two outward facing spirals next to each other which bend and separate upon loading to distribute the load and cushion the anchor contact with the heart anatomy. **Figure 2G-21A** illustrates a narrower (e.g., **L1** is about 2 mm while **L2** is about 6-7 mm) and taller (e.g., **H2** is 3-4 mm) anchor profile compared to **Figure 2G-21B,** thereby allowing easier slip through or removal from chords. On the other hand, the wider version of **Figure 2G-21B** may allow a wider, more distributed load when the anchor **37** or the inner frame **32** is positioned against the native valve annulus or leaflet. One or both of the spirals may optionally be covered with cloth sleeves to facilitate spreading. No shape setting or welding may be required.

**Figure 2G-22** illustrates a collapsible loop cushion anchor design formed by two outward facing loops similar to the embodiment of Figure 2G-21A with additional support from a ledge **37D** that creates a stiffer (e.g., more rigid) loop when contacting from the distal end and a softer loop while contacting from the proximal end, thereby allowing easier disengagement from interaction with chordae tendineae anatomy when pulling out the valve prosthesis. The anchor may optionally be covered with a cloth sleeve or sock **37C.**

**Figures 2G-23A and 2G-23B** each illustrates a wire wrap anchor tip design where the anchor has a plurality of holes **37B-1** (e.g., laser cut holes) through which a wire **37C-1** can be wrapped loosely, creating a soft "cushioned" tip of the anchor **37.** In particular, **Figure 2G-23A** may optionally include sleeve or cloth sock **37C** covering the wire **37C-1** and wire ends **37F** may be welded or crimped as a stopper **37E,** and **Figure 2G-23B** may include a radiopaque marker **37G** to indicate deflection from annulus contact. Wire ends **37F of** **Figure 2G-23B** may be welded together. The wires **37C-1** of **Figures 2G-23A and 2G-23B** may be made of nitinol, cobalt chrome, stainless steel, polymer, radiopaque metal, or the like. This anchor tip design may not require any shape setting.

**Figures 2G-24A and 2G-24B** illustrate an anchor having a thin-walled hoop cut into an end of the anchor tip, where the hoop can deflect so that load can be distributed when the anchor is in contact with an object (e.g. native heart anatomy) over a larger surface area. In the illustrated embodiment, a circular shape (with a diameter **R** of, e.g., 2-4 mm) is cut into the end of the anchor tip. When compressed by contact with tissue, the circular shape forms an oval shape (as shown in Figure 2G-24B with a diameter **L3** of, e.g., 3-7 mm). The thin-walled hoop can also deflect around or between chordae when in contact. In particular, the anchor tip of **Figure 2G-24B** is more tolerable to greater contact loading due to the greater contact surface area to distribute. This anchor tip design may not require any shape setting or welding.

**Figures 2G-25A and 2G-25B** illustrate zig-zag spring anchors each having a zig-zag pattern cut into the tip of the anchor **37** to provide load distribution and cushioning. The zig-zag spring anchor of **Figure 2G-25A** may be a slanted zig-zag pattern creating an angle greater than 0° but less than 90° with a length or width **L4** (e.g., 2-3 mm) and a height **H3** (e.g., 3-5 mm), while the zig-zag spring anchor of **Figure 2G-25B** may by curved or bent (e.g., at a right angle of 90° or approximately 90°). This anchor tip design may not require any shape setting or welding.

**Figures 2G-26A, 2G-26B and 2G-26C** illustrate a whisk tip anchor formed by looping multiple wires **37C-4** over and passing through holes **37J** around the periphery of a small circular plate **37H** to form two to four or more than four wire hoops, where a center rectangular hole **37I** of the plate **37H** can fit over and be sutured onto the end of an anchor arm. Figure 2G-26A shows a side view of the whisk tip anchor and FIG. 2G-26B shows a top view of the circular plate **37H** and a close-up, side view of an anchor arm that includes a tip configured to receive the hole **37I** of the plate **37H.** The ends of the wires **37C-4** may be laser welded to the circular plate **37H.** The wires **37C-4** that are looped around may optionally be covered be a sleeve or cloth sock **37C.** **Figure 2G-26C** illustrates a top view of the whisk tip anchor, looking from the top of the wires **37C-4.** The wires may comprise nitinol or other shape memory material. For nitinol wires, a different A_{f} temperature may be used for the nitinol wires than for the inner frame 32 (e.g., an A_{f} temperature closer to body temperature), which may facilitate the nitinol wires providing a softer anchor cushion.

**Figure 2G-27A** illustrates a cylindrical braid tip anchor formed by a fine wire **37N** braided into a cylinder and looped over the anchor **37** to provide cushioning during anchor loading. The fine wire could be nitinol wire, cobalt chrome wire, stainless steel wire, polymer wire, or radiopaque metal wire, and the wire may be tube-shaped. In addition, an optional sleeve or cloth sock **37C** may be looped around the fine wire **37N.** Figure **2G-27B** shows another embodiment of a cylindrical braid tip anchor having a cone shape formed by an inverted cylinder braid tip. In both cylindrical braid tip anchors in **Figures 2G-27A and 2G-27B,** the end of the inner frame 32 or the anchor 37 may be split into two pinching arms 37P for securing the wire ends with an optional crimp sleeve **37O.** For nitinol wires, a different A_{f} temperature may be used for the nitinol wires than for the inner frame 32 (e.g., an A_{f} temperature closer to body temperature), which may facilitate the nitinol wires providing a softer anchor cushion.

### Co-Organizing Dual Frame Features

In accordance with several embodiments, it is desirable to provide complementary features on the structural components (e.g., inner and outer frames) of dual-frame transcatheter devices (e.g., prosthetic implants or replacement heart valves). These complementary features may be intended to ensure co-organization of the inner and outer frames. The co-organizing or complementary features and may or may not be in contact in the expanded and/or crimped states. However, these co-organizing features may advantageously interact to help promote alignment of the inner and outer frames during loading and deployment steps, and during any subsequent recapture steps.

The co-organizing or complementary features may be beneficial for device performance, ensuring organized frames for low loading/recapture force, symmetric device profiles during deployment for procedural consistency, and reduced strain concentrations in the frames that commonly result from asymmetric loading and that reduce device durability. Without the use of such co-organizing or complementary features, the structural components (e.g., inner and outer frames) can work against each other (e.g., through fighting for space) and can result in an undesirable asymmetric arrangement that can lead to a more difficult procedure or degradation of the device (e.g., prosthetic implant).

Transcatheter implants (e.g., replacement heart valves) are typically designed for two states, or configurations: an expanded state (e.g., following implantation at a desired location) and a crimped state (e.g., within a delivery device upon manufacture or upon recapturing). Between these two states, the implant undergoes some level or form of transition, such as diametric reduction (e.g., during loading) or expansion (e.g., during implant deployment). This transitory state between the expanded state and the crimped state, often an afterthought in design, can be important, as it can affect the ease and/or safety of the implantation procedure. In some instances, multi-frame (e.g., dual-frame) implants may have undesirable frame-to-frame interaction that creates instability within the implant and can lead to the implant presenting in an undesired, asymmetrical fashion to the anatomy during deployment, which can complicate achieving a successful implantation procedure. Another consequence of negative interaction between the frames can damage the implant cloth or skirt fabric material (e.g., resulting in leaks) and/or can damage the frames (which can lead to reduced frame durability and fatigue or failure).

Various co-organizing or complementary frame features may be designed to ensure that the inner and outer frames, or portions of the frames, remain aligned and organized throughout the transitory states. **Figure 2H** shows a side view of an embodiment of an outer frame **34** including co-organizing frame features. The co-organizing frame features include features of a proximal portion **34A** of the outer frame **34** and a distal portion **34C** of the outer frame **34.** The specific co-organizing frame features will be discussed in more detail below. In some implementations, the co-organizing or complementary frame features are designed to only engage one another during the transitory states. The co-organizing or complementary frame features may be designed to, for example, reduce degrees of freedom, link up to protect delicate sections or portions of the implant, or work like a seal to progressively join the frames in an organized fashion.

As one example, an outer frame of a dual-frame implant may include a structural component configured to engage with a portion of the inner frame of the dual-frame implant upon expansion and/or compression of the dual-frame implant (e.g., during a transition state) so as to reduce a likelihood of rotational and/or translational movement between the outer frame and the inner frame. **Figures 21-1 to 2I-3** illustrate various proximal eyelet designs configured to reduce rotational and/or translational movement between an outer frame and an inner frame of a dual-frame implant. **Figure 21-1** shows a close-up view of proximal portions **32A, 34A** of embodiments of the inner frame **32** and the outer frame **34** during a transitory state in which the proximal portions **32A, 34A** are in a crimped configuration but the distal portions **32C, 34C** are still expanded. As shown, the proximal eyelets of the outer frame **34** can include a hammer-head design to provide uniform spacing between the eyelets. The hammer-head design includes thickened side walls with flat edge surfaces for the upper eyelet and the lower eyelet of the outer frame **34.** The thickened flat side surfaces of the eyelets are configured to contact and abut against each other to provide the uniform spacing (due to uniform dimensions of the design). **Figure 21-2** shows a portion of a flat cut pattern of an embodiment of the outer frame **34** that shows one eyelet portion having a hammer-head design adapted to restrict rotational freedom of movement only. **Figure 21-3** shows a portion of a flat cut pattern of an embodiment of the outer frame **34** that shows two adjacent eyelet portions having a hammer-head design configured to restrict rotational and/translational freedom of movement. As shown, the eyelet portions (shown at the top of Figures 2I-2 and 2I-3) include two central extensions (e.g., nubs, protrusions, tabs) on one side of a central eyelet configured to engage with a cut-out feature (e.g., recess, notch, indentation) on an opposite side of an adjacent central eyelet to restrict translational height movement when adjacent eyelet portions are engaged. The upper and lower eyelets include thickened side portions that are wider/thicker on one side than on the other side. Other designs and shapes may be used to facilitate co-organization between eyelet portions of the outer frame **34.**

**Figures 2J-1 and 2J-2** help to illustrate another example of a co-organizing frame feature (e.g., slot, opening, or guide structure) of an outer frame designed to straddle an inner frame axial strut (e.g., the inner frame axial strut extends outward within the co-organizing frame feature of the outer frame) to facilitate alignment of the outer frame and the inner frame during transition between an expanded configuration and a compressed configuration, and/or vice-versa. The outer frame may have multiple co-organizing frame features spaced circumferentially around the outer frame to straddle multiple inner frame axial struts. **Figure 2J-1** illustrates a dual-frame design without co-organizing frame features. As shown, overlaid axial beams of an outer frame with a high degree of curvature in an expanded state results in non-uniform geometry in a transitory state. **Figure 2J-2** illustrates a dual-frame design with co-organizing frame features. The outer frame **34** includes the hammer-head proximal eyelet design described previously and shown in Figure 21-1. The complementary or co-organizing frame feature of the inner frame **32** is an axial beam or strut **212** on a proximal, or inflow aspect, **32A** of the inner frame **32.** The complementary or co-organizing frame feature of the outer frame **34** may be a wide diamond cell junction at the proximal, or inflow, aspect **34A** of the outer frame **34,** which overlaps a tightly-radiused segment of the shape profile. In some embodiments, the co-organizing frame feature of the outer frame **34** comprises a C-shaped or U-shaped junction (e.g., forming a slot, or guide receptacle or other mechanism) designed to straddle the corresponding inner frame axial strut **212** for alignment. As the dual-frame implant is loaded into a delivery device, the radiused outer frame C-shaped junction bends inward and straddles the inner frame axial strut **212.** which acts as a vertical rail and helps keep the outer frame **34** perfectly or nearly perfectly aligned to the inner frame **32** throughout loading, recapturing, repositioning. Once the implant is fully crimped, the curvature of the outer frame co-organizing frame feature (e.g., C-shaped or U-shaped junction) is straightened out and disengages from the inner frame axial strut **212.** The co-organizing frame features may not be engaged (or may not interact) when the implant is in a fully-expanded configuration.

**Figure 2K-1** illustrates how an outer frame without co-organizing frame features can adversely interact with an anchor **37** on an inner frame of a dual-frame valve prosthesis during crimping. **Figure 2K-2** shows how an embodiment of an outer frame **34** can be designed to include a co-organizing frame feature such that distal outflow portion **34C** of the outer frame **34** avoids interaction with inner frame anchors **37** during crimping. The distal outflow portion **34C** of the outer frame **34** may be shaped and adapted such that the distal apexes of the distal cells of the outer frame **34** do not align with or overlap with the distal anchors (e.g., ventricular anchors) **37** of the inner frame **32.** The anchors **37** may instead be designed to be located between the distal apexes of the distal cells of the outer frame **34** during crimping.

### Proximal/Inflow/Inlct Strut Features

**Figure 2J-3** illustrates an embodiment of an inner frame **32** design in which the proximal, or inlet, struts are at uneven, staggered, or offset heights in order to reduce a total (i.e., maximum) force required to retrieve and recapture a fully-expanded or partially atrially-expanded replacement heart valve, or valve prosthesis. The offset, staggered, or uneven heights distributes the force during recapture, rather than having one large spike at once as all the struts are pulled into a delivery system simultaneously, as is the case when the heights are all uniform and not offset (e.g., are axisymmetric). **Figure 2J-4** is a graph showing expected results of force reduction using the offset height design of Figure 2J-3. Referring to **Figure 2J-3,** proximal, or inlet, struts **202** of an inner frame **32** may have different heights (e.g., height difference **H4**) in a way that adjacent struts are offset relative to one another. The alternating, offset heights allow half of the struts **202** to be pulled into the delivery system first, and the remainder to be pulled in subsequently, thus creating two small spikes in recapture force rather than one large spike as shown in **Figure 2J-4.** The force reduction may be, for example, a 25-50% reduction in force. That is, the offset configuration can create sequential seating of the struts **202** inside a pusher **506** or capsule tip of a capsule subassembly **306,** lower recapture forces, reduce tension on the recapture sutures, reduce force on the dual-frame valve, and reduce compression during the recapturing process. Accordingly, a reduction in force to load a valve prosthesis and recapture a valve prosthesis is expected. The recapture force reduction may result in less tension on the suture during recapture and less compression on the mid-shaft subassembly **22** during recapture. The staggered or offset heights may also help reduce risk of a strut catching on a dstial tip or edge of the capsule subassembly **306** as the implant **30** is recaptured within the capsule subassembly **306.** The heights of the struts **202** can be varied by, for example, changing the strut length (e.g., height above a connection point to a main frame body (e.g., cell structure)), angle, or the like. There may be two different heights, with the height alternating with each strut around a circumference of the frame. There may be more than two different heights (e.g., three different heights, four different heights), with different pairs or groups of struts having different heights.

In accordance with several configurations, an outer frame of a dual-frame implant may include a cantilevered or hinged attachment tab that allows attachment between the outer frame and an inner frame in a manner that allows an angle to be formed between the attached portions of the outer frame and the inner frame because the attached portion of the outer frame bends on an independent plane from the attached portion of the inner frame, thereby reducing a radius of curvature of the dual-frame implant along the region where the outer frame and the inner frame are attached. **Figures 2L-1 to 2L-3** illustrate various examples of an attachment or connection structure between proximal eyelets and connecting struts of an outer frame of a dual-frame valve prosthesis. **Figure 2L-1** shows that a bottom (e.g., distal-most or lower-most) eyelet of the eyelets **35** of the proximal tab 33 of the proximal portion **34A** of the outer frame **34** may be connected to the proximal end of one or more struts **34E, 34F** of the outer frame **34** by a bridge **34G.** The struts can include at least two outer strut legs **34E** connected to the bridge **34G.** The struts may further include at least two inner leg struts **34F** of which one end is coupled to an upper inner portion of a respective one of the at least two outer leg struts **34E.** The bridge **34G** may have a predetermined length between the lowermost eyelet of the eyelets **35** and a junction **C.** In addition, the at least two outer legs **34E** may extend downwards from the junction **C.** When at least one of the plurality of eyelets of each of the plurality of tabs of the outer frame **34** is engaged with at least one of the plurality of eyelets of the plurality of tabs of the inner frame, the bridge **34G** of each connection structure is in surface contact with a respective tab of the inner frame **32.** In several instances, this design may require tangency with the inner frame eyelets when the outer frame **34** and the inner frame **32** are aligned and engaged together, which can force a high radius of curvature profile that can result in high strain during crimping and a concentrated fatigue strain on the reverse taper of the junction C between the bridge **34G** and the proximal end of the struts.

**Figure 2L-2** shows another example of a linking element or connection structure of the outer frame **34.** In this example, the bridge **34G** has been substantially shortened compared to that shown in FIG. 2L-1. The bridge **34G** is not connected to the bottom, or distal-most, eyelet but is connected to a proximal-most, or upper, eyelet via an outer framework **34I** of the tab **33** that extends from the bridge **34G** and surrounds the more distal eyelets, thereby forming a "pop tab" configuration like the tab used to open a can of soda pop. The bridge **34G** in **Figure 2L-2** may have the same or shorter length than that in **Figure 2L-1.** Similar to the embodiment of **Figure 2L-1,** the at least two outer leg struts **34E** in **Figure 2L-2** may extend downwards from the junction **C.** The bridge **34G** of **Figure 2L-2** may advantageously separate planes of movement such that the tab **33** can bend along a plane independent of the outer framework **341,** the bridge **34G,** and/or the outer leg struts **34E** and independent of the attached portion of the inner frame **32.** Thus, the attached portion of the outer frame **34** can bend at an angle with respect to the attached portion of the inner frame **32,** thereby facilitating a reduced radius of curvature along the proximal inflow regions of the dual-frame implant or valve prosthesis.

**Figure 2L-3** shows still another example of a linking element or connection structure of the outer frame **34.** As shown in **Figure 2L-3** the bridge **34G** and junction C have been removed completely from the structure. The at least two outer leg struts **34E** are connected to respective sides of the upper-most, or proximal-most eyelet but do not connect to respective sides of the other eyelets, thus forming a "paper clip" configuration. extend downwards from the outer tab **33B,** and the inner tab **33A** can be spaced apart from the at least two outer legs **34E** along at least a portion of an edge of the inner tab **33A.**

In accordance with several embodiments, the geometry implementations of **Figures 2L-2** and 2L-3 advantageously eliminate the requirement for eyelet tangency with the connecting struts by creating an independent plane (e.g., bendable or cantilever tab portion) for eyelet attachment between one or more attachment eyelets of the inner frame **32** and outer frame **34** and provides more flexibility for future profile designs of the outer and inner frames. For example, the inflow struts on the bendable or cantilever tab portion of the outer frame may act as a cantilever that keeps the outer frame **34** closed until the capsule subassembly **306** is fully retracted.

**Figures 2L-4 to 2L-6** illustrate various embodiments of tabs **33** and/or eyelets **35** of proximal, or inlet, struts of an outer frame. In accordance with several implementations, these embodiments may advantageously prevent, or reduce the likelihood of, suture or tether loops being cow hitched, looped, or "locked" around a tip of a proximal, or inlet, strut during removal of the suture or tether during a step of releasing the valve prosthesis from attachment to the delivery system. Instead, the suture or tether loop can be readily disconnected from the outer frame **34** of the valve prosthesis through the uppermost or proximal-most eyelet **35A.** In particular, **Figures 2L-4 and 2L-5** show a linking element or connection structure of the outer frame **34,** similar to **Figure 2L-2** forming a "pop tab" configuration like the tab used to open a can of soda pop. However, the embodiments of **Figures 2L-4 to 2L-6** can be incorporated into the "paper clip" configuration or other configurations as well. The embodiments of **Figures 2L-4 and 2L-5** can be formed by laser cutting. An uppermost or proximal-most eyelet **35A** may have a half circle (semi-circle) shape (such as shown in **Figure 2L-4**), an oval shape (such as shown in **Figure 2L-5**), or a bean shape (such as shown in **Figure 2L-6**). The uppermost or proximal-most eyelet **35A** may have a generally rounded geometry as shown in these figures. Further, a height **H5** of an attachment hole centerline of the proximal-most eyelet **35A** may be varied (e.g., decreased) such that the suture or tether cannot catch, loop or hitch on the proximal tip of the proximal, or inlet, strut. With reference to **Figure 2L-6,** the proximal-most eyelet **35A** has a radius **R** that is greater than that of **Figure 2L-4** but smaller than that of **Figure 2L-5.** In one embodiment, the radius **R** may be about 0.1 mm to 0.3 mm. The height **H6** and the height **H7** combine to be the height **H5.** Either or both height **H6** and height **H7** can be reduced to reduce height **H5.** The height **H6** may range from 0.230 mm to 0.330 mm in some embodiments and the height **H7** may range from 0.520 to 0.580 mm in some embodiments. By reducing either or both height **H6** and height **H7,** and thus reducing height **H5,** the thickness of the suture or tether, in combination with the reduced height, prevents the suture or tether from looping, catching, or hitching, on the proximal tip of the proximal, or inlet, strut. The proximal tip of the proximal, or inlet, strut may also have a rounded or chamfered outside top geometry. For example, the proximal tip of the proximal, or inlet, strut may have a radius of curvature **R2.** In accordance with several embodiments, the radius of curvature **R2** is designed to be less than the height **H5.** The side geometry of the proximal, or inlet, strut may be straight in some embodiments (such as shown in Figures 2L-2 to 2L-5) as opposed to a "snowman" side geometry (such as shown in Figure 2L-1).

Different tab configurations particularly varying the eyelet configuration as described above can bring different advantages such as ease of manufacturing the outer frame, ease of attachment of the replacement heart valve (e.g., by suturing), reduction of tensile stress, etc. In accordance with several embodiments, a series of maneuvers (e.g., posterior, anterior, lateral, and medial maneuvers) may be performed during the tether/suture release step to provide an indication of any likelihood of hitching or looping.

**Figures 2M-1** and 2M-2 illustrate various radii of curvature profiles of a dual-frame valve when an inner frame and an outer frame are engaged. For instance, when the embodiment of the outer frame of **Figure 2L-1** is engaged with the inner frame, the outer profile may have a radius of curvature as shown in **Figure 2M-1,** while when the embodiments of **Figure 2L-2** or Figure 2L-3 are engaged with the inner frame, the outer profile may have a radius of curvature smaller than that in **Figure 2M-1,** as shown in **Figure 2M-2.** A high radius of curvature may make it challenging for a physician to capture chordae tendineae beneath the annulus of a mitral valve, for example, because the outer frame of the dual-frame valve prosthesis may have to be deployed at the same time the ventricular anchors reach their full diameter. Thus, by changing the configuration of the outer frame, more specifically, by changing the configurations of the eyelet and strut connection or attachment structures of the outer frame, the radius of curvature of the dual-frame heart valve prosthesis can be adjusted so as to delay the deployment of the outer frame in addition to reducing crimping strains at locations that undergo compound radial and circumferential bending due to the curvature of the profile, e.g., at the junction **C.** Thus, the dual-frame valve prosthesis may be designed to have a reduced radius of curvature at a proximal end when in the expanded configuration, as shown in **Figure 2M-2.**

In accordance with several embodiments, the implementations shown in **Figures 2L-2** and 2L-3 and 2M-2 provide flexibility to create a new cork profile for the dual-frame valve prosthesis. The connection structures shown in **Figures 2L-2** and **2L-3** and the more gradual profile or radius of curvature of **Figure 2M-2** may allow for a delayed release of the outer frame during delivery and may reduce both crimp strain and fatigue strain. The delayed release may be accomplished by using the inflow struts as a cantilever that keeps the outer frame closed until a delivery capsule (e.g., capsule subassembly **306** described below) is fully retracted. The reduced radius of curvature may provide a significant reduction in fatigue strain at junction **C** and an improved crimp strain distribution.

**Figures 2N-1 and 2N-2** illustrate an outer frame having the "pop tab" connection structure design of **Figure 2L-2** in an expanded configuration and shows the reduced radius of curvature profile of this design. **Figures 2N-3 and 2N-4** illustrate an outer frame having the "paperclip" connection structure design of **Figure 2L-3** in an expanded configuration and shows the reduced radius of curvature profile of this design.

**Figure 20-1** illustrates a dual-frame valve prosthesis in which an inner frame **32** and an outer frame **34** are engaged in a pre-expansion state where the outer frame **34** is not deployed. **Figure 20-2** illustrates a dual-frame valve prosthesis in which an inner frame **32** and an outer frame **34** are engaged in a capsule retracted state where the outer frame **34** is deployed. As described herein, by changing the linking or connection structure (e.g., shapes, connections, etc.) of a proximal portion of the outer frame **34,** it is possible to delay the deployment of the outer frame **34** as shown in **Figure 20-2.**

In some examples, the outer frame and the inner frame of the dual-frame valve prosthesis may be engaged by aligning and attaching one or more of the plurality of eyelets **35** thereof, for example, in a "snowman" method of inner and outer frame fixation. The larger diameter of the outer frame can be served to engage with the native anatomy for the purposes of sealing and securement in the large annulus native anatomy. The smaller inner diameter of the inner frame can serve to hold tissue leaflets of a prosthetic valve and can provide a smaller prosthetic valve diameter to reduce tissue bulk, pulsatile frame loading, and frame radial crimping forces. The dual-frame valve prosthesis structures can provide the above advantages by creating an appreciable difference between the expanded diameters of the inner and outer frames.

In certain embodiments, proximal eyelet portions of the inner frame and the outer frame may be engaged with each other adapting the "snowman" method of aligning eyelets of each frame and wrapped sutures multiple times through the aligned inner and outer eyelets to hold the frame struts together at the inflow side of the valve. To maintain the appreciable difference in expanded diameters between the inner and outer frames assembled using the "snowman" methods described above, sharp bends are needed to create space between the inner and outer frames, resulting in increased strains and crimp loading forces. For instance, referring to **Figures 2P-1 and 2P-2** which show eyelets **35** of each of the inner frame **32** and the outer frame **34** being engaged with each other by a suture looping around each of the eyelets a predetermined number of times to secure the attachment of the eyelets. Here, the outer frame **34** may have an attachment configuration corresponding to the example of **Figure 2L-1** described above.

**Figures 2P-3 and 2P-4** illustrate another example of connecting or engaging the inner frame **32** and the outer frame **34** of a dual-frame valve prosthesis. For example the inner frame **32** and the outer frame **34** may include corresponding, or complementary, engagement or attachment features that allow for an angle to be formed between the engaged portions of the inner frame **32** and the outer frame **34** at the attachment point. In the examples of **Figures 2P-3 and 2P-4,** an inner lock tab member **33A** of the tab **33** of the outer frame **34** comprises a puzzle piece lock tab end configured to fit within a corresponding slot on a proximal inflow end of a corresponding tab or strut **202** of the inner frame **32,** thereby providing a compact mechanical lock between the strut of the inner frame **32** and the inner lock tab member **33A** of the outer frame **34.** As shown in **Figure 2P-3,** the "puzzle piece lock tab" design may advantageously enable a larger angle between the inner frame and the outer frame at the attachment point than the embodiment of **Figures 2P-1 and 2P-2** and may provide a more gradual curve profile for the outer frame **34,** thereby reducing strain and crimp loading force.

For certain embodiments, the inner lock tab member **33A** comprises a joint (e.g., dovetail-shaped joint) that fits within a correspondingly-shaped slot of the strut **202** of the inner frame **32**(e.g., a simple planar fit), thus reducing a load off the suture by the mechanical lock between the interacting metal components of the frames. The connection or engagement can involve use of a single suture lashing to keep the two frames coplanar at the joint point or mechanical fit interface, or can optionally involve off-center/off-axis laser cutting that can provide a tapered or beveled fit between the tabs **33** of the outer frame **34** and the inner frame **32** to reduce the suture usage while keeping the tab **33** of the outer frame **34** and the inner frame **32** coplanar by the spring force of the tab holding the frames together as shown in **Figure 2P-4. Figure 2P-4** also shows a detailed cross-section view along section line B-B. The detailed cross-section view shows in more detail how the interface between the inner lock tab **33A** and the strut tab opening of the inner frame **32** can optionally be beveled with off-axis laser cutting to lock the metal tabs of the inner frame and the outer frame together without requiring any sutures.

In another example, referring to **Figures 2P-5 and 2P-6,** the proximal ends of the inner frame and outer frame may be connected or joined using a dovetail joint connection structure. This embodiment can provide a dovetail joint in which a proximal end or strut of the inner frame **32** comprises a dovetail shape (e.g., cut with a perpendicular laser cutting operation), while a strut of the outer frame **34** has an angled cut to match the angle of the dovetail joint member on the inner frame **32,** thereby forming a dovetail joint or matched fit that allows fitting the parts together one way but preventing the parts from pulling apart any other way. The dovetail angle of the inner frame **32** and the off-center taper angle of the strut of the outer frame **34** can be adjusted to allow for different angle between inner frame **32** and the outer frame **34** (e.g., 45 degrees, 60 degrees, 90 degrees, or other angle). Two alternative optional techniques for preventing the inner and outer frames from coming apart (e.g., inner frame dovetail member backing out of dovetail groove on outer frame under loading conditions) include (1) that eyelets **35** of the inner frame and the outer frame may be optionally engaged to each other by a tensioned suture or tether wrapping therethrough and/or (2) the outer frame may comprise a snap lock **34J** integrally or detachably connected to a strut of the outer frame to secure the attachment of the inner frame **32** and the outer frame **34,** as shown in **Figure 2P-6.**

The joint structure as illustrated in **Figures 2P-3 and 2P-4 or Figures 2P-5 and 2P-6** can advantageously facilitate achievement of a greater angle between the inner frame and outer frame at the attachment point, while also reducing valve space in the crimp length direction and avoiding total reliance on suture wraps for fixation. The joint structure as illustrated in **Figures 2P-3 and 2P-4 or Figures 2P-5 and 2P-6** can also advantageously provide for easier access and sewing during manufacture of the connection structure. **Figure 2P-7** illustrates a close-up view of another example of a dovetail joint structure. As shown, one or more dovetail tabs can be formed to provide secure engagement.

### Delivery Device

Referring briefly back to **Figure 1****,** the delivery device **15** can include a shaft assembly **12** comprising a proximal end and a distal end, with a handle **14** coupled to the proximal end of the shaft assembly **12.** The delivery device **15** can be used to hold the implant (e.g., prosthesis, replacement heart valve) for advancement of the same through the vasculature to a treatment location. In some embodiments, the shaft assembly **12** can hold at least a portion of an expandable implant (e.g., prosthesis, replacement heart valve) in a compressed state for advancement of the implant within the body. The shaft assembly **12** may then be used to allow controlled expansion of the implant at a desired implantation location (e.g., treatment location). In some embodiments, the shaft assembly **12** may be used to allow for sequential controlled expansion of the implant as discussed in detail below.

The shaft assembly **12** of the delivery device **15** can include one or more subassemblies, such as an outer sheath subassembly **20,** a rail subassembly **21,** a mid-shaft subassembly **22,** a release subassembly **23,** a manifold subassembly **24,** and/or a nose cone subassembly, as will be described in more detail below. In some embodiments, the shaft assembly **12** of the delivery device **15** may not have all of the subassemblies disclosed herein. The delivery device **15** may include multiple layers of concentric subassemblies, shafts, or lumens. The various lumen or shaft subassemblies will be described starting from an outermost layer. In some embodiments, the subassemblies disclosed below may be in a different radial order than is discussed.

### Outer Subassembly

[0144] **Figure 3A** shows a perspective view of an embodiment of the outer sheath subassembly **20** of the delivery device **15** of the delivery system **10.** The outer sheath subassembly **20** forms a radially outer covering, or sheath, to surround an implant retention area and prevent at least a portion of the implant (e.g., replacement heart valve or valve prosthesis) **30** from radially expanding until ready for implantation. Specifically, the outer sheath subassembly **20** can prevent a distal end portion of the implant **30** from radially expanding.

The outer sheath subassembly **20** can include an outer proximal shaft **302** having a proximal end portion operably coupled (e.g., via threaded outer sheath adapter **303**) to a capsule knob **905** (which may be a distal-most knob, as shown in **Figures 9A** and **9B**) of the handle **14** such that rotation of the capsule knob **905** causes proximal and distal translation of the outer sheath subassembly **20** (e.g., clockwise and counter-clockwise rotation). A capsule subassembly **306** can be attached to a distal end of the outer proximal shaft **302.** The components of the outer sheath subassembly **20** can form an outer-most lumen for the other subassemblies to pass through.

The outer proximal shaft **302** may be a tube formed of a plastic, but could also be formed of a metal hypotube or other material. The outer proximal shaft **302** may include an outer jacket or liner made of fluorinated ethylene propylene (FEP) material, polytetrafluoroethylene (PTFE) material, ePTFE material, or other polymeric material so as to make the outer surface of the outer proximal shaft **302** smooth and/or hemostatic. The outer proximal shaft **302** may include a connector (e.g., flexible reflow member) at its distal end to facilitate connection or coupling to the capsule subassembly **306.** At least a portion of the outer proximal shaft **302** may comprise a laser cut hypotube with a universally flexible pattern (e.g., an interrupted spiral pattern or an interrupted coil).

**Figure 3B** shows a side cross-section view of the capsule subassembly **306.** The capsule subassembly **306** may include a distal hypotube, or capsule stent, **308,** an inner liner inside of the hypotube **308,** a distal capsule tip **309,** and one or more outer liners or jackets **311** surrounding the hypotube **308.** The one or more outer liners or jackets **311** may comprise polyether block amide (e.g., PEBAX^{®} material) or other suitable polymer or thermoplastic elastomer material, such as polytetrafluoroethylene (PTFE) or expanded polytetrafluoroethylene (ePTFE). The inner liner may comprise PTFE, which may be pre-compressed before application to the inside of the hypotube **308.** The distal capsule tip **309** may comprise an atraumatic tip adapted to act as a funnel to facilitate recapture (e.g., crimping) of a valve prosthesis or other implant. The distal capsule tip **309** may be comprised of polyetheretherketone (PEEK) or other thermoplastic, polymeric, or metallic material. The distal capsule tip **309** may be loaded with radiopaque material (e.g., 5-40% barium sulfate loading) to facilitate detection (e.g., made fluorogenic) under radiographic imaging (e.g., fluoroscopy). The distal capsule tip **309** may fit within an open distal end of the hypotube **308.**

**Figure 3C** shows a perspective view of the distal hypotube, or capsule stent **308.** The capsule stent **308** can be formed from one or more materials, such as PTFE, ePTFE, polyether block amide (e.g., PEBAX), polyetherimide (e.g., Ultem^{®} material), PEEK, urethane, Nitinol, stainless steel, and/or any other biocompatible material. The capsule stent **308** is preferably flexible while still maintaining a sufficient degree of radial strength to maintain an implant (e.g., replacement valve) **30** within the capsule stent **308** without substantial radial deformation, which could increase friction between the capsule stent **308** and an implant contained therein. The capsule stent **308** also preferably has sufficient column strength to resist buckling, and sufficient tear resistance to reduce or eliminate the possibility of the implant tearing and/or damaging the capsule stent **308.** The proximal end and/or distal end of the distal hypotube, or capsule stent **308** may include multiple laser cut windows **313** adapted to make the proximal and/or distal end fluorogenic and/or echogenic to facilitate visualization under certain imaging modalities (e.g., noninvasive ultrasound imaging or invasive fluoroscopic imaging). In several implementations, a separate radiopaque element or member is not added to the hypotube **308** to facilitate imaging because of the presence of the laser cut windows **313.** The laser cut windows **313** may also promote adhesion of the outer jacket **311** to the capsule stent **308** and to the inner liner(s) by allowing glue or other adhesive to flow through the laser cut windows **313.** One or more layers of connection members made of PEBAX or other suitable material may surround the laser cut windows **313** to facilitate coupling of the hypotube, or capsule stent **308** to the distal capsule tip **309.**

The hypotube **308** may be formed of a plastic or metallic material. In some implementations, the hypotube **308** can be a metal hypotube. If metallic, the metallic material of the hypotube **308** may comprise cobalt chrome, stainless steel, titanium or metal alloy, such as nickel-titanium alloy material. The coil construction or cut patterns of the outer proximal shaft **302** and/or the hypotube **308** can allow the outer proximal shaft **302** to follow the rail subassembly **21** in any desired direction. A cut pattern of the outer proximal shaft **302** and/or the hypotube **308** may be modified (e.g., cut per revolution, pitch, spine distance) to control tension resistance, compression resistance, flexibility, and torque resistance. For example, cuts per revolution may range between 1.5 and 5.5, pitch may range between 0.005" and 0.15", and spine distance may range between 0.015" and 0.125". The hypotube **308** may advantageously provide both tension and compression. The one or more outer liners or jackets **311** may allow the capsule subassembly **306** to be more flexible. The capsule hypotube **308** can bend in multiple directions. In some implementations, a distal terminus of the outer liner or jacket **311** may be positioned proximal of the distal terminus of the hypotube **308.**

The capsule subassembly **306** may have a similar diameter as the outer proximal shaft **302** or a different diameter. In some embodiments, the capsule subassembly **306** has a uniform or substantially uniform diameter along its length. In some embodiments, the capsule subassembly **306** can be 28 French or less in size (e.g., 27 French). In some embodiments, the capsule subassembly **306** may include a larger diameter distal portion and a smaller diameter proximal portion. The capsule subassembly **306** can be configured to retain the implant (e.g., valve prosthesis) **30** in the compressed position within the capsule subassembly **306** (e.g., within an implant retention area **316** occupying a distal-most ~2 inches (or ~50 mm) of the capsule subassembly **306**.) Additional structural and operational details of a capsule subassembly, such as those described in connection with capsules in U.S. Publication No. 2019/0008640 and U.S. Publication No. 2019/0008639, which are hereby incorporated by reference herein, may be incorporated into the capsule subassembly **306.**

The outer sheath subassembly **20** is configured to be individually slidable (translatable) with respect to the other assemblies by rotation of the capsule knob **905.** Further, the outer sheath subassembly **20** can slide (translate) distally and proximally relative to the rail subassembly **21** together with the mid-shaft subassembly **22,** manifold subassembly **24,** release subassembly **23,** and/or nose cone subassembly.

**Figure 3D** schematically illustrates how at least a portion of a length of one or more components of the capsule subassembly **306** (e.g., inner liner **310**) can include excess material such that the capsule subassembly **306** includes built-in slack along a portion of its length (e.g., a portion of the length proximal to the implant retention area **316**) to facilitate flexible bending of the capsule subassembly **306** (e.g., to navigate tight turns within a heart or vasculature surrounding the heart).

**Figures 3E to 3G** illustrate an alternative embodiment of a distal capsule tip of capsule subassembly **306.** Comparing with the distal capsule tip **309** of **Figure 3B** (which has a straight shape end or a perpendicular flush cut distal end), distal capsule tip **309A** has an uneven end (e.g., lobed tip or waved shape), as shown in **Figure 3E****,** formed by alternately protruding and recessed lobes **309A-1 and 309A-2.** Such an uneven end of the distal capsule tip **309A** allows a staged deployment or recapture of anchors **37.** **Figure 3F** illustrates a side partial cross-section view of the distal capsule tip **309A** and schematically shows the staged or offset deployment or recapture of anchors **37** due to the lobed design of the distal capsule tip **309A.** **Figure 3G** is a flat plan view that illustrates that when recapturing anchors, the capsule subassembly **306** can recapture, for example, one or more (e.g., two, three or more) anchors **37** first, then the remaining anchors **37** (either individually or in pairs, trios, or other groupings) at subsequent stages (e.g., two or three stages). The staged recapture or deployment can advantageously distribute the recapture force to straighten the anchors **37** over time to reduce the overall force amplitude (e.g., by 20-40%, for example) at any one time during recapture. In this example, three lobes are shown, at 12 o'clock, 4 o'clock, and 8 o'clock. Such a configuration allows some anchors to begin unflexing before others during the recapture process in which the capsule is advanced over J-shaped anchors. This staggering or staging of the anchor recapture distributes the forces to unflex the anchors and advance the capsule, thus reducing peak loads or forces. Other numbers of lobes or shapes of lobes may be used.

### Rail Subassembly

**Figure 4A** shows a perspective view of a rail subassembly **21** of the delivery device **15** of the delivery system **10** of **Figure 1****.** **Figure 4A** shows approximately the same view as Figure 3A, but with the outer sheath subassembly **20** removed, thereby exposing the rail subassembly **21.** **Figure 4B** further shows a cross-section of the proximal and distal end portions of the rail subassembly **21** to view the pull wires that facilitate steering of the rail subassembly **21.** The rail subassembly **21** can include a rail shaft **402** (or rail) generally attached (and operably coupled) at its proximal end to the handle **14.** The rail shaft **402** can be made up of a rail proximal shaft **404** directly attached to the handle **14** at a proximal end and a rail hypotube **406** attached to the distal end of the rail proximal shaft **404** (e.g., via a connector, ring-like structure, or insert **407**)**.** The rail subassembly **21** is operably coupled to the handle **14** via primary flex adapter **403A** (which controls medial-lateral trajectory of the distal end portion of the rail subassembly **21** via one or more distal pull wires **410A**), via secondary flex adapter **403B** (which controls anterior-posterior trajectory of the distal end portion of the rail subassembly **21** via one or more proximal pull wires **410B**), and via rail adapter **405** (which includes a side needleless injection port to facilitate flushing and de-airing functions). The rail proximal shaft **404** may include an interrupted spiral cut pattern along a large portion of its length to facilitate compression. The rail hypotube **406** can further include an atraumatic rail tip **408** at its distal tip. The atraumatic rail tip **408** may not comprise slits and is configured to extend up to 1 inch beyond the distal terminus of the rail hypotube **406** and is configured not to dig into the outer shaft subassembly **20** to avoid friction and fatigue and to prolong use. These components of the rail subassembly **21** can form a rail lumen for the other inner subassemblies to pass through.

**Figure 4B** shows a side cross-section view of the rail subassembly **21** of **Figure 4A****.** As shown in **Figure 4B****,** attached to an inner surface of the rail hypotube **406** are one or more pull wires **410** which can be used apply forces to the rail hypotube **406** and steer the rail subassembly **21.** The pull wires **410** can extend distally from the primary and secondary flex knobs **915** (illustrated in **Figures 9A** and **9B**) in the handle **14** to the rail hypotube **406.** In some embodiments, pull wires **410** can be attached at different longitudinal locations on the rail hypotube **406,** thus providing for multiple bending locations in the rail hypotube **406,** allowing for multidimensional steering. For example, the rail hypotube **406** may provide a primary bend or flex along a medial/lateral trajectory and a secondary bend or flex along an anterior/posterior trajectory.

The rail hypotube **406** can include a number of circumferential slots (e.g., laser cut into the hypotube) to facilitate bending and flexibility. The rail hypotube **406** can generally be broken into a number of different sections. At the most proximal end is an uncut (or unslotted) hypotube section corresponding to the location of insert **407.** Moving distally, the next section is the proximal slotted hypotube section **406P.** This section includes a number of circumferential slots cut into the rail hypotube **406.** Generally, two slots are cut around each circumferential location forming almost half of the circumference. Accordingly, two backbones are formed between the slots extending up the length of the rail hypotube **406.** This is the section that can be guided by the proximal pull wire(s) **410B.** Moving further distally is the location where the proximal pull wires **410** connect, and thus slots can be avoided. This section is just distal of the proximally slotted section **406P** and may correspond to the location of insert, or pull wire connector, **411.**

Distally following the proximal pull wire connection area is the distal slotted hypotube section **406D.** This section is similar to the proximal slotted hypotube section **406P,** but may have significantly more slots cut out in an equivalent length. Thus, the distal slotted hypotube section **406D** may provide easier bending and an increased bend angle compared to the proximal slotted hypotube section **406P.** In some embodiments, the proximal slotted section **406P** can be configured to experience a bend of approximately 90 degrees with a bend radius of between 0.25" and 1" (e.g., between 0.25" and 0.75", between 0.4" and 0.6", between 0.5" and 1", overlapping ranges thereof, or any value within the recited ranges), whereas the distal slotted section **406D** can bend at approximately 180 degrees with a bend radius of between 0.25" and 1" (e.g., between 0.25" and 0.75", between 0.4" and 0.6", between 0.5" and 1", overlapping ranges thereof, or any value within the recited ranges). Further, as shown in Figures 4A and 4B, the spines of the distally slotted hypotube section **406D** are circumferentially offset from the spines of the proximally slotted hypotube section **406P.** Accordingly, the two sections will achieve different bend patterns, allowing for three-dimensional steering of the rail subassembly **21.** In some embodiments, the spines can be offset 30, 45, or 90 degrees, though the particular offset is not limiting. At the distal-most end of the distal slotted hypotube section **406D** is the distal pull wire connection area which is again a non-slotted section of the rail hypotube **406.**

In some embodiments, one distal pull wire **410A** can extend to a distal section (e.g., to rail tip **408**) of the rail hypotube **406** and two proximal pull wires **410B** can extend to a proximal section of the rail hypotube **406;** however, other numbers of pull wires can be used, and the particular amount of pull wires is not limiting. For example, two distal pull wires **410A** can extend to a distal location and a single proximal pull wire **410B** can extend to a proximal location. In some embodiments, ring-like structures or inserts attached inside the rail hypotube **406,** known as pull wire connectors, can be used as attachment locations for the proximal pull wires **410B,** such as insert **411.** In some embodiments, the pull wires **410** can directly connect to an inner surface of the rail hypotube **406.**

The distal pull wire(s) **410A** can be connected (either on its own or through rail tip connector **408**) generally at the distal end of the rail hypotube **406.** The proximal pull wire(s) **410B** can connect (either on their own or through the insert **411**) at a location approximately one quarter, one third, or one half of the length up the rail hypotube **406** from the proximal end. In some embodiments, the distal pull wire(s) **410A** can pass through a small diameter pull wire lumen (e.g., tube, hypotube, cylinder) attached on the inside of the rail hypotube **406.** This can prevent the pull wires **410** from pulling on the rail hypotube **406** at a location proximal to the distal connection. Further, the lumen can comprise compression coils to strengthen the proximal portion of the rail hypotube **406** and prevent unwanted bending. Thus, in some embodiments the lumen is only located on a proximal portion (e.g., proximal half) of the rail hypotube **406.** In some embodiments, multiple lumens, such as spaced longitudinally apart or adjacent, can be used per distal pull wire **410A.** In some embodiments, a single lumen is used per distal wire **410A.** In some embodiments, the lumen can extend into the distal portion (e.g., distal half) of the rail hypotube **406.** In some embodiments, the lumen is attached on an outer surface of the rail hypotube **406.** In some embodiments, the lumen is not used. In some embodiments, one or more compression coils **413** extend from the insert **407** to the insert **411.** The compression coils **413** may be configured to bypass load in length between a distal primary flex point and a proximal secondary flex point. The compression coils **413** facilitate independent flex planes so that both planes of flex do not activate when one plane of flex is desired to flex. The compression coils **413** may allow for the proximally slotted hypotube section **406P** to retain rigidity for specific bending of the distally slotted hypotube section **406D.** The compression coils **413** may isolate force so only the primary flex is flexed.

For the pair of proximal pull wires **410B,** the wires can be spaced approximately 180° from one another to allow for steering in both directions. Similarly, if a pair of distal pull wires **410A** is used, the wires can be spaced approximately 180° from one another to allow for steering in both directions. In some embodiments, the pair of distal pull wires **410A** and the pair of proximal pull wires **410B** can be spaced approximately 90° from each other. Opposing wires could be used to provide anti-flex mechanism. In some embodiments, the pair of distal pull wires **410A** and the pair of proximal pull wires **410B** can be spaced approximately 0° from each other. However, other locations for the pull wires can be used as well, and the particular location of the pull wires is not limiting. In some embodiments, the distal pull wire **410A** can pass through a lumen attached within the lumen of the rail hypotube **406.** This can prevent an axial force on the distal pull wire **410A** from creating a bend in a proximal section of the rail hypotube **406.** The rail subassembly **21** is disposed so as to be slidable (e.g., translatable) over the radially inner subassemblies. As the rail hypotube **406** is bent, it presses against the other subassemblies to bend them as well, and thus the other subassemblies of the delivery device **15** can be configured to steer along with the rail subassembly **21** as a cooperating single unit, thus providing for full steerability of the distal end of the delivery device **15.** Additional structural and operation details of a rail subassembly, such as those described in connection with rail assemblies in U.S. Publication No. 2019/0008640 and U.S. Publication No. 2019/0008639, which are hereby incorporated by reference herein, may be incorporated into the rail subassembly **21.**

**Figure 4C** schematically illustrates how an outer compression coil **413A** and proximal pull wire **410B1** can have a longer length than an inner compression coil **413B** and proximal pull wire **410B2** of the rail subassembly **21** so that they don't occupy the same space, to reduce lumen obstruction during bending, and/or to facilitate ease of bending in one direction.

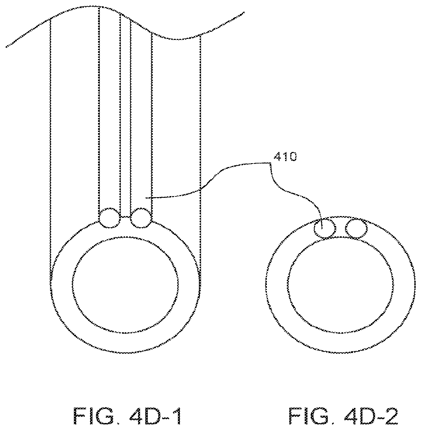

**Figure 4D-2** schematically illustrates a method of manufacturing that comprises thru-wall welding performed during manufacture of the rail subassembly (as compared to prior direct wire welding techniques). Figure 4D-1 illustrates a prior art welding technique and Figure 4D-2 illustrates an embodiment of a thru-wall welding technique. The thru-wall welding technique may advantageously be used to weld the pull wires **410** to the inserts (e.g., insert **407, 411** tip **408**) within a lumen of the rail hypotube **406.** In accordance with several embodiments, thru-wall welding advantageously does not comprise welding directly to the pull wires **410.** Welding directly to the wires **410** (as is shown in Figure 4D-1) can cause annealing and embrittling of a majority or of an entirety of a circumference of a pull wire (which has hard temper for strength) if heated too much. With reference to Figure 4D-2, thru-wall welding can involve intentionally forming through-holes in between an outer diameter and inner diameter of a wall of a lumen and controlling a wall thickness to facilitate thru-wall welding in a manner that penetrates the hypotube or lumen wall but limits circumferential extent of heating of the pull wire (e.g., less than 20% of circumference, less than 25% of circumference, less than 30% of circumference). In some embodiments, thru-wire welding allows for welding along a single line (e.g., a line extending between the pull wires) instead of along multiple lines (e.g., one line for each pull wire).

### Mid-shaft Subassembly

Moving radially inwardly, the next subassembly is the mid-shaft subassembly **22.** **Figure 5A** shows a perspective view of the mid-shaft subassembly **22** of the delivery device **15** of the delivery system **10.** **Figure 5B** shows a side view of the mid-shaft subassembly **22.** The mid-shaft subassembly **22** can include a distal mid-shaft hypotube **502** generally attached at its proximal end (e.g., via laser welding or a heat shrink connector) to a mid-shaft proximal tube **504,** which in turn can be attached at its proximal end to the handle **14** (e.g., via mid-shaft adapter **505),** and a distal outer retention member or pusher **506** located at the distal end of the mid-shaft hypotube **502.** These components of the mid-shaft subassembly **22** can form a lumen (e.g., middle lumen) for other inner subassemblies to pass through.

The mid-shaft subassembly **22** can be located within a lumen (e.g., rail lumen) of the rail subassembly **21.** The mid-shaft hypotube **502** can be formed of a metallic alloy (e.g., cobalt chrome, nickel-chromium-cobalt alloy, nickel-cobalt base alloy, nickel-titanium alloy, stainless steel and titanium). The mid-shaft hypotube **502** may comprise an interrupted spiral cut pattern. In alternative embodiments, the mid-shaft hypotube **502** comprises a longitudinally pre-compressed high density polyethylene (HDPE) tube. Figure 5A shows a similar view as Figure 4A, but with the rail subassembly **21** removed, thereby exposing the mid-shaft subassembly **22.**

Similar to the other subassemblies, the mid-shaft hypotube **502** and/or mid-shaft proximal tube **504** can comprise a tube or lumen, such as a hypodermic tube or hypotube (not shown). The tubes can be made from one of any number of different materials including Nitinol, stainless steel, and medical grade plastics. The tubes can be a single piece tube or multiple pieces connected together. Using a tube made of multiple pieces can allow the tube to provide different characteristics along different sections of the tube, such as rigidity and flexibility. The mid-shaft hypotube **502** can be a metal hypotube. The mid-shaft hypotube **502** can have a number of slots/apertures cut into the hypotube. In some embodiments, the cut pattern can be the same throughout. In some embodiments, the mid-shaft hypotube **502** can have different sections having different cut patterns. The mid-shaft hypotube **502** can be covered or encapsulated with a layer of ePTFE, PTFE, or other material so that the outer surface of the mid-shaft hypotube **502** is generally smooth. At least a portion of a length of the mid-shaft proximal tube **504** may be covered with a heat shrink tubing or wrap.

The pusher **506** may be configured for radially retaining a portion of the implant (e.g., prosthesis) **30** in a compacted configuration, such as a proximal end of the implant **30.** For example, the pusher **506** may be a ring or covering that is configured to radially cover a proximal end portion (e.g., suture eyelets portion or proximal-most inflow portion) of the implant **30.**

**Figures 5B****-1 to 5B-3** illustrate an embodiment of a distal pusher **506** of a mid-shaft subassembly **22,** in which **Figure 5B****-3** is a cross sectional view of the line 5B-3-5B-3 of **Figure 5B****-2.** **Figures 5B****-4 to 5B-6** illustrate another embodiment of a distal pusher **506A** of the mid-shaft subassembly **22,** in which **Figure 5B****-6** is a cross sectional view of the line 5B-6-5B-6 of **Figure 5B****-5.** A distal pusher **506** of **Figures 5B-****1 to 5B-3** and a distal pusher **506A** of **Figures 5B****-4 to 5B-6** have substantially the same outer and inner diameters **Φ1** and **Φ2** forming a cylindrical shape when viewed from the top. However, the distal pusher **506A** does not have a lip and cup portion **507** having a height **H7,** thus having a flat top surface **509,** compared to the distal pusher **506** having a thin wall having a radius of curvature **R1** at the upper surface. Therefore, a total height **H6** of the distal pusher **506** of **Figures 5B****-1 to 5B-3** is reduced by about a height **H7.** Further, removal of the material comprising the lip and cup portion **507** may leave only a flat surface to oppose an inflow side of the valve prosthesis during capsule retraction for valve deployment. The distal pusher **506A** may have increased room (e.g., increased cross-sectional area) to fit the inflow struts of the outer frame **34** against the inside of the pusher **506A.** The distal pusher **506A** may also provide reduced docking forces (e.g., about 50% reduction in docking force compared to the distal pusher **506)** as the suture portions attached to the proximal-most or inflow struts tension the outer frame **34** against the flat pusher surface without any lip or bump to pull the eyelets **35** over.

**Figure 5C** shows a side cross-section view showing a close-up view of a distal end portion of the mid-shaft subassembly **22,** which shows the proximal end portion (e.g., proximal-most portion, or just the suture eyelets **35)** of the implant **30** being retained within the pusher **506.** The pusher **506** can also be considered to be part of the implant retention area **316** and may be at the proximal end of the implant retention area **316.** The pusher **506** may comprise a frustoconical or cup shape that is riveted or fastened on its opposite sides to the distal end of the mid-shaft hypotube **502.** The pusher **506** may be formed of PEEK material, ferrous material, platinum iridium, or other fluorogenic material to facilitate radiographic imaging. The pusher **506** may also be formed of other thermoplastic, polymeric, or metallic materials. The pusher **506** may be loaded with radiopaque material (e.g., 5-40% barium sulfate loading) to facilitate detection (e.g., made fluorogenic) under radiographic imaging (e.g., fluoroscopy). The mid-shaft subassembly **22** may be disposed so as to be individually slidable (e.g., translatable) with respect to the other subassemblies. The mid-shaft adapter **505** operably couples to the depth knob **920** to effect ventricular/atrial movement within a heart (e.g., for implementations in which the implant **30** is a mitral or tricuspid replacement heart valve). Additional structural and operational details of a mid-shaft subassembly **22.** such as those described in connection with mid assemblies in U.S. Publication No. 2019/0008640 and U.S. Publication No. 2019/0008639, which are hereby incorporated by reference herein, may be incorporated into the mid-shaft subassembly **22.**

### Release and Manifold Subassemblies

In accordance with several configurations, a delivery device includes a suture-based release mechanism that includes a plurality of suture portions that are only coupled to a distal end portion of the delivery device and no not extend along the delivery device to a proximal handle that controls operation of the suture-based release mechanism. A first end of each of the plurality of suture portions may be fixedly attached to the distal end portion of the delivery device and a second end of each of the plurality of suture portions are releasably attached to the distal end portion of the delivery device after being inserted through a retention member (e.g., opening or eyelet) of an implant (e.g., replacement heart valve). The suture portions may be released (e.g., decoupled) from the implant by operator actuation of an actuator on a handle of the delivery device.

The suture-based release mechanism may include dual coaxial sliding shafts, or lumens. It should be appreciated that reference to lumens in the disclosure may be referring to shafts or tubes comprising lumens. The dual coaxial sliding shafts may be operably coupled to the actuator on the handle of the delivery device. The first end of each of the plurality of suture portions may be fixedly attached to a distal tip of an inner lumen of the dual coaxial sliding shafts. The second end of each of the plurality of suture portions may be releasably coupled to one or more retention members of the distal end portion of the inner shaft. Translation of the outer shaft with respect to the inner shaft of the dual coaxial sliding shafts or lumens by actuation of the actuator on the handle may cause the suture portions to be disengaged or decoupled from the one or more retention members of the distal end portion of the inner shaft.

Moving radially inward from the mid-shaft subassembly **22,** **Figure 6A** shows a perspective view of a release subassembly **23** of the delivery device **15** of the delivery system **10.** **Figure 6B** shows a side cross-section view of the release subassembly **23** of Figure 6A. The release subassembly **23** operates in conjunction with the manifold subassembly **24** to facilitate retention and release of the implant or prosthesis **30.** The release subassembly **23** extends through a central lumen of the mid-shaft subassembly **22.** The release subassembly **23** includes a release shaft **602** that includes a lumen. The manifold subassembly **24** extends through the lumen of the release subassembly **23.** The mid-shaft subassembly **22** acts as a compression member backstop and the manifold subassembly **24** acts as the tension member such that the mid-shaft subassembly **22** prevents retreating of the implant **30** when the capsule subassembly **306** is pulled back and the manifold subassembly prevents deployment/expansion of the implant **30** (or distal movement of the implant **30).**

The distal portion of the release shaft **602** may include laser cut portions having various spine patterns. For example, a distal-most portion (e.g., ~1 cm) of the release shaft **602** may include a dual spine laser cut pattern and a portion proximal of the distal-most portion (e.g., ~5 cm proximal of the distal-most portion) may include a universal laser cut spine pattern. The dual spine pattern portion may only travel through the primary distal flex portion of the rail hypotube **406** and the universal spine pattern portion may travel through both the primary and secondary flex portions of the rail hypotube **406.** At least a portion of a length of the release shaft **602** may be surrounded by a heat-shrink wrap or liner. The proximal end of the release shaft **602** is operably coupled to the handle **14** (e.g., via release adapter **604).** The release subassembly **23** also includes a distal release tip **605** coupled to a distal end of the release shaft **602** via coupler **607,** which may be formed of PEBAX or other thermoplastic elastomer material. The distal release tip **605** may be welded to the distal end of the release shaft **602.** The release adapter **604** includes release snaps **606** on opposite lateral sides. The release snaps **606** engage with a distal potion of the manifold adapter **704** after release of the tethers or sutures so as to prevent movement of the manifold subassembly **24** and release subassembly **23** with respect to each other, which could cause the windows **610** of the distal release tip **605** to close and inadvertently retain one of the sutures or tethers. Thus, the release snaps **606** convert the release/manifold mechanism from a normally-closed configuration to an open configuration and allows the manifold subassembly **24** and release subassembly **23** to track proximally together. The release subassembly **23** further includes a release spring **608** that extends between the release adapter **604** and a location within a manifold adapter **704** of the manifold subassembly 24.

**Figures 6C, 6D, and 6E** show a close-up, side view, side cross-section view, and bottom view, respectively, of the distal release tip **605.** The distal release tip **605** cooperates in conjunction with a distal end portion of the manifold subassembly **24** to facilitate prevention of premature release of the implant **30** and to facilitate release (e.g., untethering) of the implant **30** when ready for final implantation. The distal release tip **605** includes three windows **610** spaced apart around a circumference of the distal release tip **605** and three slots **612,** with each slot **612** positioned between two adjacent windows **610.** The windows **610** may be laser cut into the distal release tip **605.** The three windows **610** may be equally spaced apart circumferentially and the slots **612** may be positioned equally circumferentially between adjacent windows **610.** A distal end of each of the slots **612** includes an inwardly-protruding retention member **614** (e.g., tab, protrusion, lock, anchor). The inwardly-protruding tabs **614** are adapted to be aligned with and extend within corresponding slots of the manifold subassembly **24** so as to control axial movement (e.g., to provide positive datums for distal and proximal travel) and to prevent rotation of the release subassembly **23** with respect to the manifold subassembly **24,** as will be described in more detail below.

Moving radially inward, **Figure 7A** shows a perspective view of the manifold subassembly **24** of the delivery device **15.** **Figure 7B** shows a side cross-section view of the manifold subassembly **24** of **Figure 7A****.** The manifold subassembly **24** extends through and along the lumen of the release subassembly **23.** The manifold subassembly **24** includes a proximal subassembly **701** and a distal subassembly **703.** The proximal subassembly **701** includes a proximal shaft **702** having a proximal end that extends into the handle **14** of the delivery device **15** and is operably coupled to the handle **14** via a manifold adapter **704.** The proximal shaft **702** may be coupled to the distal subassembly **703** by a manifold cable **705.** The manifold cable **705** may comprise a multi-layer cable comprised of two, three, four, five or more layers. In some implementations, the manifold cable **705** comprises a tri-layer cable in which two outer layers function for tension and act together to prevent unwrapping of the outer layers and an inner layer comprises a single-filar coil that provides compression and prevents collapse. In some implementations, each layer is wound in an opposite direction as the adjacent layer (e.g., clockwise, counter-clockwise, clockwise or counter-clockwise, clockwise, counter-clockwise). The wire size, wire tension, pitch, number of filars in each layer, material, and material properties may vary. An inner coil may comprise one to ten filars closely wound with a 0 to 0.005" gap. The middle and outer coils may each comprise one to ten filars and be closely wound with a 0 to 0.010" gap. The manifold cable **705** may be formed of one or more materials, including, for example, nitinol, ferrous material such as stainless steel, and/or cobalt chrome material. The temper (e.g., strength) of the wires may range from 100 KSI to 420 KSI (kip/square inch) and an ultimate tensile strength of the manifold cable **705** may be greater than 110 pounds of force. The cross-section of the wires may be flat or round. The tri-layer cable may be configured to prevent diameter change during stretching. In other implementations, the proximal shaft **702** extends all the way to and is bonded with a proximal end of the distal subassembly **703.**

**Figure 7C** shows a close-up view of the distal subassembly **703** of the manifold subassembly **24.** **Figure 7D** shows a bottom view of the distal subassembly **703** of the manifold subassembly **24.** As shown, the distal subassembly **703** includes a proximal tether retention component **706** and a distal tether retention component **707.** The distal tether retention component **707** may be coupled (e.g., permanently bonded, welded) to a distal end of the proximal tether retention component **706.** As shown best in **Figure 7D****,** the distal tether retention component **707** may comprise a cog that includes outwardly-extending tether cleats **708** circumferentially spaced around the cog. Openings or gaps **709** exist between adjacent tether cleats **708** to receive portions of the tether or suture **710.** The distal tether retention component **707** may be formed of metal through an electrical discharge machining process. The proximal tether retention component **706** may also be formed of metal and formed via a laser cutting or electrical discharge machining process. The distal tether retention component **707** may include proximal and distal seal members **711, 713** (e.g., retention rings) that are sealed (e.g., welded, glued or otherwise adhered) to opposite upper and lower sides of the distal tether retention component **707** during manufacture to seal off the openings or gaps **709** between the tether cleats **708** so as to prevent the tether or suture **710** from being removed or uncoupled from the distal tether retention component **707.** In accordance with several embodiments, the tether **710** is intended to be permanently coupled to (i.e., non-removable from) the distal tether retention component **707.** The number of tether cleats **708** may correspond to the number of eyelets on the implant **30** (e.g., upper eyelets of the outer frame **34).** The number of tether cleats **708** is nine in the illustrated embodiment; however, other numbers of tether cleats **708** may be used.

The tether or suture **710** may be a continuous piece of tether or suture that forms offset proximal loops and distal loops along its continuous length upon assembly during manufacturing. The proximal loops are wrapped around the tether cleats **708** and the distal loops are fed through a respective eyelet on a proximal end of the implant or prosthesis **30** (e.g., upper eyelet of an outer frame **34)** and then removably coupled to the delivery device **15** (e.g., the proximal tether retention component **706** of the manifold subassembly **24).**

During assembly, the continuous tether or suture **710** may be coupled to the distal tether retention component **707** according to the following example implementation. One end of the continuous tether or suture **710** may start at a location spaced distal to the distal tether retention component **707.** With the one end remaining there, the tether **710** is then wrapped around a first tether cleat **708** and then fed back through an opening or gap **709** on the other side of the first tether cleat **708** to form a first proximal loop and then brought back to a location spaced distal to the distal tether retention component **707** to start formation of a first distal loop. The process is repeated for each of the tether cleats **708** until all of the proximal and distal loops are formed and the second end of the continuous tether **710** is brought near the first end of the continuous tether **710** and the two ends are knotted together and bonded to form a single continuous strand. The tether assembly process may be facilitated by an assembly component that can be placed at an appropriate spacing distance distal of the distal tether retention component **707** and that includes pegs around which portions of the continuous tether **710** can be wrapped to form the distal loops at uniformly-spaced distances from the distal tether retention component **707.** The proximal loops may be prevented from unhooking from the tether cleats **708** by the proximal and distal seal members **711, 713.** The continuous tether **710** may comprise ultra-high-molecular-weight polyethylene (UMHWPE) force fiber suture, an aramid suture, or an aramid and UMHWPE blend suture material. In some embodiments, aramid material may advantageously bond and prevent floss and/or fretting failure due to asymmetric loading of the suture during detachment. In accordance with several embodiments, the continuous tether **710** advantageously does not run an entire length of the delivery device or system (e.g., all the way to the handle) because elongation at load would be significant and any mechanism added to compensate could add increased complexity and could potentially be unreliable and/or not user-friendly.

**Figure 7E** shows a flat cut pattern of the proximal tether retention component **706** of the distal subassembly **703.** As shown, the proximal portion of the proximal tether retention component **706** comprises a dual spine laser cut pattern. The dual spine laser cut pattern of the proximal tether retention component **706** may match a dual spine laser cut pattern of the rail subassembly **21** and the release subassembly **23.** The distal end portion of the proximal tether retention component **706** comprises three circumferentially spaced slots **714** and three openings or windows **715.** The slots **714** are configured to align circumferentially with the slots **612** of the distal release tip **605** and the openings or windows **715** are configured to align circumferentially with the windows **610** of the distal release tip **605.** Other numbers of slots **714** and openings **715** (e.g., two, four, five, six, seven, eight, nine) may also be used in other embodiments. Each opening **715** includes a tab, finger, or peg, **716** extending a certain distance into a respective opening **715** from a distal edge of the respective opening **715.** A length of each tab **716** is sufficient such that one or more distal tether loops can be looped over a top (or proximal end) of a respective tab **716** and pushed distally so as to retain the one or more distal tether loops. As shown, the three tabs **716** each have a different length in order to facilitate the initial tether assembly process. However, in other configurations, the three tabs **716** may have an equal or substantially equal length. Each tab **716** may receive one or more distal tether loops. In one implementation where there are nine distal tether loops, each tab **716** may retain three distal tether loops. The slots **714** may be equally circumferentially spaced around a longitudinal axis of the proximal tether retention component **706** and may be sized and spaced so as to align with corresponding slots **612** of the release subassembly **23** so as to receive a respective inwardly-protruding retention member **614.**

### Operation of the Suture-Release Mechanism

**Figures 8A** **and** **8B** show distal end portions of the release and manifold subassemblies in a locked configuration and unlocked configuration, respectively. The locked configuration shown in **Figure 8A** is the default configuration after assembly. The release and manifold subassemblies are intended to remain in the locked configuration until a clinician has determined that the implant **30** is in a final desired implantation location. In the locked configuration, the proximal ends of the tabs **716** are positioned proximal of the proximal edge of the release windows **610** such that the distal tether loop(s) wrapped around the tabs **716** cannot be unhooked from the tabs **716,** which could cause premature release of the tether **710.** For simplicity and to avoid confusion in the figure, only one distal tether loop is shown wrapped around one of the tabs **716;** however, two, three, or more tether loops may be hooked onto, or wrapped around, each of the tabs **716.** The spring **608** shown in **Figure 6A** (which is biased in a compressed configuration) keeps the release adapter **604** and the manifold adapter **704** apart and forces the release subassembly **23** distal in compression so that the release subassembly 23 and the manifold subassembly 24 do not move longitudinally with respect to each other, thereby keeping the release subassembly **23** and the manifold subassembly **24** in the locked configuration shown in **Figure 8A** until an operator is ready to release the suture(s) or tether(s). As discussed in connection with **Figures 9A** and **9B****,** a safety member (e.g., pin) **927** of the handle also prevents the manifold subassembly **24** from moving distally out of the locked configuration until ready.

Once the clinician has determined that the implant **30** is in a final desired implantation position and all verification processes have been performed and confirmed, the safety member **927** is removed from the handle and the spring **608** is placed even more in compression. As the release knob **925** is rotated distally, the spring **608** is compressed further and pushes the manifold subassembly **24** distally out of the release subassembly **23** into the unlocked configuration shown in **Figure 8B****.** As shown in **Figure 8B****,** the manifold subassembly **24** has been pushed distally enough with respect to the release subassembly **23** that the proximal end of at least one of the tabs **716** is within the release window **610** such that a distal tether loop of the tether **710** can be unhooked from the tab **716,** especially upon continued distal advancement of the manifold subassembly **24.** **Figure 8C** illustrates how one of the tether or suture loops transitions from being tethered to being untethered, or released, as the release and manifold subassemblies effect transition between a locked configuration and an unlocked configuration. Also as shown in **Figure 8C****,** the corresponding slots **612** and **714** are aligned so as to prevent rotation of the manifold subassembly **24** with respect to the release subassembly **23** (due to inwardly-protruding retention members **614),** thereby retaining alignment of the tabs **716** within the windows **610** of the release subassembly **23.** **Figure 8D** shows an implant **30** fully tethered between eyelets on a proximal end of the implant (e.g., upper cyclet of an outer frame **34** of a valve prosthesis **30)** and the manifold subassembly **24** of the delivery device **15.** As shown, there are nine tether loops or portions connected to nine eyelets; however, the number may vary as desired and/or required. The suture or tether retention mechanism described in connection with Figures 8A-8D advantageously does not require the tethers or sutures **710** to extend through and along a long portion of the length of the delivery device **15** (e.g., to a proximal handle **14),** thereby advantageously preventing or reducing the likelihood of snagging or catching of the suture or tether portions on intervening components within the delivery device, preventing or reducing the likelihood of tangling of suture or tether portions due to decreased lengths, reducing complexity of operation required by an operator to release a tether, simplifying assembly and manufacture, and/or reducing an amount of suture or tether material required. Instead, the suture or tether portions are advantageously only coupled to the distal end portion of the delivery device.

### Handle

**Figure 9A** shows a perspective view of the handle **14** of the delivery device **15.** **Figure 9B** shows a side cross-section view of the handle **14.** The handle **14** includes multiple actuators, such as rotatable knobs, that can manipulate different components (e.g., cause movement of respective subassemblies of the shaft assembly **12)** of the delivery system **10.** The distal end of the handle **14** includes a capsule knob **905.** Rotation of the capsule knob **905** in one direction causes proximal movement of the outer sheath subassembly **20** in an axial direction so as to unsheathe and deploy a distal portion (e.g., ventricular portion) of the implant **30** from the capsule subassembly **306.** Rotation of the capsule knob **905** in the opposite direction causes distal movement of the outer sheath subassembly **20** (including the capsule subassembly **306)** so as to recapture, retrieve, or resheath, the implant **30** within the capsule subassembly **306.** The outer sheath subassembly **20** may be individually translated with respect to the other subassemblies in the delivery device **15.** With reference back to **Figure 5C****,** the distal end of the implant **30** can be released first, while the proximal end (e.g., proximal-most eyelets **35** but not a proximal circumferential shoulder of an outer frame) of the implant **30** can remain radially compressed within the pusher **506** of the mid-shaft subassembly **22.** Because the capsule assembly **306** is so robust and provides both tension and compression strength, only the proximal-most portion of the implant **30** (e.g., the eyelets **35)** need to be retained by the pusher **506** and the pusher **506** can be relatively short in length. The tethers **710** and release subassembly **23** and manifold subassembly **24** also remain within the mid-shaft subassembly **22** until rotation of a release knob **925.**

Moving proximally, the handle **14** includes a stabilizer mounting area **910** adapted to interface with a clamp of a stabilizer assembly **1100** configured to control the medial /lateral position of the delivery device **15.** Moving further proximally are the primary flex rail knob **915A** and the secondary flex rail knob **915B.** Rotation of the primary flex rail knob **915A** causes flexing of the primary flex portion, or distal slotted hypotube section **406D** of the rail hypotube **406** to effect changes in medial/lateral trajectory. Rotation of the secondary flex rail knob **915B** causes flexing of the primary flex portion, or proximal slotted hypotube section **406P** of the rail hypotube **406** to effect changes in anterior/posterior trajectory. However, the number of flex rail knobs **915** can vary depending on the number of pull wires used.

Proximal to the secondary flex rail knob **915B** is a depth knob **920** that, in some embodiments, controls simultaneous movement of the outer assembly **20.** mid-shaft subassembly **22,** release subassembly **23,** manifold subassembly **24,** and nose cone subassembly either distal or proximal (thereby moving the delivery device **15** ventricular or atrial for a mitral valve or tricuspid valve implantation). The depth knob **920** may move the subassemblies together relative to the rail subassembly **21.** Further proximal is the release knob **925** (sometimes also referred to as the manifold knob since it controls simultaneous longitudinal movement of both the release subassembly **23** and the manifold subassembly **24** until the release subassembly **23** encounters a hard stop member within the handle **14** and then only the manifold subassembly **24** continues to move longitudinally in a distal direction with respect to the release subassembly **23).** The release knob **925** may be rotated proximally to put tension on the manifold subassembly **24** during loading or during recapture, or retrieval, of the implant **30.** The release knob **925** may be rotated distally to deploy the proximal portion (e.g., atrial portion) of the implant **30** after the capsule subassembly **306** has been retracted to deploy the distal portion (e.g., ventricular portion) of the implant **30.** Distal movement of the release knob **925** takes tension off the manifold subassembly **24.** As discussed above, the safety stop member **927** prevents the release knob **925** from moving distally enough to allow release of the implant **30** until the safety stop member **927** is removed from the handle **14.** Once the safety stop member **927** has been removed, continued distal movement of the release knob **925** causes the manifold subassembly **24** to move distally relative to the release subassembly **23** (after the release subassembly **23** abuts against a mechanical stop member within the handle **14** that prevents further distal movement of the release subassembly **23)** to facilitate release of the tether **710** from the manifold subassembly **24** (e.g., the distal tether loops are allowed to be pushed off of the tabs **716** of the proximal tether retention member **706** of the manifold subassembly **24** by the windows **610** of the release subassembly **23).** The proximal-most knob is the nose cone knob **930,** rotation of which causes proximal and distal movement of the nose cone subassembly.

### Nose Cone Subassembly

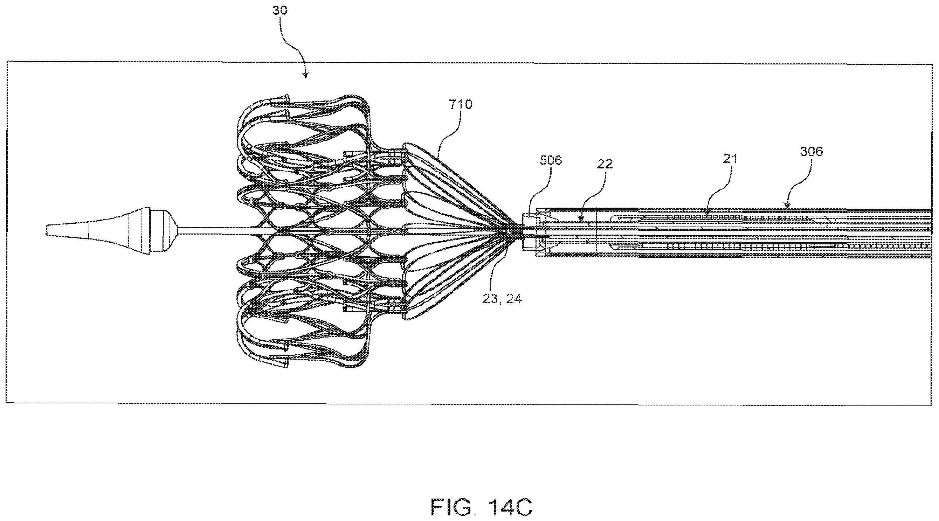

The nose cone subassembly is the most radially-inward subassembly and may include a nose cone shaft having a distal end connected to a nose cone **87** (labeled in Figure 14C). For example, the knob **930** can be a portion of the nose cone subassembly that extends from a proximal end of the handle **14.** Thus, a user can rotate the knob **930** to translate the nose cone shaft distally or proximally individually with respect to the other shafts. This can be advantageous for proximally translating the nose cone **87** into the outer sheath assembly **20** /capsule subassembly **306,** thus facilitating withdraw of the delivery device **15** from the patient. The nose cone **87** can have a tapered tip. The nose cone **87** can be made of a thermoplastic or elastomer (e.g., PEBAX or polyurethane) for atraumatic entry and to minimize injury to venous vasculature. The nose cone **87** can also be radiopaque to provide for visibility under fluoroscopy. The nose cone assembly is preferably located within a lumen of the manifold subassembly **24.** The nose cone assembly can include a lumen for a guide wire to pass therethrough. Additional structural and operation details of a handle and a nose cone assembly, such as those described in connection with handles and nose cone assemblies in U.S. Publication No. 2019/0008640 and U.S. Publication No. 2019/0008639, which are hereby incorporated by reference herein, may be incorporated into the handle **14** and nose cone subassembly herein.

### Introducer Assembly

**Figure 10** shows components of an introducer assembly **1000** of the delivery system **10.** The introducer assembly **1000** includes an introducer sheath **1005,** a dilator **1010,** an introducer **1012,** and a loader **1015.** The dilator **1010** helps to dilate the vasculature for insertion of the delivery device **15** and/or introducer sheath **1005.** The dilator **1010** may be removed and replaced with additional dilators (e.g., dilators of differing diameters) if desired and/or required. After removal of the dilator **1010,** the introducer **1012** (which may be inserted into and advanced along the introducer sheath **1005** so that a tapered distal tip of the introducer **1012** extends beyond an open distal end of the introducer sheath **1005)** and the introducer sheath **1005** are advanced together into the dilated vasculature through an incision. For a transfemoral delivery approach, the vasculature is a femoral vein within a leg of the subject. The introducer sheath **1005** may include a side portion to facilitate heparinized saline or other flushing fluid. The introducer sheath **1005** may be configured to remain stationary with respect to the leg of the subject. The loader **1015** is adapted to be inserted into the proximal end of the introducer sheath **1005** in order to open up aggressive one-way valves in the introducer sheath **1005** prior to insertion of the delivery device **15** to make insertion of the delivery device **15** through the introducer sheath **1005** easier. The loader **1015** may also advantageously reduce friction between the delivery device **15** and the introducer sheath **1005** while the delivery device **15** is inserted and while the delivery device **15** is manipulated during an implantation procedure. In some implementations, the introducer **1012** and introducer sheath **1005** may not be used and the delivery device **15** may be inserted directly into the dilated vasculature.

### Stabilizer Assembly

**Figure 11** illustrates how the handle **14** of the delivery device **15** interfaces with an embodiment of the stabilizer assembly **1100** of the delivery system **10.** **Figure 11A** shows a perspective view of the stabilizer assembly **1100** without the delivery device **15** attached. **Figure 11B** shows a top view of the stabilizer assembly **1100** of Figure 11A. The stabilizer assembly **1100** includes a clamp **1105,** a guide assembly **1110,** a rail **1115,** and a base **1120.** The clamp **1105** is configured to couple to the stabilizer mounting area **910** of the handle **14** of the delivery device **15.** The guide assembly **1110** is configured to cause changes in the medial/lateral position of the delivery device **15** by movement along the rail **1115.** The rail **1115** may be mounted on and secured to the base **1120.** Additional details regarding the stabilizer assembly **1100** may be found in US Pat. Publ. No. 2020/0108225 published on January 10, 2020, the entire contents of which are incorporated by reference herein.

### Delivery Methods

**Figure 12** illustrates a schematic representation of a transseptal delivery approach. As shown in Figure 12, in one embodiment the delivery system 10 can be placed in the ipsilateral femoral vein **1074** and advanced toward the right atrium **1076.** A transseptal puncture using known techniques can then be performed to obtain access to the left atrium **1078.** The delivery system **10** can then be advanced in to the left atrium **1078** and then to the left ventricle **1080.** Figure 12 shows the delivery system **10** extending from the ipsilateral femoral vein **1074** to the left atrium **1078.** In embodiments of the disclosure, a guide wire is not necessary to position the delivery system **10** in the proper position, although in other embodiments, one or more guide wires may be used.

Accordingly, it can be advantageous for a user to be able to steer the delivery system **10** through the complex areas of the heart in order to position a replacement mitral valve in line with the native mitral valve. This task can be performed with or without the use of a guide wire with the above disclosed system. The distal end of the delivery system 10 can be advanced into the left atrium **1078.** A user can then manipulate the rail subassembly **21** to target the distal end of the delivery system **10** to the appropriate area. A user can then continue to pass the bent delivery system **10** through the transseptal puncture and into the left atrium **1078.** A user can then further manipulate the delivery system **10** to create an even greater bend in the rail subassembly **21.** Further, a user can torque the entire delivery system **10** to further manipulate and control the position of the delivery system **10.** In the fully bent configuration, a user can then place the replacement valve in the proper location. This can advantageously allow delivery of a replacement valve to an in-situ implantation site, such as a native mitral valve, via a wider variety of approaches, such as a transseptal approach.

**Figure 13** illustrates a schematic representation of a portion of an embodiment of a replacement heart valve (implant **30)** positioned within a native mitral valve of a heart **83.** Further details regarding how the implant **30** may be positioned at the native mitral valve are described in U.S. Pat. Pub No. 2015/032800 published on November 19, 2005, the entirety of which is hereby incorporated by reference, including but not limited to Figures 13A-15 and paragraphs [0036]-[0045]. A portion of the native mitral valve is shown schematically and represents typical anatomy, including a left atrium **1078** positioned above an annulus **1106** and a left ventricle **1080** positioned below the annulus **1106.** The left atrium **1078** and left ventricle **1080** communicate with one another through the annulus **1106.** Also shown schematically in Figure 13 is a native mitral leaflet **1108** having chordae tendineae **1111** that connect a downstream end of the mitral leaflet **1108** to the papillary muscle of the left ventricle **1080.** The portion of the implant **30** disposed upstream of the annulus **1106** (toward the left atrium **1078)** can be referred to as being positioned supra-annularly. The portion generally within the annulus **1106** is referred to as positioned intra-annularly. The portion downstream of the annulus **1106** is referred to as being positioned sub-annularly (toward the left ventricle **1080).**

As illustrated in Figure 13, the implant **30** can be positioned so that the ends or tips of the distal anchors **37** are on a ventricular side of the mitral annulus **1106.** The distal anchors **37** can be positioned such that the ends of tips of the distal anchors **37** are on a ventricular side of the native leaflets beyond a location where chordae tendineae **1111** connect to free ends of the native leaflets. The distal anchors **37** may extend between at least some of the chordae tendineae **1111** and, in some situations can contact or engage a ventricular side of the annulus **1106.** It is also contemplated that in some situations, the distal anchors **37** may not contact the annulus **1106,** though the distal anchors **37** may still contact the native leaflet **1108.** In some situations, the distal anchors **37** can contact tissue of the left ventricle **1080** beyond the annulus **1106** and/or a ventricular side of the leaflets **1108.**

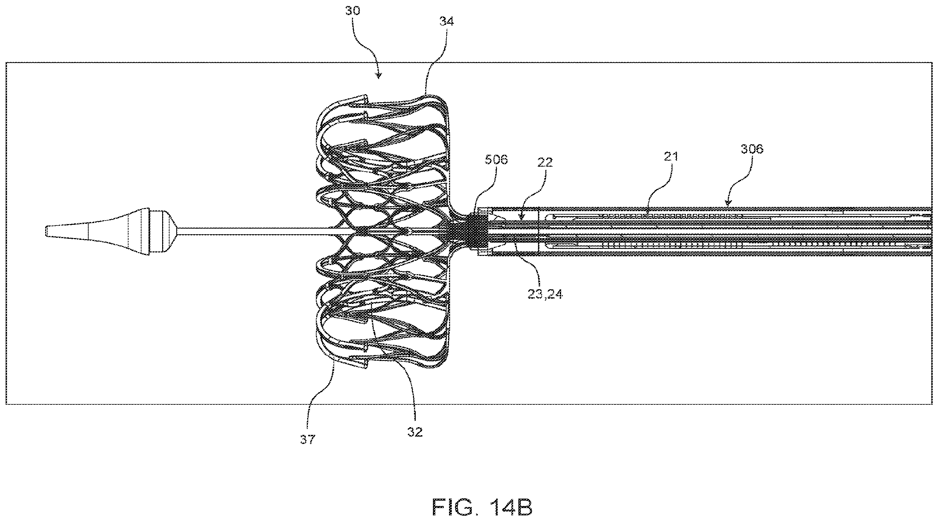

**Figures 14A****-14E** illustrate operation of the delivery device **15** by showing various steps of deployment and implantation of the implant (e.g., replacement heart valve) **30** using the delivery device **15** described herein. Figures 14A-14E show the positioning of the various subassemblies of the delivery device **15** with respect to each other and with respect to the implant **30** at the various steps of the procedure. The subassemblies are shown in a side cross-section view to facilitate visualization of the various subassemblies. For sake of simplicity and illustration, various portions of the implant **30** (e.g., skirt assembly **38** and padding **39)** are not shown. **Figure 14A** illustrates the delivery device **15** at a time in an implantation procedure in which the replacement heart valve **30** is completely retained within the capsule subassembly **306** of the outer subassembly **20** in a compressed configuration. As shown, a proximal-most portion (e.g., eyelet portion) of the replacement heart valve **30** is retained within the pusher **506** of the mid-shaft subassembly **22** and the remainder of the replacement heart valve **30** is compressed by the capsule subassembly **306.** With reference to **Figure 14B****,** the capsule subassembly **306** has been retracted proximally (e.g., toward a proximal handle **14** of the delivery device **15** by rotating capsule knob **905** of the handle **14)** to a position such that the replacement heart valve **30** is no longer constrained by the capsule subassembly **306** and the replacement heart valve **30** has been allowed to partially self-expand. The proximal-most portion (e.g., eyelet portion) of the replacement heart valve **30** remains constrained in a compressed configuration by the pusher **506** of the mid-shaft subassembly **22** such that the entire replacement heart valve **30** is not yet fully deployed.

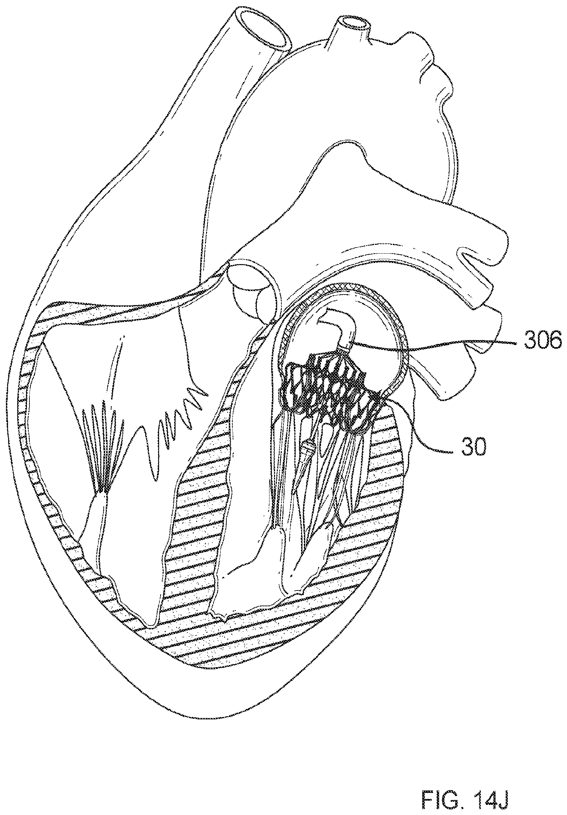

As can be appreciated, the deployment of the distal and mid portions of the replacement heart valve **30** may occur in stages over time and not in an immediate full deployment. For example, the distal anchors **37** of the inner frame **32** of a dual-frame structure may be deployed first prior to deployment of the outer frame **34** (e.g., while the outer frame **34** and mid portion of the inner frame **32** remain constrained within the capsule subassembly **306),** such as shown for example, in Figure 5C. The distal anchors **37** of the inner frame **32** may be positioned through chordae tendineae of a native heart valve (e.g., mitral valve) and/or subannularly so as to capture the native leaflets of the heart valve between the distal anchors **37** and a main body of the outer frame so as to keep the native leaflets in an open configuration and to anchor the replacement heart valve **30** as a whole. Such a configuration and position is shown in Figure 14J.

With reference to **Figure 14C****,** the manifold subassembly **24** and the release subassembly **23** have been advanced distally by rotation of the release knob **925** (as discussed previously herein) while the mid-shaft subassembly **22** remains fixed such that the proximal-most portion (e.g., eyelet portion) of the replacement heart valve **30** is advanced distally out of the pusher **506** of the mid-shaft subassembly **22,** thereby deploying the replacement heart valve **30** into a fully-expanded configuration. However, the replacement heart valve **30** still remains tethered to the manifold subassembly **24** by the tether(s) **710** because the manifold subassembly **24** and the release subassembly **23** are in the "locked" configuration, as described previously herein in connection with Figures 8A-8D.

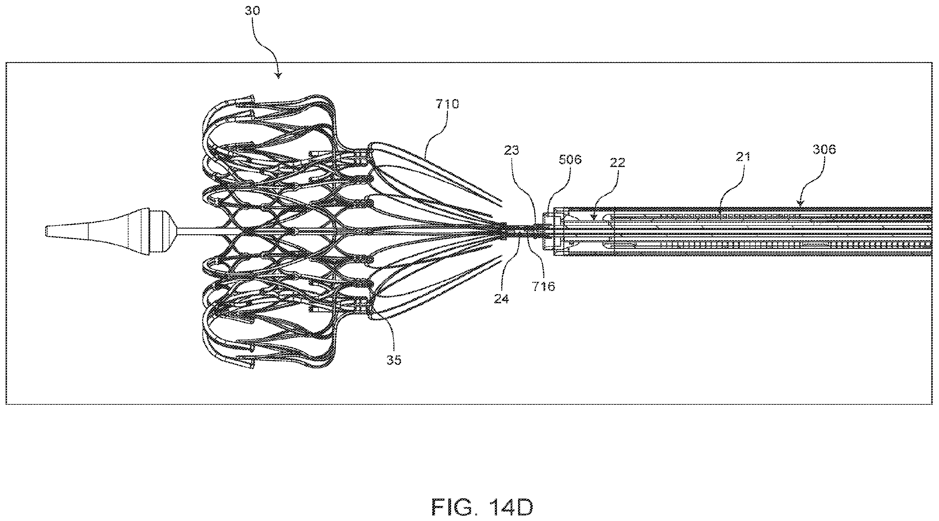

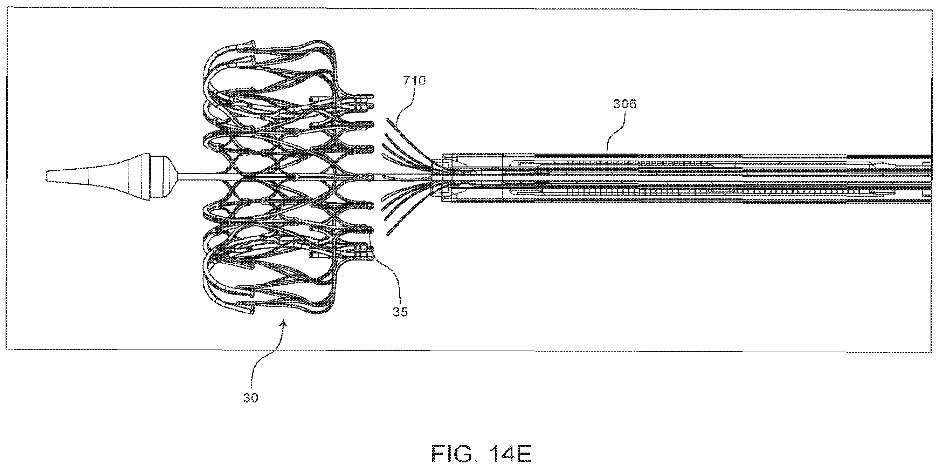

With reference to **Figure 14D****,** the manifold subassembly **24** has been moved distally relative to the release subassembly **23** (to transition the release subassembly **23** and the manifold subassembly **24** into the unlocked configuration described in connection with Figures 8A-8D) and the suture loop ends of the tether(s) **710** that were previously coupled to the tabs **716** of the manifold subassembly **24** have been uncoupled or released. With reference to **Figure 14E****,** the manifold subassembly **24** and the release subassembly **23** are retracted proximally together until the free suture loop ends of the tether(s) **710** are pulled out of the proximal eyelets **35** of the replacement heart valve **30** and the delivery device **15** is then removed from the implantation location, thereby leaving the replacement heart valve **30** in its final implantation location. The manifold subassembly **24** and the release subassembly **23** may be retracted into the outer sheath subassembly **20** or the outer sheath subassembly **20** may be advanced to cover the distal ends of the manifold subassembly **24** and the release subassembly **23** prior to withdrawal of the delivery device **15.** However, the distal ends of the manifold subassembly **24** and the release subassembly **23** may alternatively remain distal of (outside) the outer sheath subassembly **20** as the delivery device **15** is withdrawn.

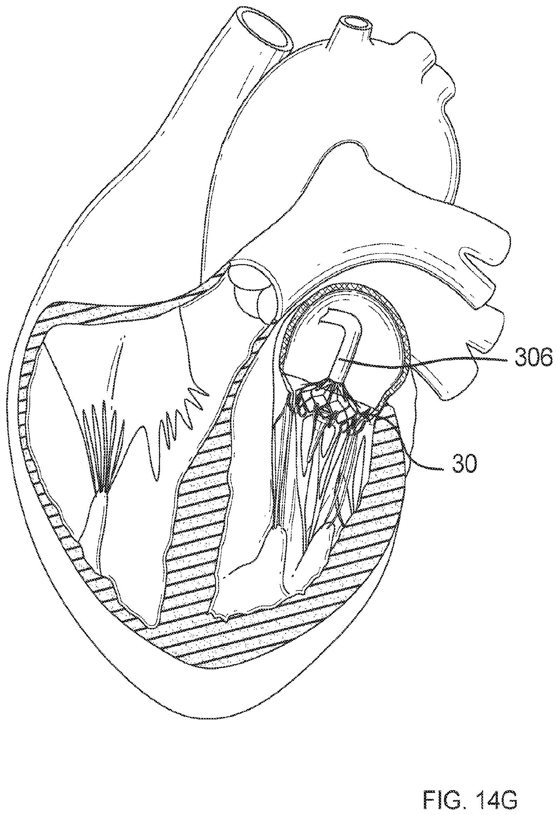

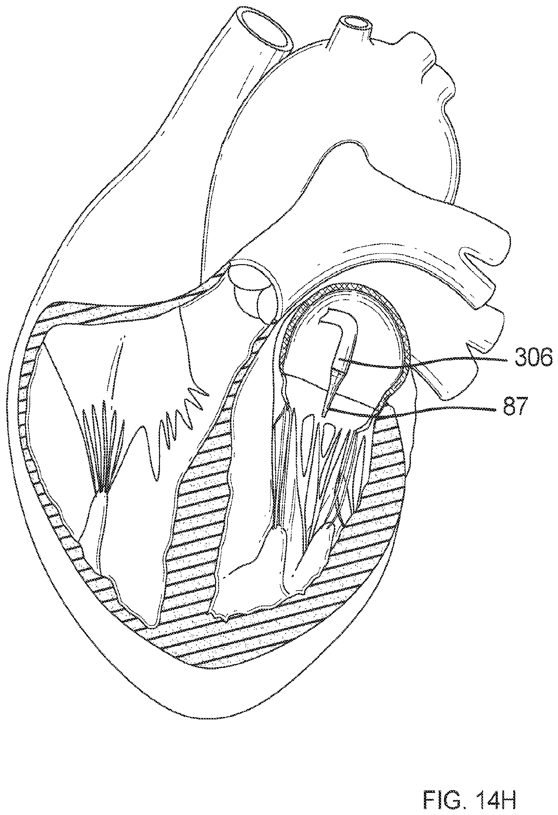

Figures **14F****-4K** illustrate various steps of deployment and recapture of the implant (e.g., replacement heart valve) **30** using the delivery device **15** described herein. For sake of simplicity and illustration, only the inner frame **32** and outer frame **34** of the implant **30** is illustrated (e.g., skirt assembly **38** and padding **39** as shown in Figure 2C is not shown). The capsule subassembly **306** advantageously facilitates recapture of the implant **30** after an initial deployment. **Figure 14F** illustrates an initial deployment of the implant **30** from the delivery device **15.** For example, the initial deployment may be within a mitral valve annulus following a transfemoral and/or transseptal approach. Note that the implant **30** remains tethered to the delivery device **15** upon initial full deployment of the implant **30** to a fully expanded configuration. In some instances, a clinician may decide after performing various tests (e.g., using various imaging modalities and measurements) that the initial deployment location is not ideal. For example, the ideal position may be more superior (e.g., toward the atrium) or inferior (e.g., toward the ventricle) of the initial deployment location. In order to prevent damage to the implant **30** and to the heart, the implant **30** may be recaptured prior to movement of the implant **30** to a new implantation location. Recapturing of the implant **30** may be performed by advancing the capsule subassembly **306** of the outer sheath subassembly **20** distally over the implant **30** to cause the implant **30** to transition to a compressed configuration. Figures 14G and 14H show various stages of recapturing of the implant **30.** As shown in **Figure 14G****,** the capsule subassembly **306** has been advanced distally (e.g., by rotating capsule knob **905** in a first direction) to capture the proximal portion of the implant **30.** **Figure 14H** shows full recapture of the implant **30,** with the capsule subassembly **306** being fully advanced distally (e.g., until contact with a nose cone **87** of the nose cone subassembly or until the implant **30** is fully retained within the capsule subassembly **306).** The configuration of Figure 14H corresponds to the configuration of Figure 14F but within the heart location.

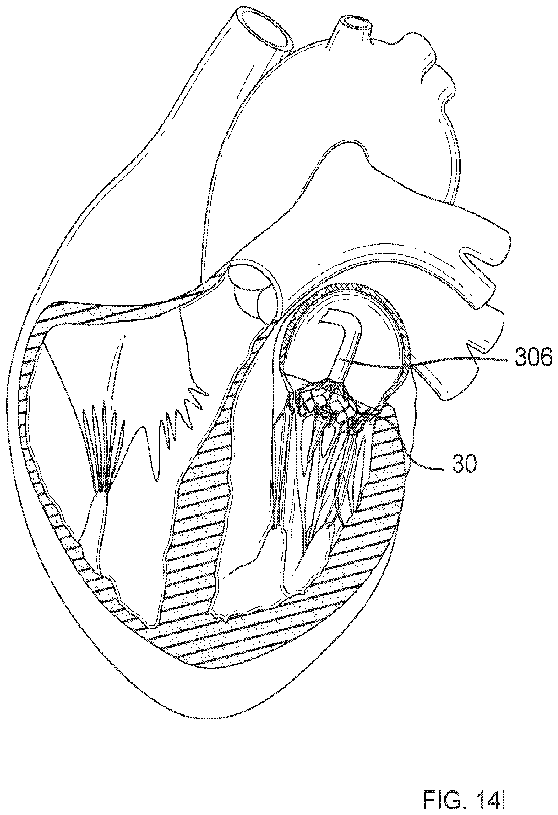

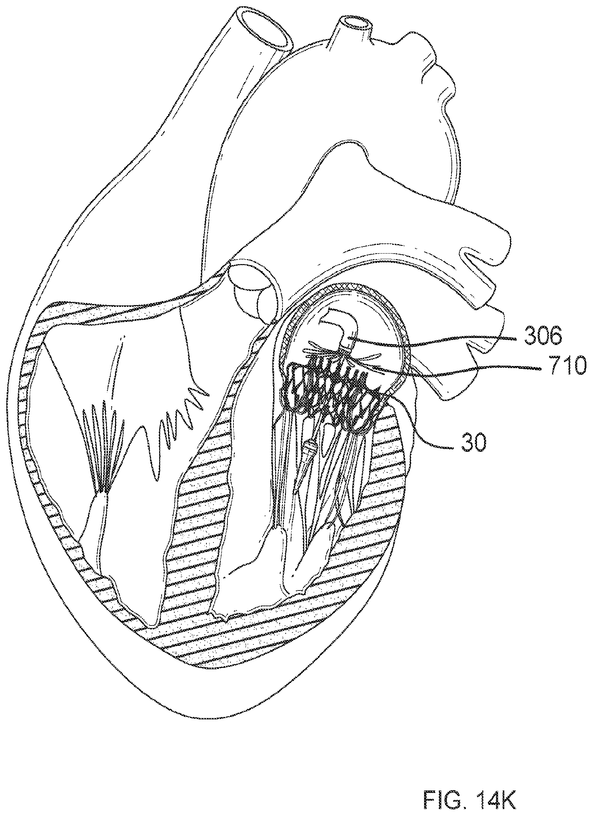

After movement of the distal end of the delivery device **15** to a new location, the capsule subassembly **306** of the outer sheath subassembly **20** can again be retracted proximally (e.g., by rotating capsule knob **905** in an opposite, second direction from the first direction) to unsheathe the distal portion of the implant **30** (e.g., at a new implantation location within a mitral valve annulus or tricuspid valve annulus), as shown in **Figure 14I****.** The manifold and release subassemblies **23, 24** may then be advanced distally together (e.g., by rotation of release knob **925)** to deploy the proximal-most portion of the implant **30** (e.g., proximal eyelets, posts or struts) out of the pusher **506** of the mid-shaft subassembly **22,** as shown in **Figure 14J****.** After confirmation that the fully-deployed implant **30** is in an ideal and proper final implantation location, the tether **710** (e.g., tether loop ends) may be caused to be released from the manifold subassembly **24** (as shown in **Figure 14K****)** by continued rotation of the release knob **925** so that the release knob **925** translates further distally until the release subassembly **23** encounters a physical stop member in the handle **14** and the manifold subassembly **24** continues to translate distally with respect to the release subassembly **24.** The delivery device **15** can be retracted and removed from the heart and then from the vasculature and then from the subject altogether.

### Skirt Assembly and Methods of Manufacturing or Assembling

**Figures 15A** and **15B** illustrate different views of a configuration of a fully-assembled implant (e.g., valve prosthesis) **1230** including a skirt assembly **1238** (shown in **Figures 17A****-17D)** positioned between the frames **1232, 1234** (shown in **Figures 16A** and **16B****)** and padding **1239** surrounding the anchors **1237.** The implant **1230** can be similar to the configuration of the implant **30** illustrated in and described in relation to **Figures 2** - **2K-2.** Reference numerals of the same or substantially the same features may share the same last two digits.

**Figure 15C** shows a prosthetic leaflet stitched to an inner frame **32** of a dual-frame valve prosthesis (e.g., implant **30, 1230).** The inner frame **32** of the dual-frame valve prosthesis may include a prosthetic valve assembly composed of a plurality of flexible leaflets **1108A** arranged to collapse in a tri-leaflet arrangement and reinforcing strips **1108B** for securing the plurality of prosthetic leaflets **1108A** to the inner frame **32** and securing a cusp edge portion **1108C** of each prosthetic leaflet **1108A** to the first end portion of the reinforcing strip **1108B.** The dual-frame valve prosthesis (e.g., implant **1230)** may be implanted to replace any heart valve (e.g., mitral valve, tricuspid valve, aortic valve, pulmonic valve) and the inner frame **32** of the dual-frame valve prosthesis may be configured to have an "hourglass" profile or shape when in an expanded configuration, as described elsewhere herein. Although the prosthetic leaflet stitching and valve assembly implementations are generally described herein with reference to a dual-frame valve prosthesis, the leaflet stitching and valve assembly implementations may also be used for assembly/manufacturing of a single frame implant or implants with more than two frames (e.g., three or more frames). For example, aortic and pulmonic prosthetic valve implants may incorporate a single frame (e.g., single frame valve with an hourglass profile) instead of a dual frame.

**Figure 15D-1 to 15D-5** show double stitching applied to a prosthetic leaflet to securely attach to an inner frame of the dual-frame valve prosthesis; however, the double stitch line **1108D** can be incorporated into stitching for any prosthetic valve (e.g., single frame or more than two frames) and not only dual-frame valve prostheses. The double stitch line **1108D** can be seen in **FIGS. 15D-1 to 15D-3** by following, or connecting, the two separate rows of dots in the figures. Methods of assembling prosthetic leaflets **1108A** to other components of the dual-frame valve prosthesis (e.g., portions or components of a skirt assembly and/or frame assembly) include folding over portions of the prosthetic leaflet edges or cloth skirt edges so as to cover exposed suture portions and to prevent direct contact between suture portions or potentially abrasive skirt edges and the prosthetic leaflets (e.g., belly portions of the prosthetic leaflets). The skirt assembly (e.g. skirt assembly **1238, 1248)** may include multiple skirt portions. For example, the skirt assembly may include a first portion that includes a double stitch line with pre-drilled laser holes configured to align with holes of a double stitch line of a prosthetic leaflet. In other implementations, there are no pre-drilled laser holes and the stitching is sewn through cloth or tissue free hand without pre-formed (e.g., laser-drilled) holes. The first portion may comprise a reinforcing cloth skirt strip **1248A** adapted to facilitate attachment of the skirt assembly to the prosthetic leaflets. The skirt assembly may also include a main portion **1248B** adapted to facilitate attachment to a frame structure. In some implementations, a first portion of the skirt assembly (e.g., reinforcing cloth skirt strip(s) **1248A)** that is sutured to the prosthetic leaflet **1108A** can be folded on itself (either outwardly or inwardly) so as to cover a first line of exposed sutures **1109A,** thereby preventing contact of the leaflet **1108A** with a potentially abrasive skirt edge formed by cutting of the reinforcing cloth skirt strip **1248A** and also preventing any portion of the sutures **1109A, 1109B** from contacting the leaflet **1108A,** which contact could also cause abrasion over time. **Figures 15D-4 and 15D-5** illustrate examples of different portions of the reinforcing cloth skirt strip **1248A** of the skirt assembly being folded over itself to prevent exposure or contact of the sutures **1109** with the leaflet **1108A.**

For example, a method of assembling the leaflet **1108A** to a dual-frame valve structure includes securing at least a component or portion of the skirt assembly (e.g., skirt assembly **1238)** to an inner frame via a first line of sutures **1109A** using reinforcing strips (e.g., reinforcing strips **1248A);** securing the leaflets **1108A** to the reinforcing strips **1248A** via the primary suture or first line of sutures **1109A;** folding the reinforcing strips **1248A** over the first line of sutures **1109A** to cover them and then suturing the folded-over portion of the reinforcing strips **1248A** of the skirt assembly with a second line of sutures (e.g., secondary sutures) **1109B** parallel to and spaced apart from the first line of sutures **1109A,** which also do not contact any portion of the leaflet **1108A.** The primary sutures **1109A** and secondary sutures **1109B** create more than one stitch line **1108D** (e.g., a double stitch line, or two stitch lines). Again, the method of assembly may be applied to a single frame valve structure in addition to a dual-frame valve structure.

With reference to **FIGS. 15E-1 to 15E-4,** the method of assembling the leaflets **1108A** to the dual-frame valve structure (e.g., implant **30, 1230)** may alternatively or additionally include folding a cusp edge portion or tab **1108C** of the leaflets **1108A** inwardly and applying sutures **1109** to secure the folded cusp edge portion or tab **1108C** to the reinforcing cloth strips **1237A** of the skirt assembly. In this implementation, neither the sutures **1109** nor the skirt assembly (e.g., reinforcing cloth strips **1237A)** are in contact with a belly portion of the leaflets **1108A.** Again, this method of assembling may be applied to a single frame valve structure as well.

In some implementations, a double stitch line **1108D** can include a second stitch line at the cusp edge portion **1108C** of each prosthetic leaflet **1108A** where it is attached to other components of the dual-frame valve prosthesis, so as to increase the retention strength of the stitch line and more evenly distribute stress throughout valve opening and closing while adding minimal extra bulk. The folded cusp edge portion or tab **1108C** being positioned between the cloth of the skirt assembly and the exposed suture portions advantageously acts as a barrier to prevent abrasion on leaflet bellies as the prosthetic valve opens and closes over time. The leaflets **1108** may be formed of bovine or porcine pericardial tissue (such as RESILIA^{®} bovine pericardial tissue). The RESILIA bovine pericardial tissue may advantageously resist calcification.

**Figure 16A** illustrates a configuration of the inner frame **1232** coupled to a prosthetic valve assembly **1231** comprising a plurality of prosthetic valve leaflets (not shown). **Figure 16B** illustrates a configuration of the outer frame **1234.** The inner frame **1232** can be similar to the configuration of the inner frame **32** and the outer frame **1234** can be similar to the configuration of the outer frame **34** illustrated in and described in relation to **Figures 2-2K-2.** Reference numerals of the same or substantially the same features may share the same last two digits.

**Figures 17A****-17D** illustrate a configuration of the skirt assembly **1238.** The skirt assembly **1238** can include a cloth material. For example, the skirt assembly **1238** can include a single, integral piece of cloth or multiple pieces of cloth coupled together. The skirt assembly **1238 can** include a proximal, or inflow, portion **1238A,** a middle, or intermediate, portion **1238B,** and a distal, or outflow, portion **1238C.**

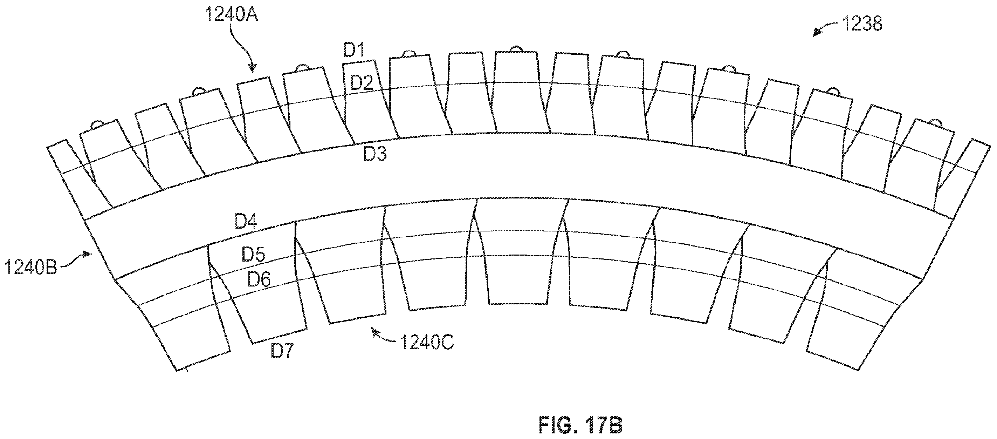

As shown in **Figure 17B****,** the skirt assembly **1238** can include varying diameters. For example, the skirt assembly **1238** can include a plurality of diameters **D1, D2, D3, D4, D5, D6, D7.** In some configurations, the third diameter **D3** can be the greatest diameter. In some configurations, the seventh diameter **D7** can be the smallest diameter. The first, second, fourth, fifth, and sixth diameters **D2, D3, D4, D5, D6** can be between the third diameter **D3** and the seventh diameter **D7.** The plurality of diameters **D1, D2, D3, D4, D5, D6, D7** can be the same diameter or each of the diameters can be different. In accordance with several implementations, the skirt assembly **1238** techniques described herein advantageously facilitate transitioning between varying diameters within one single piece of cloth without having to cut the cloth into multiple components. Advantageously, by having a skirt assembly **1238** as an integral component with varying diameters **D1, D2, D3, D4, D5, D6, D7,** the amount of cloth used can be reduced and the thickness of the skirt assembly **1238** can be reduced. By reducing the thickness of the skirt assembly **1238,** the loading and retrieval forces exerted on the implant **1230** during delivery and retrieval can be reduced.

As shown in the illustrated configuration, the skirt assembly **1238** can include a plurality of portions or extensions **1240A, 1240C** to vary the diameter of the skirt assembly **1238.** For example, the middle portion **1238B** can include a body portion **1240B,** the inflow portion **1238A** can include a plurality of proximal portions of extensions **1240A** extending from the body portion **1240B,** and the outflow portion **1238C** can include a plurality of distal portions or extensions **1240C** extending from the body portion **1240B.** The proximal extensions **1240A** can be configured to be positioned between the inner frame **1232** and the outer frame **1234.** For example, the outer frame **1234** may include a plurality of openings **1234D** (as shown in **Figure 16B****)** and the proximal extensions **1240A** can be received by the plurality of openings **1234D** such that the proximal extensions **1240A** can be positioned between the inner and outer frames **1232, 1234.** The body portion **1240B** can be configured to be positioned exterior to the outer frame **1234** when the implant **1230** is assembled. The distal extensions **1240C** can be configured to be positioned between the inner frame **1232** and the outer frame **1234** on the inflow side of the implant **1230.** For example, the distal extensions **1240C** can be inserted through the space distal to a distal edge of the outer frame **1234** such that the distal extensions **1240C** can be positioned between the inner and outer frames **1232, 1234** on the outflow side of the implant **1230.**

In the illustrated configuration, the skirt assembly **1238** has a plurality of trapezoidal portions **1240A, 1240C.** In other configurations, the skirt assembly **1238** can include portions **1240A, 1240C** having a square shape, a triangular shape, a circular shape, or any other suitable shape. The plurality of proximal extensions **1240A** can include 18 proximal extensions **1240A.** In other configurations, the plurality of proximal extensions **1240A** can include any number of proximal extensions (e.g., less than or more than 18 proximal extensions). The plurality of distal extensions **1240C** can include 9 distal extensions. In other configurations, the plurality of distal extensions **1240C** can include any number of distal extensions (e.g., less than or more than 9 distal extensions).

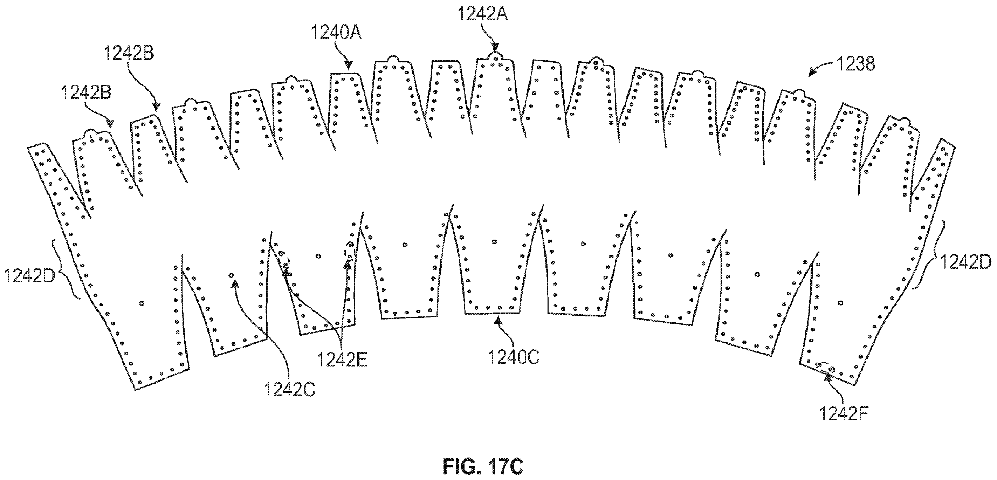

As shown in **Figure 17C****,** the skirt assembly **1238** can include a plurality of features **1242A, 1242B, 1242C, 1242D, 1242E, 1242F, 1242G, 1242H** configured to assist in the assembly of the skirt assembly **1238** and the implant **1230.** For example, the plurality of features can include a plurality of tabs **1242A** that can extend from one or more of the proximal extensions **1240A.** The tabs **1242A** can be configured to be positioned between the eyelets **1235** of the inner frame **1232** and the outer frame **1234.** Advantageously, the tabs **1242A** can prevent corrosion of the eyelets **1235.** In the illustrated configuration, the plurality of tabs **1242A** can include 9 tabs **1242A** on alternating proximal extensions **1240A.** In some configurations, the plurality of tabs **1242A** can be on each of the proximal extensions **1240A** or on fewer than half of the proximal extensions **1240A.**

In some configurations, the plurality of features can include a keying feature **1242B.** The keying feature **1242B** can be positioned on one side of one or more of the proximal extensions **1240A.** The keying feature **1242B** can indicate which side of the proximal extensions **1240A** should be positioned on top of adjacent proximal extensions **1240A** when the skirt assembly **1238** is folded and sewed into the folded configuration, as further describe below in reference to **Figure 17D****.**

In some configurations, the plurality of features can include a plurality of holes **1242C** in the distal extensions **1240C.** For example, each of the distal extensions **1240C** can include one or more holes **1242C.** In the illustrated configurations, each distal extension **1240C** has a single hole **1242C.** The plurality of holes **1242C** can allow blood to flow into the enclosed volume of the implant **1230** (e.g., the volume between the inner frame **1232** and prosthetic valve assembly **1231,** and the outer frame **1234** and skirt assembly **1238).** The plurality of holes **1242C** can be sized such that blood can flow through the holes **1242C** into the implant **1230** but the blood is prevented or restricted from flowing out of the implant **1230.** The holes **1242C** can be positioned between the anchors **1237** of the inner frame **1232** (shown in **Figure 16A****)** when the implant **1230** is assembled so that the anchors **1237** do not restrict the blood from through the holes **1242C.** Moreover, the holes **1242C** can assist the manufacturer in properly attaching the skirt assembly **1238** to the inner and outer frames **1232, 1234** by ensuring the holes **1242C** are positioned between the anchors **1237.**

In some configurations, the plurality of features can include at least one tapered section **1242D.** The at least one tapered section **1242D** can be positioned on the outside of the outer frame **1234.** In some configurations, the at least one tapered section **1242D** can include two tapered sections **1242D** that can be sewn together when the implant **1230** is assembled.

In some configurations, the plurality of features can include first alignment features **1242E** and second alignment features **1242F.** The first alignment features **1242E** can be positioned on at least one side of at least one distal extension **1240C** and/or adjacent the hole(s) **1242C.** In the illustrated configuration, each distal extension **1240C** includes a pair of first alignment features **1242E** positioned on either side of the hole **1242C.** The first alignment features **1242E** can be configured to align with a distal portion of the anchors **1237** to ensure proper placement of the skirt assembly **1238** relative to the inner and outer frames **1232, 1234.** The first alignment features **1242E** can include a plurality of holes, a plurality of dots, and/ or other visual or tactile indicator.

The second alignment features **1242F** can be positioned on at least one distal extension **1240C.** In the illustrated configuration, each distal extension **1240C** includes second alignment features **1242F** along an edge of the distal extension **1240C.** The second alignment features **1242F** can be configured to be aligned with the inner skirt of the prosthetic valve assembly **1231** to ensure proper placement of the skirt assembly **1238** relative to the inner and outer frames **1232, 1234.** The second alignment features **1242F** can include a plurality of holes, a plurality of dots, and/or other visual or tactile indicator.

**Figure 17D** illustrates the skirt assembly **1238** in a folded configuration with the distal extensions **1240C** sewed together and the tapered sections **1242D** sewed together. When the skirt assembly **1238** is folded, adjacent proximal extensions **1240A** can overlap and/or adjacent distal extensions **1240C** can overlap such that the adjacent proximal extensions **1240A** and/or the adjacent distal extensions **1240C** can be sewn together.

In some embodiments, a cloth material of the skirt assembly may be treated to soften an edge which may be roughened when laser cutting is applied. **Figures 17E-1 and 17E-2** show softened edges of cloth material used for the skirt assembly of **Figures 17A to 17D****.** The roughened edge can be softened by applying a soldering iron with heat within a threshold temperature to an edge of the integral piece of cloth material. For example, a soldering iron can be applied to melt the fibers of the cloth into one smooth melted edge **1238D.** Alternatively, a z-axis feature of a laser to defocus the laser can be applied to create a thicker area of melted cloth that is smooth along the edge.

**Figure 17F** shows a process of applying an interlocking stitch of the cloth material used for the skirt assembly of **Figures 17A to 17D** to eliminate knots. In the current method, a transcatheter heart valve is generally hand sewn using a suture, and therefore, there is typically a knot that acts as a speed bump for a delivery system to go over when the valve is crimped. In some implementations, an interlocking stitching technique can be applied to eliminate the knot. The interlocking stitch may use a woven structure of a suture itself to puncture and interlock within its own strands and can secure the suture without creating a bulky knot. In some implementations, with reference to **Figure 17F****,** a needle tip can be punctured within a center of the woven structure of the suture to form an interlocked structure, which can create a secure beginning or end point for the suture. The interlocking method may include sewing a needle through a force fiber (1), pulling a suture taut (2), sewing the needle through the force fiber again to create the interlock stitch on the opposite side (3), and finally pulling the suture taut again (4) to complete the interlock stitch.

### Additional Tether Retention Assembly Configuration

**Figures 18A****-18F** show a configuration of a distal subassembly **1303.** The distal subassembly **1303** can be similar to the configuration of the distal subassembly **703** illustrated in and described in relation to **Figures 7A****-7E.** Reference numerals of the same or substantially the same features may share the same last two digits.

As shown in **Figures 18A****-18C,** the distal tether retention component **1307** can be configured to retain the tether or suture **710.** The tether or suture **710** can include a plurality of distal loops **1320.** The distal tether retention component **1307** can be spaced from the proximal tether retention component **1306.** For example, the distal subassembly **1303** can include a middle component **1312** between the proximal and distal tether retention components **1306, 1307.** In some configurations, the middle component **1312** can include a tube. The middle component **1312** can be made of a metal material, such as stainless steel. In some configurations, the proximal tether retention component **1306** can have a diameter greater than a diameter of the middle component **1312** and/or the manifold cable **705.** In some configurations, the distal tether retention component **1307** can have a diameter greater than the diameter of the middle component **1312** and/or the manifold cable **705.**

As shown in **Figure 18A****,** the distal tether retention component **1307** can include a plurality of slots **1318** along the portion of the distal tether retention component **1307** that extends radially beyond the middle component **1312** and/or the manifold cable **705.** The plurality of slots **1318** can include a length that extends along a longitudinal axis of the distal subassembly **1303.** The illustrated configuration has nine slots **1318** in the distal tether retention component **1307.** Other numbers of slots **1318** (e.g., two, four, five, six, seven, eight) may also be used in other configurations. The slots **1318** can be configured to receive portions of the tether or suture **710** and prevent the tether or suture **710** from being removed or uncoupled from the distal tether retention component **1307.**

As shown in **Figures 18B** **and** **18C****,** the proximal tether retention component **1306** can include a plurality of slots **1314** along the portion of the proximal tether retention component **1306** that extends radially beyond the middle component **1312** and/or the manifold cable **705.** The plurality of slots **1314** can include a length that extends along a longitudinal axis of the distal subassembly **1303.** The number of slots **1314** of the proximal tether retention component **1306** may correspond with the number of slots **1318** of the distal tether retention component **1307.** The illustrated configuration has nine slots **1314** in the proximal tether retention component **1306.** Other numbers of slots **1314** (e.g., two, four, five, six, seven, eight) may also be used in other configurations. In some configurations, one or more of the slots **1314** can be configured to receive a distal loop **1320** of the tether or suture **710.** In other configurations, one or more of the slots **1314** can be configured two or more distal loops **1320** of the tether or suture **710.** In some configurations, the slots **1314** of the proximal tether retention component **1306** can align with the slots **1318** of the distal tether retention component **1307.** In other configurations, the slots **1314** of the proximal tether retention component **1306** can be offset from the slots **1318** of the distal tether retention component **1307.**

**Figure 18D** illustrates the tether or suture **710** being secured to the distal subassembly **1303.** As previously described, the slots **1314** can receive a distal loop **1320** of the tether or suture **710.** A release (or locking) tether/suture **1322** can extend through the distal loops **1320,** thus preventing the implant **30, 1230** from being released from the distal subassembly **1303** until ready. For example, a free end **1324** of the release tether/suture **1322** can be inserted through the distal loops of the tether or suture **710** to secure the tether or suture **710** to the implant **30, 1230.**

**Figures 18E** **and** **18F** illustrate the tether or suture **710** being removed from the distal subassembly **1303.** The release tether/suture **1322** can be withdrawn so that a free end **1324** of the release tether/suture **1322** can pass through the distal loops **1320,** thus releasing the implant **30, 1230** from the tethered attachment to the distal subassembly **1303.** Multiple release (or locking) tethers/sutures **1322** may be used in some embodiments (e.g., one for each distal loop **1320** or one for multiple distal loops **1320).**

**Figures 19A** **and** **19B** illustrate another configuration of a proximal tether retention component **1406** and a middle component **1412** similar to the embodiments of the proximal tether retention component **706, 1306** and the middle component **1312** illustrated in and described in relation to **Figures 7A****-7E** and **18A-18F.** Reference numerals of the same or substantially the same features may share the same last two digits.

The plurality of slots **1414** of the proximal tether retention component **1406** can include three slots **1414.** Each of the slots **1414** can be configured to receive one or more distal loops **1320** of the tether or suture **710** (not shown). In some configurations, as shown in **Figure 19A****,** the shaft extending between the middle component **1412** and the manifold cable **705** can include a plurality of apertures **1426.** The apertures **1426** can be circumferentially spaced apart. As shown, the apertures **1426** can align with the slots **1414.** In some configurations, the apertures **1426** can be at least partially offset from the slots **1414.**

### Clocking or Implant Orientation Control

**Figures 20A****-20C** illustrate a configuration of a handle **1514** similar to the embodiments of the handle **14** illustrated in and described in relation to **Figures 1** and **11****.** The handle **1514** can be configured to rotate an implant **30, 1230** during delivery. For example, the implant **30, 1230** can be rotated to avoid certain anatomical structures, to enhance sealing of the implant **30, 1230,** and/or to avoid erosion in certain anatomical areas (e.g.. the aortic root in the atrium).

As shown, the handle **1514** can include a capsule knob **1505** (similar to the capsule knob **905** illustrated in and described in relation to **Figures 9A** and **9B****),** an orientation mechanism **1516** configured to rotate the implant **30, 1230** (not shown) during implantation, and a linear guide **1524.** For example, the orientation mechanism **1516** can include an orientation knob **1516** extending from a side of the handle **1514** that can be rotated about a longitudinal axis of the orientation knob **1516.** In some configurations, the handle **1514** can include a rotation mechanism **1518** coupled to the orientation knob **1516.** When the orientation knob **1516** is rotated, the rotation mechanism **1518** can also rotate. In some configurations, the rotation mechanism **1518** can include a worm gear mechanism **1520** and an adapter **1522.** The orientation knob **1516** can be coupled to the worm gear mechanism **1520** and be configured to rotate the worm gear mechanism **1520** when the orientation knob **1516** is rotated. The worm gear mechanism **1520** can be coupled to the linear guide **1524** such that the worm gear mechanism **1520** can rotate the linear guide **1524** when the orientation knob **1516** is rotated. The adapter **1522** can be coupled to the linear guide **1524** such that the linear guide **1524** can rotate the adapter **1522** when the linear guide **1524** is rotated. The adapter **1522** can be coupled to the outer proximal shaft **302** of the capsule assembly **306** (not shown). When the linear guide **1524** rotates the adapter **1522,** the adapter **1522** can rotate the outer proximal shaft **302.** In some configurations, the adapter **1522** can be configured to control linear motion of the outer proximal shaft **302** when the capsule knob **1505** is rotated.

In some configurations, the orientation knob **1516** can rotate the outer proximal shaft **302** of the capsule assembly **306.** During delivery of the implant **30, 1230,** the orientation knob **1516** can be actuated to rotate the outer proximal shaft **302** of the capsule subassembly **306** and the implant **30, 1230** within the capsule assembly **306** for positioning the implant **30, 1230** within the patient.

In some configurations, the orientation knob **1516** can include a plurality of indicators on an outer surface of the orientation knob **1516.** The indicators on the orientation knob **1516** can correlate with the rotation of the implant **30, 1230.** For example, the indicators can show a certain number of degrees that the implant **30, 1230** has been rotated. In some configurations, the orientation knob **1516** can be directly coupled to the outer proximal shaft **302** of the capsule assembly **306** such that rotating the orientation knob **1516** can directly rotate the outer proximal shaft **302.** In some configurations, the orientation mechanism **1516** can be a lever configured to be pushed and/or pulled to rotate the implant **30, 1230** during delivery.

**Figures 20D, 20E, 20F and 20G** further illustrate an embodiment of an orientation mechanism of **Figure 20C** connected to an outer lumen **20A** within which an implant (e.g., implant **30, 1230)** can be rotated. The detailed gear mechanism is described above in connection with **Figures 20B** **and** **20C****,** and therefore, the detailed description of the gear mechanism of the orientation mechanism is omitted here. By rotating the orientation mechanism or knob **1516,** as shown in **Figure 20F to Figure 20G****,** an implant **30, 1230** (not shown) can be rotated during implantation via the gear mechanism to position the implant to have a desired rotational orientation (e.g., to avoid potential for conduction disturbances caused by contact of a portion of the implant with certain tissue). As discussed previously, the gear mechanism can include a worm gear mechanism **1520** and a capsule adapter **1522.** The orientation knob **1516** can be coupled to the worm gear mechanism **1520** and be configured to rotate the worm gear mechanism **1520** when the orientation knob **1516** is rotated. The worm gear mechanism **1520** can be coupled to a linear guide **1524** such that the worm gear mechanism **1520** can rotate the linear guide **1524** (and thus the outer sheath subassembly **20** and capsule subassembly **306** and the implant positioned therein) when the orientation knob **1516** is rotated. Rotation of the outer sheath subassembly **20** may passively cause rotation of other subassemblies and the implant due to being operably coupled to the outer sheath subassembly 20 but may not be rotated directly by rotation of the orientation knob **1516.**

An implant (e.g., dual-frame valve prosthesis or replacement heart valve) may be pre-loaded with a desired orientation based on pre-procedural planning. For example, a predicted location of a bundle of His may be identified and a predicted amount of secondary flex believed to be required to implant the implant within a heart valve location may be determined. An orientation of the implant may be set during loading so as to avoid contact of an anchor or other implant portion with the bundle of His based on the determination. In addition, or alternatively, real-time clocking may be performed via the orientation mechanism 1516 based on direct or indirect fluoroscopy markers. Referring to **Figures 20H to 20I****,** which illustrate a virtual representation of implant **30, 1230** superimposed on images (e.g., fluoroscopic images) of the patient's inner body (e.g., heart anatomy) that have been taken before performing the rotation, the implant **30, 1230** can be positioned by rotating the orientation knob **1516,** to avoid contact of one or more anchors **37** or other portions of the implant **30, 1230** with, for example, the bundle of His of the patient, represented by the marker **3000** superimposed on the image. The rotation (orientation) of the implant **30, 1230** can be performed intraprocedurally (e.g., by rotating from **Figure 20H to Figure 20I****)** before deployment of the implant, to prevent (or reduce the likelihood of) the anchors **37** from contacting the bundle of His or other undesired tissue contact location based on the location of the marker **3000.** That is, the orientation mechanism **1516** can be used not only during implantation as described with reference to **Figures 23A****-23C** but also before delivery of the implant by marking a point **3000** to be avoided, e.g., the bundle of His of the patient, on the image taken before performing the delivery of the implant. With respect to indirect visualization, a relationship (e.g., angle offset Θ) between an anchor-free zone and a fluoroscopic indicator on the implant can be determined. Then, a marker **3000** identifying a location on the bundle of His can be marked and the angle offset Θ can be drawn on a preoperative image (e.g., CT scan) of the patient's heart. The implant view can then be set to place the fluoroscopic plane orthogonal to the fluoroscopic indicator. The implant can then be loaded consistent to the determined angle offset Θ. Then, the clinician can bring the fluoroscopic indicator into a center of view in a fluoroscopic image to place the implant in a desired orientation without flipping to a direct fluoroscopic view. The fluoroscopic indicator may be an existing feature of the implant and not a separate indicator. In this instance, the loading step may not be necessary.

**Figure 21** illustrates another configuration of a handle **1614** similar to the embodiments of the handle **14, 1514** illustrated in and described in relation to **Figures 1****,** **11****,** and **20A****-20C.** Reference numerals of the same or substantially the same features may share the same last two digits. The handle **1614** can be configured to rotate an implant **30, 1230** during delivery. The orientation knob **1616** can extend along a longitudinal axis of the handle **1614** and be configured to rotate about the longitudinal axis of the handle **1614.** The orientation knob **1616** can be configured to rotate the outer proximal shaft **302** and the implant **30, 1230** when the orientation knob **1616** is rotated.

**Figures 22-23C** illustrate an implant **30** delivered to a heart. As shown, the heart may include a hot spot **2000.** The hot spot **2000** can be within the ventricular septum of the heart near the aortic valve that includes conduction fibers (e.g., right and/or left branches of the bundle of His). When the implant **30** is delivered to the tricuspid valve of the heart, the anchors **37** of the implant **30** may contact the conduction fibers within the hot spot **2000.** If the anchors **37** of the implant **30** contact the conduction fibers, this can cause an atrioventricular block ("AV block") within the tricuspid valve. Advantageously, any of the orientation knobs **1516, 1616** described herein can be used to rotate, or clock, the implant **30** during delivery so that the anchors **37** of the implant **30** do not contact the conduction fibers. For example, the clocking of the implant may advantageously cause the anchors 37 to avoid the main fibrous bundle running along the right ventricle septum near the aortic valve. In addition, clocking functionality may facilitate use of asymmetric implant designs that may offer additional benefits, such as enhanced sealing capability or avoidance of erosion in key areas, such as the aortic root in the atrium. Although the implant **30** is shown and described, other implants (e.g., implant **1230** or other implants described herein) can also be delivered or "clocked" as described herein.

### Additional Statements and Terminology

From the foregoing description, it will be appreciated that an inventive product and approaches for implant delivery systems are disclosed. While several components, techniques and aspects have been described with a certain degree of particularity, it is manifest that many changes can be made in the specific designs, constructions and methodology herein above described without departing from the spirit and scope of this disclosure.

The section headings used herein are merely provided to enhance readability and are not intended to limit the scope of the embodiments disclosed in a particular section to the features or elements disclosed in that section. Certain features that are described in this disclosure in the context of separate implementations can also be implemented in combination in a single implementation. Conversely, various features that are described in the context of a single implementation can also be implemented in multiple implementations separately or in any suitable subcombination. Moreover, although features may be described above as acting in certain combinations, one or more features from a claimed combination can, in some cases, be excised from the combination, and the combination may be claimed as any subcombination or variation of any subcombination. In some embodiments, the delivery system or delivery device comprises various features that are present as single features (as opposed to multiple features). For example, in one embodiment, the delivery system includes a single delivery device with a single implant. Multiple features or components are provided in alternate embodiments.

Moreover, while methods may be depicted in the drawings or described in the specification in a particular order, such methods need not be performed in the particular order shown or in sequential order, and that all methods need not be performed, to achieve desirable results. Other methods that are not depicted or described can be incorporated in the example methods and processes. For example, one or more additional methods can be performed before, after, simultaneously, or between any of the described methods. Further, the methods may be rearranged or reordered in other implementations. Also, the separation of various system components in the implementations described above should not be understood as requiring such separation in all implementations, and it should be understood that the described components and systems can generally be integrated together in a single product or packaged into multiple products. Additionally, other implementations are within the scope of this disclosure.

Conditional language, such as "can," "could," "might," or "may," unless specifically stated otherwise, or otherwise understood within the context as used, is generally intended to convey that certain embodiments include or do not include, certain features, elements, and/or steps. Thus, such conditional language is not generally intended to imply that features, elements, and/or steps are in any way required for one or more embodiments.

Spatially relative terms, such as "proximal", "distal", "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (c.g., compositions and apparatuses including device and methods). For example, the term "comprising" will be understood to imply the inclusion of any stated elements or steps but not the exclusion of any other elements or steps.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than or equal to 10% of, within less than or equal to 5% of, within less than or equal to 1% of, within less than or equal to 0.1% of, and within less than or equal to 0.01% of the stated amount. If the stated amount is 0 (e.g., none, having no), the above recited ranges can be specific ranges, and not within a particular % of the value. For example, within less than or equal to 10 wt./vol. % of, within less than or equal to 5 wt./vol. % of, within less than or equal to 1 wt./vol. % of, within less than or equal to 0.1 wt./vol. % of, and within less than or equal to 0.01 wt./vol. % of the stated amount.

Some embodiments have been described in connection with the accompanying drawings. The figures are drawn to scale, but such scale should not be limiting, since dimensions and proportions other than what are shown are contemplated and are within the scope of the disclosed inventions. Distances, angles, etc. are merely illustrative and do not necessarily bear an exact relationship to actual dimensions and layout of the devices illustrated. Components can be added, removed, and/or rearranged. Further, the disclosure herein of any particular feature, aspect, method, property, characteristic, quality, attribute, element, or the like in connection with various embodiments can be used in all other embodiments set forth herein. Additionally, it will be recognized that any methods described herein may be practiced using any device suitable for performing the recited steps.

In some configurations, the delivery system comprises one or more of the following: means for introducing the delivery device, means for stabilizing the delivery device, means for steering the delivery device, means for releasing the implant from the delivery device, etc.

While a number of embodiments and variations thereof have been described in detail, other modifications and methods of using the same will be apparent to those of skill in the art. Accordingly, it should be understood that various applications, modifications, materials, and substitutions can be made of equivalents without departing from the unique and inventive disclosure herein or the scope of the claims.

Examples are set out in the following list of numbered clauses:
1. A delivery device for delivering an implant, the delivery device comprising:
   a shaft assembly comprising a proximal end portion and a distal end portion, wherein the proximal end portion of the shaft assembly comprises a handle including at least one actuator;
   a plurality of suture portions,
   wherein a first end of each of the plurality of suture portions is permanently coupled to the distal end portion of the shaft assembly,
   wherein a second end of each of the plurality of suture portions is removably coupled to at least one retention member of the distal end portion of the shaft assembly after being inserted through a coupling member of an implant,
   wherein actuation of the at least one actuator causes the second end of each of the plurality of suture portions to be decoupled from the at least one retention member of the distal end portion of the shaft assembly.
2. The delivery device of clause 1, wherein the plurality of suture portions is formed from a single continuous suture strand.
3. The delivery device of clause 1, the at least one actuator comprises a rotatable knob.
4. The delivery device of clause 1, wherein the at least one retention member comprises a plurality of retention members spaced circumferentially around the distal end portion of the shaft assembly.
5. The delivery device of clause 1, wherein the second end of each of the plurality of suture portions comprises a suture loop that is configured to be looped onto the at least one retention member.
6. The delivery device of clause 5, wherein the at least one retention member comprises an axially extending tab.
7. The delivery device of clause 1, wherein the shaft assembly comprises an inner manifold shaft and an outer release shaft, wherein the outer release shaft is coaxial with the inner release shaft.
8. The delivery device of clause 7, wherein the first end of each of the plurality of suture portions is fixed to the inner manifold shaft, wherein the second end of each of the plurality of suture portions is removably coupled to at least one retention member of the inner manifold shaft, wherein actuation of the at least one actuator causes translation of the inner manifold shaft with respect to the outer release shaft, and wherein translation of the inner manifold shaft with respect to the outer release shaft causes at least one structure of the distal end portion of the outer release shaft to decouple the second end of each of the plurality of suture portions from the at least one retention member.
9. The delivery device of clause 8, wherein the at least one structure comprises a plurality of windows, and wherein the second end of each of the plurality of suture portions is inserted through one of the plurality of windows prior to being removably coupled to the at least one retention member of the inner manifold shaft.
10. A delivery system for delivering a replacement heart valve, the delivery system comprising:
   an outer release lumen comprising a proximal end and a distal end;
   an inner manifold shaft comprising a proximal end and a distal end,
   wherein the manifold shaft is coaxially positioned within the release shaft, and
   nine suture loops;
   wherein a distal portion of the manifold shaft comprises three tabs arranged circumferentially around the distal portion of the manifold shaft,
   wherein each of the three tabs is adapted to receive a first end of three of the nine suture loops,
   wherein a distal portion of the release shaft comprises three windows,
   wherein each window of the three windows is adapted to align with a respective one of the three tabs of the manifold shaft,
   wherein a second end of each of the nine suture loops is non-removably coupled to a cog at the distal end of the manifold shaft,
   wherein a portion of each of the nine suture loops is looped through a respective eyelet positioned at a proximal end of the replacement heart valve, and
   wherein sliding movement of the manifold shaft in a distal direction while keeping the release shaft fixed in position causes a distal edge of each window of the release shaft to push the second end of each of the nine suture loops proximally along a respective one of the three tabs of the manifold shaft until the second end of each of the nine suture loops is released from the respective one of the three tabs, thereby allowing the replacement heart valve to be decoupled from the delivery system.
11. The delivery system of clause 10, wherein the release shaft comprises at least one radially inwardly-protruding retention member configured to be received within at least one slot of the manifold shaft so as to prevent rotation of the release shaft with respect to the manifold shaft to thereby maintain alignment of each window with a respective tab.
12. The delivery system of clause 10 or 11, wherein each of the three tabs has a different length or substantially the same length.
13. The delivery system of any of clauses 10 to 12, wherein the nine suture loops are formed from a single continuous strand.
14. A delivery device for delivery of a replacement heart valve, the delivery device comprising:
   an outer shaft comprising a proximal end and a distal end;
   an inner manifold shaft comprising a proximal end and a distal end, wherein the manifold shaft is coaxially positioned within the outer shaft, wherein the inner manifold shaft comprises a distal tether retention component at the distal end of the manifold shaft and a proximal tether retention component comprising a plurality of slots, wherein the proximal tether retention component is spaced proximally of the distal tether retention component;
   nine suture loops, wherein each of the nine suture loops comprise a first end opposite a loop end, wherein the first end is non-removably coupled to the distal tether retention component; and
   a release suture configured to releasably secure the nine suture loops to the inner manifold shaft;
   wherein each of the plurality of slots is adapted to receive the loop end of one or more of the nine suture loops,
   wherein the loop end of each of the nine suture loops is looped through a respective eyelet positioned at a proximal end of a replacement heart valve,
   wherein each loop end of the nine suture loops is configured to receive the release suture, and
   wherein removing the release suture from the loop end of the nine suture loops allows the suture loops to be released from the plurality of slots, thereby allowing the replacement heart valve to be decoupled from the delivery device.
15. The delivery device of clause 14, wherein the plurality of slots comprise nine slots.
16. The delivery device of clause 14, wherein the plurality of slots comprise three slots.
17. The delivery device of any one of clauses 14 to 16, wherein the nine suture loops are formed from a single continuous strand.
18. The delivery device of any one of clauses 14 to 17, wherein the distal tether retention manifold extends radially outward from a longitudinal axis of the inner manifold shaft.
19. The delivery device of any one of clauses 14 to 18, wherein the proximal tether retention manifold extends radially outward from a longitudinal axis of the inner manifold shaft.
20. The delivery device of any one of clauses 14 to 19, wherein the inner manifold comprises a central shaft, wherein the distal tether retention manifold and the proximal tether retention manifold extends from the central shaft.
21. The delivery device of clause 20, wherein the central shaft comprises a plurality of apertures.
22. The delivery device of clause 21, wherein each of the plurality of apertures aligns with the plurality of slots.
23. A delivery device for delivery of a replacement heart valve, the delivery device comprising:
   an outer release shaft comprising a proximal end and a distal end;
   an inner manifold shaft comprising a proximal end and a distal end,
   wherein the manifold shaft is coaxially positioned within the release shaft, and
   a plurality of suture loops;
   wherein a distal portion of the manifold shaft comprises a plurality of tabs arranged circumferentially around the distal portion of the manifold shaft,
   wherein each of the plurality of tabs is adapted to receive a first end of at least one of the plurality of suture loops,
   wherein a distal portion of the release shaft comprises a plurality of windows,
   wherein each window of the plurality of windows is adapted to align with a respective one of the plurality of tabs of the manifold shaft,
   wherein a second end of each of the plurality of suture loops is non-removably coupled to the distal end of the manifold shaft,
   wherein a portion of each of the plurality of suture loops is looped through a respective eyelet positioned at a proximal end of the replacement heart valve, and
   wherein sliding movement of the manifold shaft in a distal direction while keeping the release shaft fixed in position causes a distal edge of each window of the release shaft to push the second end of each suture loop proximally along a respective one of the plurality of tabs of the manifold shaft until the second end of each suture loop is released from the respective one of the plurality of tabs, thereby allowing the replacement heart valve to be decoupled from the delivery device.
24. The delivery device of clause 23, wherein each of the plurality of tabs has a different length or substantially the same length.
25. A delivery system for delivering an expandable implant to a body location, the delivery system comprising:
   an outer sheath subassembly comprising an outer proximal shaft having an outer lumen and a proximal end and a distal end, wherein the outer sheath subassembly comprises a capsule subassembly configured to retain the expandable implant in a compressed configuration;
   a rail subassembly located within the outer lumen, the rail subassembly comprising a rail shaft having a proximal end and a distal end, wherein the rail subassembly comprises one or more pull wires attached to the rail shaft configured to provide an axial force on the rail shaft to steer the rail subassembly;
   a mid-shaft subassembly within a lumen of the rail subassembly, the mid-shaft subassembly comprising a hypotube having a lumen and a proximal end and a distal end, wherein the mid-shaft subassembly comprises a pusher configured to radially restrain at least a proximal-most portion of the expandable implant; and
   a proximal handle comprising a capsule knob operably coupled to the outer sheath subassembly and one or more flex knobs operably coupled to the one or more pull wires, wherein rotation of the capsule knob causes translation of the outer sheath subassembly, and wherein rotation of the one or more flex knobs causes bending of the rail shaft.
26. The delivery system of clause 25, wherein a distal tip of the capsule subassembly comprises a plurality of lobes configured to facilitate staggered recapture of distal anchors of the expandable implant as the outer sheath subassembly is translated distally to recapture the expandable implant after initial deployment.
27. The delivery system of clause 25, wherein the pusher comprises a flat distal contact surface without a lip or cup portion.
28. The delivery system of clause 25, wherein the handle further comprises an orientation knob configured to rotate an orientation of the expandable implant.
29. The delivery system of clause 28, wherein the orientation knob is operably coupled to a worm gear that is operably coupled to an adapter and linear guide of the outer sheath subassembly positioned within the handle such that rotation of the orientation knob causes rotation of the linear guide of the outer sheath subassembly and thus rotation of the implant retained within the capsule subassembly of the outer sheath subassembly.
30. The delivery system of clause 25, further comprising the expandable implant, wherein the expandable implant comprises a frame having an inflow end and an outflow end, wherein the inflow end comprises an axial inflow strut having an eyelet configured to receive a tether loop to facilitate releasable attachment of the expandable implant to the delivery system, wherein a radius of curvature of a proximal upper edge of a proximal tip of the axial inflow strut is less than a height from the proximal tip of the axial inflow strut to a distal-most edge of the eyelet.
31. The delivery system of clause 30, wherein the eyelet has a semi-circular shape, an oval shape, or a bean shape.

## Claims

1. A delivery system (10) for delivering an expandable implant (30) to a body location, the delivery system comprising:
an outer sheath subassembly (20) comprising an outer proximal shaft (302) having an outer lumen and a proximal end and a distal end, wherein the outer sheath subassembly comprises a capsule subassembly (306) configured to retain the expandable implant in a compressed configuration;
a rail subassembly (21) located within the outer lumen, the rail subassembly comprising a rail shaft (402) having a proximal end and a distal end, wherein the rail subassembly comprises one or more pull wires (410) attached to the rail shaft configured to provide an axial force on the rail shaft to steer the rail subassembly;
a mid-shaft subassembly (22) within a lumen of the rail subassembly, the mid-shaft subassembly comprising a hypotube (502) having a lumen and a proximal end and a distal end, wherein the mid-shaft subassembly comprises a pusher (506) configured to radially restrain at least a proximal-most portion of the expandable implant; and
a proximal handle (14) comprising a capsule knob (905) operably coupled to the outer sheath subassembly and one or more flex knobs (915) operably coupled to the one or more pull wires, wherein rotation of the capsule knob causes translation of the outer sheath subassembly, and wherein rotation of the one or more flex knobs causes bending of the rail shaft.

2. The delivery system of claim 1, wherein a distal tip (309) of the capsule subassembly comprises a plurality of lobes (309A) configured to facilitate staggered recapture of distal anchors of the expandable implant as the outer sheath subassembly is translated distally to recapture the expandable implant after initial deployment.

3. The delivery system of claim 1 or claim 2, wherein the pusher comprises a flat distal contact surface without a lip or cup portion.

4. The delivery system of any preceding claim, wherein the handle further comprises an orientation knob (1516) configured to rotate an orientation of the expandable implant.

5. The delivery system of claim 4, wherein the orientation knob is operably coupled to a worm gear (1520) that is operably coupled to an adapter (1522) and linear guide (1524) of the outer sheath subassembly positioned within the handle such that rotation of the orientation knob causes rotation of the linear guide of the outer sheath subassembly and thus rotation of the implant retained within the capsule subassembly of the outer sheath subassembly.

6. The delivery system of any preceding claim, further comprising the expandable implant, wherein the expandable implant comprises a frame (34) having an inflow end and an outflow end, wherein the inflow end comprises an axial inflow strut having an eyelet (35) configured to receive a tether loop (710) to facilitate releasable attachment of the expandable implant to the delivery system, wherein a radius of curvature of a proximal upper edge of a proximal tip of the axial inflow strut is less than a height from the proximal tip of the axial inflow strut to a distal-most edge of the eyelet.

7. The delivery system of claim 6, wherein the eyelet has a semi-circular shape, an oval shape, or a bean shape.

8. The delivery system of any preceding claim, wherein the capsule subassembly comprises a distal hypotube (308).

9. The delivery system of any preceding claim, wherein a distal tip of the capsule subassembly comprises an atraumatic tip adapted to act as a funnel to facilitate recapture of the expandable implant.

10. The delivery system of any preceding claim, wherein a distal tip of the capsule is loaded with radiopaque material to facilitate detection under radiographic imaging.

11. The delivery system of any preceding claim, wherein the rail shaft comprises a rail proximal shaft (404) directly attached to the handle at a proximal end, and a rail hypotube (406) attached to the distal end of the rail proximal shaft.

12. The delivery system of claim 11, wherein the pull wires are attached to an inner surface of the rail hypotube.

13. The delivery system of claim 12, wherein the pull wires are attached at different longitudinal locations on the rail hypotube.

14. The delivery system of claim 13, wherein the rail hypotube provides a primary bend or flex along a medial/lateral trajectory and a secondary bend or flex along an anterior/posterior trajectory.

15. The delivery system of any preceding claim, wherein the hypotube (502) of the mid-shaft subassembly is attached at its proximal end to a mid-shaft proximal tube (504), wherein the mid-shaft proximal tube is attached at its proximal end to the handle, and wherein the pusher is located at the distal end of the mid-shaft hypotube.
